# EUROPEAN PATENT SPECIFICATION

(11) **EP 1 572 918 B1**
(45) Date of publication and mention of the grant of the patent: **19.10.2011**
(21) Application number: 03721571.2
(22) Date of filing: 09.04.2003
(51) Int. Cl.: A61K 38/04, A61K 38/08, A61K 38/14, A61K 39/02, A61K 39/12, A61K 39/385

(54) **SYNTHETIC GLYCO-LIPO-PEPTIDES AS VACCINES**
SYNTHETISCHE GLYKOLIPOPEPTIDE ALS IMPFSTOFFE
GLYCOLIPOPEPTIDES DE SYNTHESE UTILISES COMME VACCINS

(30) Priority: 15.04.2002 US 372105 P; 06.05.2002 US 377595 P
(43) Date of publication of application: 14.09.2005
(73) Proprietor: Oncothyreon Inc., Seattle, WA 98121 (US)
(72) Inventor: KOGANTY, R., Rao, Edmonton, Alberta T6J 3R3 (CA); JIANG, Zi-Hua, Edmonton, Alberta T6J 4R8 (CA); YALAMATI, Damayanthi, Edmonton, Alberta T6J 6T4 (CA); GANDHI, Sham, Edmonton, Alberta T6L 2T5 (CA); BUDZYNSKI, Wladyslaw, Edmonton, Alberta T6R 2P6 (CA); KRANTZ, Mark, J., Edmonton, Alberta T6W 1C3 (CA); LONGENECKER, B., Michael, Edmonton, Alberta T6R 1C8 (CA)
(74) Representative: Broughton, Jon Philip
(86) International application number: PCT/US2003/010750
(87) International publication number: WO 2003/089574

(56) References cited:
- EP-A- 0 230 893
- WO-A-01/36433
- WO-A-93/21211
- US-A- 4 868 155
- US-A- 5 837 249
- DATABASE CAPLUS [Online] CHEMICAL ABSTRACTS SERVICE, COLUMBUS, OHIO, US; 1997, DIEZ-BARRA, ENRIQUE ET AL: "Solvent-free phase transfer catalysis. Improvements on serine O-alkylation" XP002369800 retrieved from STN Database accession no. 127:220955
- PRICE ET AL: 'Summary on the ISOBM TD-4 Workshop: Analysis of 56 Monoclonal Antibodies against the MUCI Mucin.' TUMOR BIOLOGY. vol. 19, no. 1, December 1998, pages 1 - 20, XP002071245
- TOYOKUNI ET AL: 'Synthetic Vaccines: Synthesis of A Dimeric Tn Antigen-Lipopeptide Conjugate That Elicits Immune Responses against Tn-Expressing Glycoproteins.' J.AM.CHEM.SOC. vol. 116, 1994, pages 395 - 396, XP000946183
- REICHEL ET AL: 'Synthetic Carbohydrate-Based Vaccines: Synthesis of an L-Glycero-D-Manno-Heptose Antigen-T-Epitope-Lipopeptide Conjugate.' CHEM.COMMUN. vol. 2, 1997, pages 2087 - 2088, XP009062463

## Description

This application is a non-provisional of Koganty, et al., Serial No. 60/372,105, filed April 15, 2002,attorney docket no. KOGANTY4-USA, and of Ser. No. 60/377,595, filed May 6, 2002 (KOGANTY4.1-USA).

### Cross-Reference to Related Applications

Jiang,et al., PCT/US00/31281, filed Nov. 15, 2000 (our docket JIANG3A-PCT).

Longenecker, et al., 08/229,606, filed April 12, 1994 (our docket LONGENECKER5-USA, and PCT/US95/04540, filed April 12, 1995 (our docket LONGENECKER5-PCT).

Wong, USP 6, 013, 779.

Budzynski et al., 60/278,698, filed March 27, 2001, and PCT/IB02/02188, filed March 27, 2002, "Vaccine for modulating between T1 and T2 immune responses".

### BACKGROUND OF THE INVENTION

### Field of the Invention:

The present invention relates to immunotherapy and diagnosis. In particular, it relates to the use of novel synthetic glycolipopeptides as vaccines against cancers and pathogens presenting cross-reactive epitopes. The glycolipopeptides may also be used, in labeled or immobilized form, as diagnostic reagents.

### Description of the Background Art

### The Immune System.

The ability of vertebrates to protect themselves against infectious microbes, toxins, viruses, or other foreign macromolecules is referred to as immunity. The art distinguishes between natural, and acquired or specific immunity (Abbas, et al., Cellular and Molecular Immunology, W. B. Saunders Company, 1991; Hood, et al., Immunology, 2nd Edition, The Benjamin/Cummings Publishing Company Inc., 1984).

Natural immunity is comprised of defense mechanisms which are active before exposure to microbes or foreign macromolecules, are not enhanced by such exposure, and do not distinguish among most substances foreign to the body. Effectors of natural immunity are physical barriers such as skin or mucous membranes, phagocytic cells such as macrophages or neutrophils, a class of lymphocytes termed natural killer cells, and the complement system. Complement is a serum protein complex that is destructive to certain bacterial and other cells sensitized by specific, complement-fixing antibodies; its activity is effected by a series of interactions resulting in proteolytic cleavages and which can follow one or the other of at least two pathways (Illustrated Stedman's Medical Dictionary, 24th Edition, Williams and Wilkins, Baltimore/London, 1982).

Acquired or specific immunity comprises defense mechanisms which are induced or stimulated by exposure to foreign substances.

In vertebrates, the mechanisms of natural and specific immunity cooperate within a system of host defenses, the immune system, to eliminate foreign invaders. In addition to microbes, cancer cells, parasites and virus-infected cells, the immune system also recognizes and eliminates cells or tissues transplanted into a subject from a genetically different individual of the same species (allografts) or from a different species (xenografts).

The events by which the mechanisms of specific immunity become engaged in the defense against invading microorganisms cancer cells, etc. are termed immune responses. Vertebrates have two basic immune responses: humoral and cellular. Humoral immunity is provided by B lymphocytes, which, after proliferation and differentiation, produce antibodies which circulate in the blood and lymphatic fluid. Cellular immunity is provided by the T cells of the lymphatic system. The cellular immune response is particularly effective against fungi, parasites, intracellular viral infections, cancer cells and foreign matter, whereas the humoral response primarily defends against the extracellular phases of bacterial and viral infections.

An "antigen" is a substance which is recognized (specifically bound) by an antibody or a T-cell receptor, regardless of whether it can induce an immune response. Foreign substances inducing specific immunity are termed "immunizing antigens", or "immunogens". An "hapten" is an antigen which cannot, by itself, elicit an immune response (though a conjugate of several molecules of the hapten, or of the hapten to a macromolecular carrier, might do so). Since the present application is concerned with eliciting immune response, the term "antigen" will refer to immunizing antigens unless otherwise stated.

The time course of an immune response is subdivided into the cognitive phase, wherein specific lymphocytes recognize the foreign antigen; the activation phase, wherein specific lymphocytes respond to the foreign antigen; and the effector phase, within which antigen-activated lymphocytes mediate the processes required to eliminate the antigen. Lymphocytes are immune cells that are specialized in mediating and directing specific immune responses (Abbas, et al., loc. cit.; Hood, et al., loc. cit.).

The immune system has evolved so that it is able to recognize surface features of macromolecules that are not normal constituents of the host. A foreign molecule which is recognized by the immune system (e.g., bound by antibodies), regardless of whether it can itself elicit an immune response, is called an "antigen", and the portion of the antigen to which an antibody binds is called the "antigenic determinant", or "epitope". When the antigen is a polypeptide, it is customary to classify epitopes as being linear (i.e., composed of a contiguous sequence of amino acids along the polypeptide chain) or nonlinear (i.e., composed of amino acids brought into proximity as a result of the folding of the polypeptide chain). (The nonlinear epitopes are also called "conformational" because they arise through the folding of the polypeptide chain into a particular conformation.)

To cope with the immense variety of epitopes encountered, the immune system of a mammalian individual contains an extremely large repertoire of lymphocytes. Each lymphocyte clone of the repertoire contains surface receptors specific for one epitope. It is estimated that the mammalian immune system can distinguish at least 10⁸ distinct antigenic determinants (Abbas, et al., loc. cit., p.8).

An initial or primary immune response to a foreign antigen enhances the ability of the immune system to respond again to that antigen (in a secondary immune response). This feature of specific immunity is called immunologic memory. Secondary immune responses are often more effective than primary responses.

The lymphocytes in an individual specifically respond to foreign antigens but are usually unresponsive to the potentially antigenic substances native to that individual. Immunologic unresponsiveness is referred to as tolerance. Self-tolerance is acquired at an early developmental stage when potentially self-recognizing lymphocytes come into contact with self-antigens and are prevented from developing to a stage at which they would be able to respond positively to self antigens (Abbas, et al., loc., cit.)

Lymphocytes are the agents of antigenic specificity in the immune response. They divide into two groups. One group, the "B-lymphocytes" or "B-cells", play a central role in the production of antibodies. Antibodies (immunoglobulins, Ig's) are proteins capable of binding antigens, and exerting effector functions that are involved in the elimination of foreign antigens. The other group consists of T-lymphocytes or T-cells that perform a variety of functions including help for B-cells, production of delayed-type hypersensitivity reactions, and specific killing of virus-infected cells (Bjorkman, et al., Annu. Rev. Biochem., 59, 253, 1990).

Normally, immune responses progress toward effector mechanisms characteristic of both B and T-lymphocytes. However, in the course of most immune responses, either B or T lymphocytes assume a dominant role, with less substantial participation of the respective other type of lymphocyte. Immune responses whose effector mechanisms are mediated preponderantly through B-cells and antibodies are termed humoral immune responses. Those responses wherein T-cells mediate the more important effector functions are referred to as cell-mediated or cellular immune responses.

B-cells constitute the population of lymphocytes central to humoral immune responses. Each clone of B-lymphocytes expresses membrane immunoglobulins (membrane Ig's, surface-bound antibody molecules) that function as antigen receptors with one unique epitope specifically per B-lymphocyte clone. These membrane Ig molecules (antigen receptors) are the sole source of B-cell specificity (Bjorkman, et al., loc. cit.). Antigens that contain an epitope complementary to the membrane Ig will bind to the antigen receptor. Such antigens are also referred to as cognate antigens of the antibody. On protein antigens, antibodies can bind linear determinants (epitopes formed by adjacent amino acid residues in the covalent sequence), or conformational determinants, which are formed by amino acid residues from separate portions of the linear polypeptide that are specially juxtaposed by polypeptide folding (Abbas, et al., loc. cit.). Binding to the antigen receptor (membrane Ig) will result in differentiation and clonal proliferation of the B-lymphocyte. Some of its progeny will differentiate into mature plasma cells which are specialized in the synthesis of antibodies corresponding in epitope specificity to the membrane Ig by which the B-lymphocyte had initially bound the antigen.

By an effector mechanism typical of humoral immune responses, antibodies will bind to cognate epitopes on the surface of invading target cells, e.g., bacteria. Following antibody binding, the components of the complement system will sequentially attach to the target cell-antibody complex, resulting ultimately in the rupture of the target cell membrane and killing of the target cell. By another antibody-mediated effector mechanism, target antigens are bound and cross-linked (opsonized) by antibodies, and are thus prepared for ingestion and subsequent destruction by phagocytes of reticuloendothelial origin, such as granulocytes or macrophages.

The antibody itself is an oligomeric molecule, classified, according to its structure, into a class (e.g., IgG) and subclass (e.g., IgGl). IgG molecules are the most important component of the humoral immune response and are composed of two heavy (long) and two light (short) chains, joined by disulfide bonds into a "Y" configuration. The molecule has both variable regions (at the arms of the "Y") and a constant region (the hinge and base of the "Y"). The regions are so named because antibodies of a particular subclass, produced by a particular individual in response to different antigens, will differ in the variable region but not in the constant region. The variable regions themselves are composed of both a relatively invariant framework, and of hypervariable loops, which confer on the antibody its specificity for a particular epitope. An antibody binds to an epitope of an antigen as a result of molecular complementarity. The portions of the antibody which participate directly in the interaction is called the "antigen binding site", or "paratope". The antigens bound by a particular antibody are called its "cognate antigens".

An antibody of one animal will be seen as a foreign antigen by the immune system of another animal, and will therefore elicit an immune response. Some of the resulting antibodies will be specific for the unique epitopes (idiotype) of the variable region of the immunizing antibody, and are therefore termed anti-idiotypic antibodies. These often have immunological characteristics similar to those of an antigen cognate to the immunizing antibody. Anti-isotypic antibodies, on the other hand, bind epitopes in the constant region of the immunizing antigen.

The typical effector phase of cell-mediated or cellular immune responses involves lysis or killing of target cells by cytotoxic or cytolytic T-lymphocytes (CTLs) through direct cell-to-cell contact. Molecules from two diverse families of cell-surface glycoproteins, the T-cell receptors (TCRs) and the major histocompatibility complex (MHC) type I glycoproteins, are the key elements of specificity in the CTL response to foreign antigens. T-cell receptors (TCRs) recognize short, linear peptide determinants of 8-24 amino acids, the generation of which usually requires unfolding and proteolytic fragmentation ("processing") of the antigenic protein (Allen, Immunology Today, 8., 270, 1987; Unanue, et al., Science, 236, 551, 1987; Bjorkman, et al., loc. cit.; Braciale, et al., Immunol. Rev., 98, 95, 1987; Unanue, Annu. Rev. Immunol. 2, 395, 1984; Ziegler, et al., J. Immunol., 127, 1869, 1981; Shimonkevitz, et al., J. Exp. Med., 158, 303, 1983; Zweerink, et al., Eur. J. Immunol., 7, 630, 1977). They can also recognize oligosaccharide determinants. (Henningsson, et al., Cancer Immunol. Immunother., 25, 231, 1989; Fung, et al., Cancer Res., 50, 4308, 1990). Unlike antibodies, T-cell receptors cannot recognize conformational epitopes.

The second difference in antigen recognition by antibodies and T-cell receptors is the involvement of a third molecule that performs the role of presenting the antigen to the T-cell receptor. For B-cells, such molecules are not necessary, as the membrane Ig (antibody) forms a stable bimolecular complex with the antigenic protein. For T-cells, the antigenic peptide must be bound by an MHC glyco-protein, and it is this complex of MHC molecule plus peptide that forms the structure recognized by the T-cell receptor. MHC glycoproteins are thus peptide-binding proteins which function as antigen-presenting molecules (Bjorkman, et al., loc. cit.).

Cytotoxic T-lymphocytes, as the main effector cells of the cell-mediated immune response, recognize peptides bound to MHC Class I proteins, and are capable of killing virus-infected cells and cancer cells (Zinkernagel, et al., Nature, 248, 701, 1974; Rouse, et al., Rev. Infect. Dis., 10, 16, 1988; Lukacher, et al., J. Exp. Med., 160, 814, 1984; McMichael, et al., N. Engl. J. Med., 309, 13, 1983; Wraith , et al., J. Gen. Virol., 68, 433, 1987; Cerundolo, et al., Eur. J. Immunol., 17, 173, 1987; Kast, et al., Cell, 59, 603, 1989). CTLs generally display a cell surface marker termed CD8 (Abbas, et al., loc. cit., p. 310). The CD designation refers to a nomenclature system of cell surface markers whereby a surface marker characteristic of a cell lineage or differentiation stage which has a defined structure and is recognized by a group ("cluster") of monoclonal antibodies, is called a member of a cluster of differentiation (CD; Abbas, et al., loc. cit., pp. 19; 398-401).

Another important set of T-lymphocytes is generally CD8-negative and displays the CD4 marker. These T-cells participate in the cognitive and activation phases of both the humoral and cell-mediated immune responses and are referred to as T-helper cells. T-helper cells, through their T-cell receptors, recognize foreign antigens bound to MHC Class II molecules on the surface of accessory cells such as mononuclear phagocytes (macrophages), follicular dendritic cells of the spleen and lymph nodes, Langerhans cells of the epidermis, or venular endothelial cells (Abbas, et al., loc. cit., p. 122-3).

Two distinct subsets of T-helper lymphocytes, termed T_{H}1 and T_{H}2, direct the immune system toward either a primarily cell-mediated or primarily humoral type of response, respectively (Mosmann, et al., Adv. Immunol., 46, 111, 1989). The T_{H}1 and T_{H}2 cells are categorized by their different functions and by the constellation of immunological mediators (cytokines) they produce. Generally, T_{H}1 cells secrete interferon-y and interleukin-2 (IL-2), and contribute to cell-mediated immune responses such as delayed-type hypersensitivity (DTH) and macrophage activation. T_{H}2 cells produce interleukin-4 (IL-4), interleukin-5 (IL-5), and interleukin-10 (IL-10) and help B-cells to generate antibody response (Mosmann, et al., J. Immunol., 136, 2348, 1986; Cherwinski, et al, J. Exp. Med., 166, 1229, 1987; Fiorentino, et al., J. Exp. Med. 170, 2081, 1989).

The tendency for either the cell-mediated or humoral immune response to predominate is believed to be a consequence of cross-regulation (Parish, Transplant. Rev., 13, 35, 1972; Mosmann, Ann. New York Acad. Sci., 628:337, 1991). Thus T_{H}1 cells would inhibit the elicitation of T_{H}2 responses, e.g., by secretion of interferon-γ. Conversely, T_{H}2-cells could inhibit the generation of T_{H}1-responses by producing cytokines such as IL-4 and IL-10 (Salk, et al., Science, 260, 1270, 1993).

### Traditional and Nontraditional Immunogens

Traditional immunogens are usually macromolecular in nature, and are taken up by antigen presenting cells such as macrophages for processing and presentation in the context of major histocompatibility complexes of proteins that are known as MHC class I and II. However such macromolecular immunogens generate several types of non-specific immune responses due to the size of the protein that contain enormous number of epitopes that the immune system may recognise and mount immune responses. Such broad spectrum of responses may not always be beneficial, particularly in the case of self-antigens, which are primary targets in cancers and autoimmune disorders. Cancer antigens share many common features with normal antigens, hence, an immune response to a cancer antigen may also recognize the normal antigen.

Another problem with traditional immunogens relates to the manner of producing them. Purification of the natural immunogen to homogeneity may be difficult and expensive. If the amino acid sequence of a protein immunogen is known, it may be produced by recombinant DNA techniques. However, it may be difficult to achieve the same glycosylation as in the natural immunogen, and recombinant DNA expression does not, by itself, eliminate biological contaminants. For example, a protein produced recombinantly in bacterial cells must be separated from bacterial proteins. Consequently peptides are preferred as replacement to whole protein based products where possible.

For these reasons, there has been interest in the use of fragments of traditional immunogens as vaccines. Such fragments are likely to elicit a more specific immune response, because they present fewer epitopes, and the peptides may be produced by nonbiological synthetic methods.

Unfortunately, small peptide sequences generally do not elicit a humoral immune response. One solution is to chemically link small peptide haptens to an immunogenic carrier protein such as keyhole limpet hemocyanin (KLH). Another is to link several haptens to a branched lysine core to form a larger, immunogenic molecule, as in the so-called MAP-4. Tam, Proc. Nat. Acad. Sci. (USA), 85: 5409-13 (1988). A third is simply to link, end-to-end, a sufficient number of copies of the epitope of interest, with or without a spacer, to create an artificial antigen large enough to act as an immunogen.

Small peptides can elicit a cellular immune response, but this response can be enhanced by first associating the peptides with antigen-presenting cells or with liposomes.

### Glycolipopeptides

Boons and coworkers have described a compound which contains a L-glycero-D-manno-heptose sugar that will act as a B-epitope, the peptide sequence YAFKYARHANVGRNAFELFL (SEQ ID NO:1) that has been identified as a MHC class II restricted recognition site for human T-cells and is derived from an outer-membrane protein of Neisseria meningitidis and the lipopeptide S-2-3[dipalmitoyloxy]-(R/S)-propyl-N-palmitoyl-R-Cysteine (Pam3Cys).The sugar is attached via a spacer (-CH2-CH2-CH2-NHCO-Gly-) to the terminal Leu of the aforementioned sequence. The lipopeptide is attached to the terminal Tyr. The lipopeptide Pam3Cys is a highly potent B-cell and macrophage activator derived from the immunologically active N-terminal sequence of the principal lipoprotein of Escherichia coli and has been used in synthetic peptide-based vaccine and cancer vaccine development. See 128.192.9.100/boonsgroup/target.htm. See also Reichel, F., et. al., "Synthetic carbohydrate-based vaccines:synthesis of an L-glyero-D-manno-heptose antigen-T epitope-lipopeptide conjugate", Chem. Commun., 2087-8 (1997).

Toyokuni, et al., J. Am. Chem. Soc., 116:395-96 (1994) describes a dimeric Tn antigen-lipopeptide conjugate, specifically, di-Tn coupled to tripalmitoyl-S-glycerylcysteinyl serine (P₃CS).

Kudryashov, et al., Proc. Nat. Acad. Sci. (USA), 98:3264-9 (2001) describes glycolipopeptides with one or three Lewis-Y pentasaccharides attached to serine residues of a heptapeptide terminated with a Pam₃Cys moiety.

### Lipopeptide Vaccines

Hopp USP 5,019,383 and EP Appl 93,851 teach coupling a lipophilic group to the amino terminal of a peptide. The coupling is through X, a polyfunctional group having 3 to 5 functional groups (carbonyl and amido groups preferred), at least one of which is bound to the amino terminal of the peptide, and at least one of which is bound to at least one C12-C36 alkyl or alkenyl group, which may be straight or branched, and substituted or unsubstituted.

Heber-Katz, USP 5,837,249 (1998) and EP Appl 203,676 (1986) describe a vaccine which is a peptide-fatty acid conjugate. The peptide comprise a viral epitope. In one embodiment, a Lys-Gly_Gly spacer is attached to the amino terminal end of the viral epitope, and fatty acid (palmitoyl) groups are attached to both the free amino terminal and the epsilon amino group of the terminal Lys. Heber-Katz further contemplated delivering these lipopeptides as part of a liposome.

Lipopeptides are also contemplated by Boutillon, USP 6,015,564, USP 5,993,823, USP 5,871,746, EP Appl 1,065,212, EP Appl 945,461, EP Appl 491,628. Lipophilic groups are attached to both the amino and carboxy termini of the peptides. See also Martinon, et al., J. Immunol. 149:3416-22 (1992); Sauzet, et al., Vaccine, 13: 1339-45 (1995); Vitiello, et al., J. Clin. Invest., 95:341-9 (1995); Mortara, et al., J. Virol., 72:1403-10 (1998).

A synthetic lipopeptide antigen (BLP25), H₂N-STAPPAHGVTSAPDTRPAPGSTAPPK(Pal)G-OH (FIG. 34, of Jiang, et al., PCT/US00/31281)(SEQ ID NO:11), a modified 25-amino-acid sequence that is derived from tumor-associated MUC1 mucin, has been shown to elicit Th1 responses against MUC1.

### Synthesis of Peptides, Glycopeptides, and Lipopeptides

There are two basic strategies for synthesizing oligomers and polymers, sequential synthesis and block synthesis. In sequential synthesis, one monomer is added at a time to a growing oligomer or polymer. In solid phase synthesis, one end of the latter is attached to a solid phase support. In solution synthesis, the growing oligomer or polymer is free in solution, but protective groups limit where the monomer can be added.

Solid phase sequential synthesis of large peptides and glycopeptides suffers from inconsistencies in terms of both purity and yield of the final product. With increase in the number of amino acids that are to be sequentially coupled results in an exponential increase in the number of deleted peptides as impurities. An impurity, which is formed as a result of 'no coupling', is carried forward to the next step due to its attachment to the solid phase. With every coupling, even at 98% coupling efficiency, the number of deletions (uncoupled or miscoupled peptides) increases by **2ⁿ,** where n is the number of amino acids coupled. Thus the synthesis of a 20 amino acid sequence results in the formation of 2²⁰ or, over a million deleted peptides as impurities, in addition to one correct product. While many of these deleted products are formed in undetectable traces, they add up to significant amounts and their removal presents a challenge. Identification and definition of impurities is an impossible task since many of the impurities are very similar to the desired product. In clinical grade manufacture detection, identification and quantification of significant impurities are probably the most important regulatory issues, which must be complied with at significant costs and time.

In block synthesis, two or more oligomeric blocks are condensed together to form the final oligomer or polymer. The blocks themselves may be isolated from nature directly, derived by fragmentation of a naturally occurring molecule, or synthesized. If synthesized, they may themselves be prepared by block or sequential synthesis.

Block synthesis has been in sporadic practice since before the advent of solid phase sequential synthesis. Solid phase sequential synthesis has received much broader attention ever since while block coupling methods have almost disappeared. One of the best known examples of block coupling is the synthesis of human Formyl Methionyl Adrenocorticotropin (For-Met-hACTH). This 40 amino acid sequence was successfully synthesized in pure form by Yajima and his coworkers (Chem. Parm. Bull., 30, 866 (1982); Chem. Pharm. Bull., 34, 4362, 1986) Chillemi et al have synthesized six peptides ranging in size from 8 to 15 amino acids by solution phase synthesis (Int. J. Peptide Protein Res., 35, 271, 1990). More recently G-J. Boons synthesized a chemically linked conjugate of an adjuvant tripalmitoyl cysteine (Pam₃Cys) derived from *E*. *coli* and a T-cell epitope from *Neisseria menigitidis* and sialic acid, a structure that can be classified as glyco-lipo-peptide. So far there have been no reports of MUC1 related peptides of any kind through block synthesis. Block synthesis will address the needs of complex peptides and glyco-peptides. While the synthesis is slow to start, it has advantages of large scale synthesis, purity of the peptides and quality control at every step which is lacked in solid phase synthesis.

### SUMMARY OF THE INVENTION

The present invention deals with design, synthesis of multi-epitopic glyco-lipo-peptides and their applications as immunotherapeutics. These molecules are smaller in size than traditional immunogens, such as mucins, but structurally well defined. These structures are designed to integrate features such as carbohydrate and peptide epitopes derived from glyco-proteins of human or pathogenic origin, and lipid chains to enable them to self assemble into large particles.

Alternatively, these structures may also be incorporated into liposome membrane thus becoming integral components that participate in the construction of liposome membrane. So incorporated, they function like a macromolecular immunogen. These relatively small but well-defined structures are characterized by their ability to self assemble into larger particles while preserving the multiple epitopes that were built into these small molecules. As an immunogen, the small molecule mimics the parent macromolecular glyco-protein while being highly specific in eliciting therapeutic immune responses.

Preferably, the glycolipopeptides of the present invention can elicit both a humoral and a cellular immune response, the humoral response being to at least one component B-epitope, and the cellular immune response being to at least one component T-epitope (the B- and T-epitopes may be the same or different). Preferably, the humoral and/or cellular immune response to the glycolipopeptide is protective against at least one disease.

This invention also deals with novel solution phase methods developed for the synthesis of glycolipopeptides as well as large-scale commercial production in highly purified form. The step-wise solution phase assembly through progressive blocks enables the production of fairly complex and large glyco-lipo-peptides in a highly controlled manner from start to finish. Such stepwise process control is not possible with solid phase methods that are widely practised for the production of peptide based pharmaceutics.

As a proof of principle of this invention, described here are the design, synthesis and immune responses of two glyco-lipo-peptides (Figures 1 and 2). These structures are based on human cancer associated MUC1 mucin (see the list of abbreviations in Table 2) and incorporate two of the carbohydrate epitopes (Figure 3) that are widely regarded as cancer associated.

### BRIEF DESCRIPTION OF THE DRAWINGS

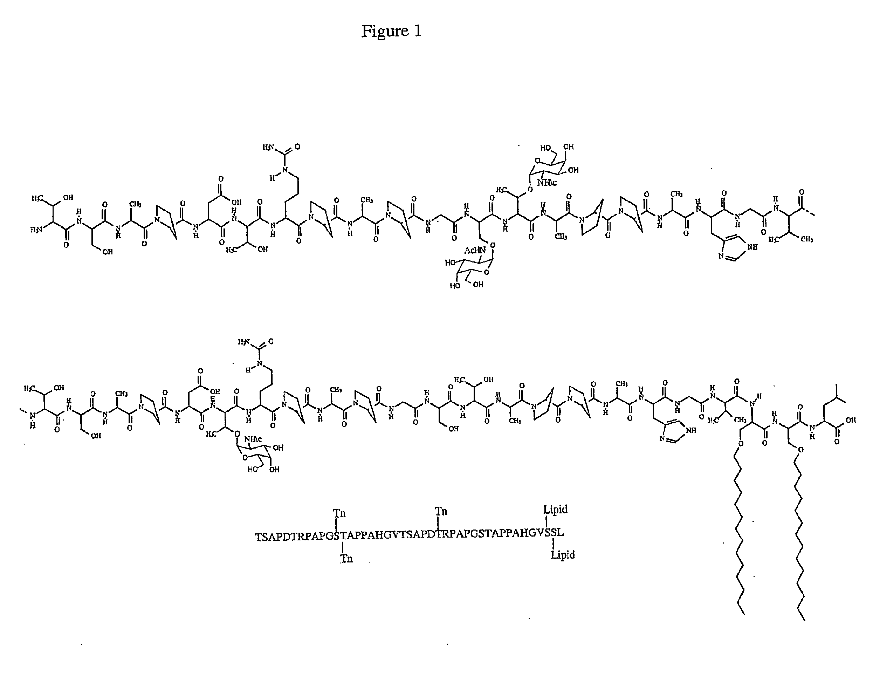

Figure 1: Complete sequence and structure of glyco-lipopeptide 1a. Peptide sequence is SEQ ID NO:2.

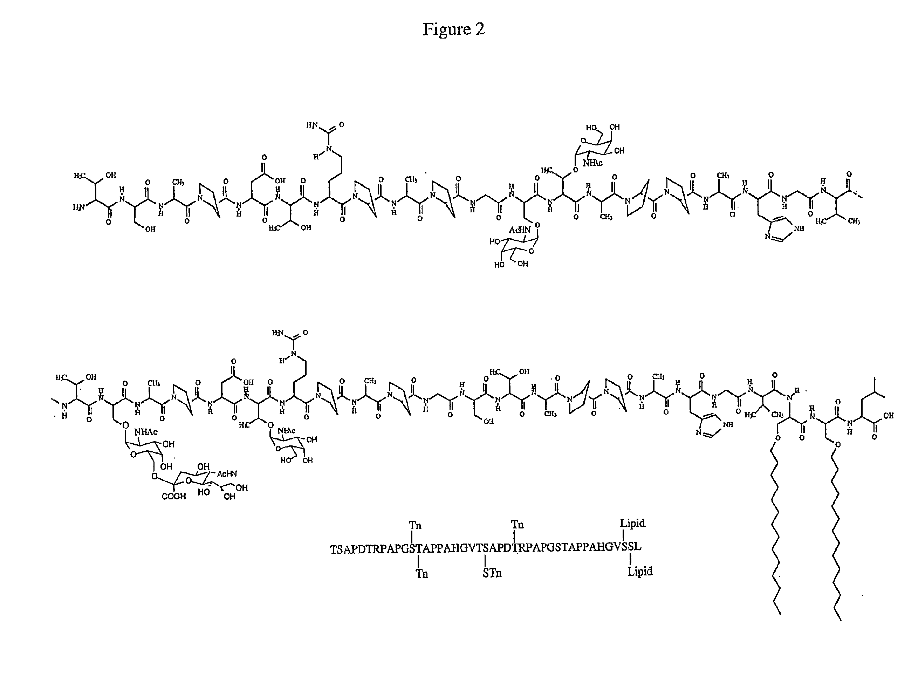

Figure 2: Complete structure and sequence of glyco-lipopeptide 1b. Peptide sequence is SEQ ID NO:2.

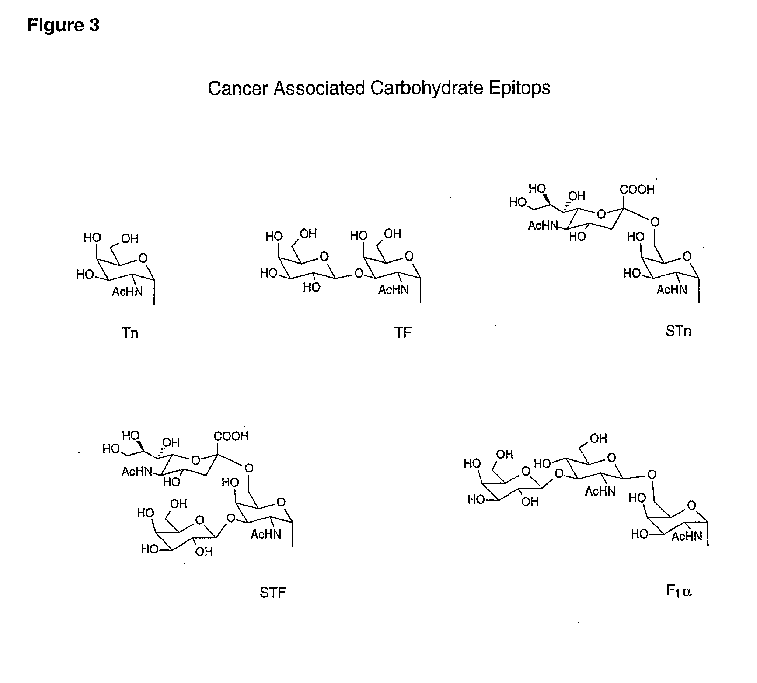

Figure 3: Structures of commonly found cancer associated carbohydrate epitopes

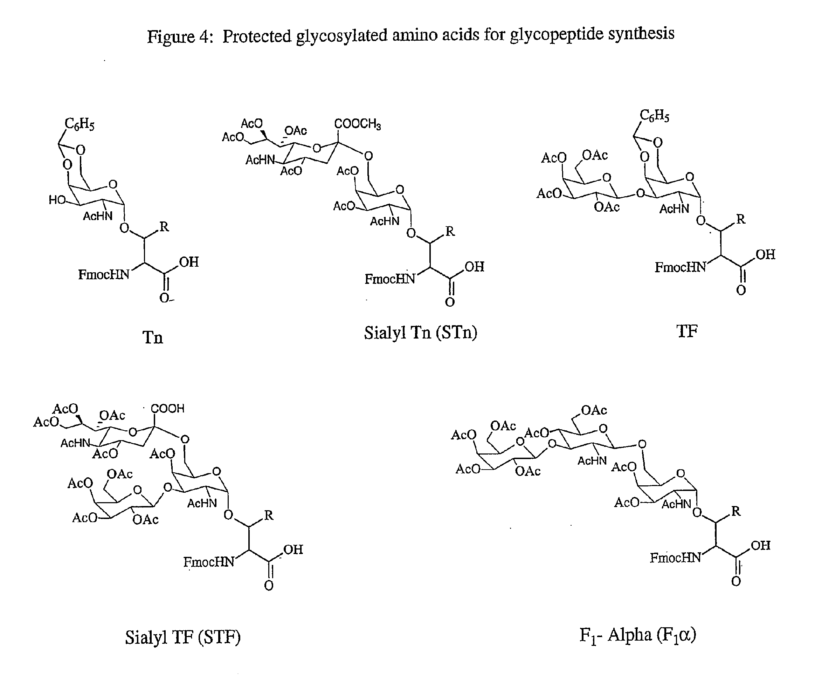

Figure 4: Complete structures of O-glycosylated serines and threonines for glycopeptide syntheses.

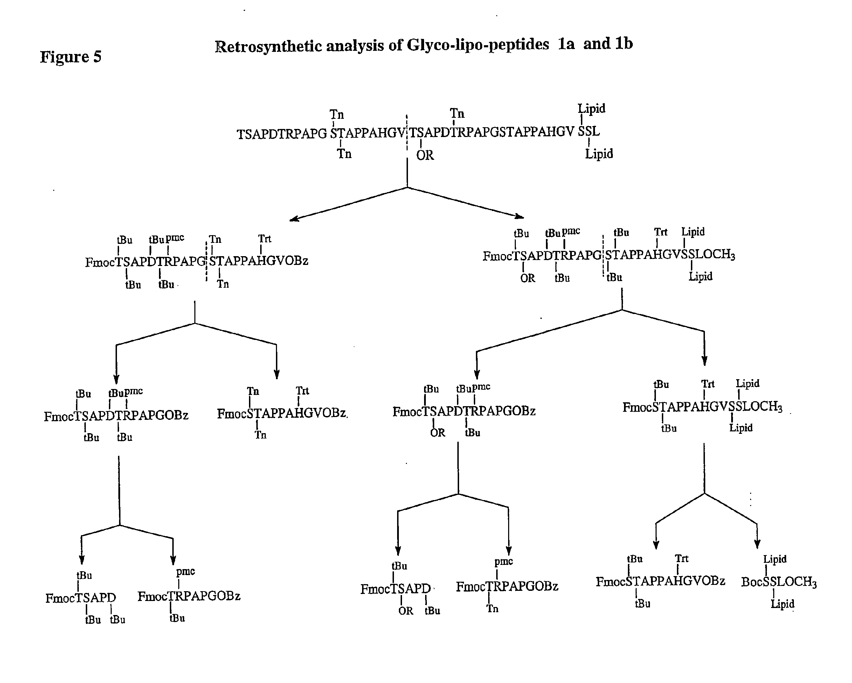

Figure 5: Retro synthetic analysis and breakdown of glyco-lipo-peptides 1a and 1b, peptide sequence SEQ ID NO:2.

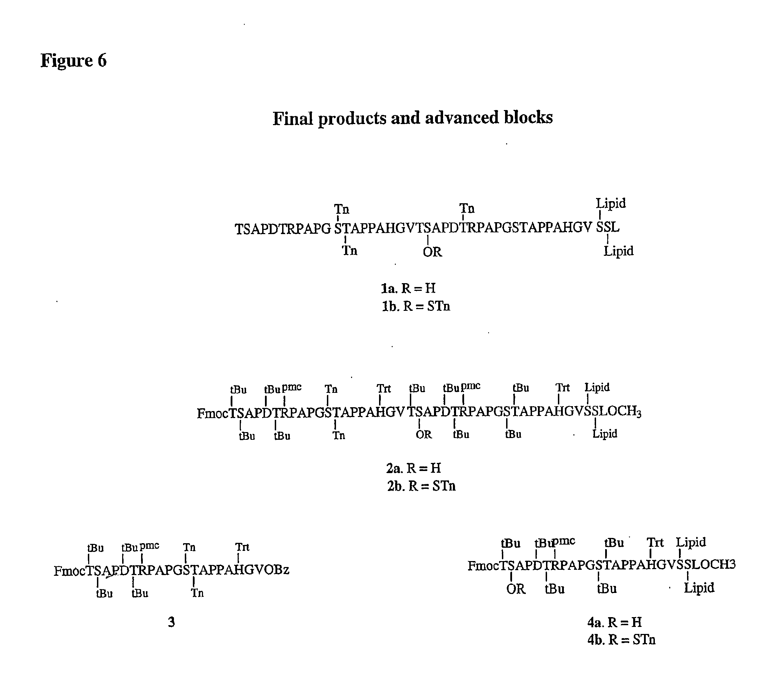

Figure 6: Final peptide blocks (SEQ ID NO:2) and deblocked products.

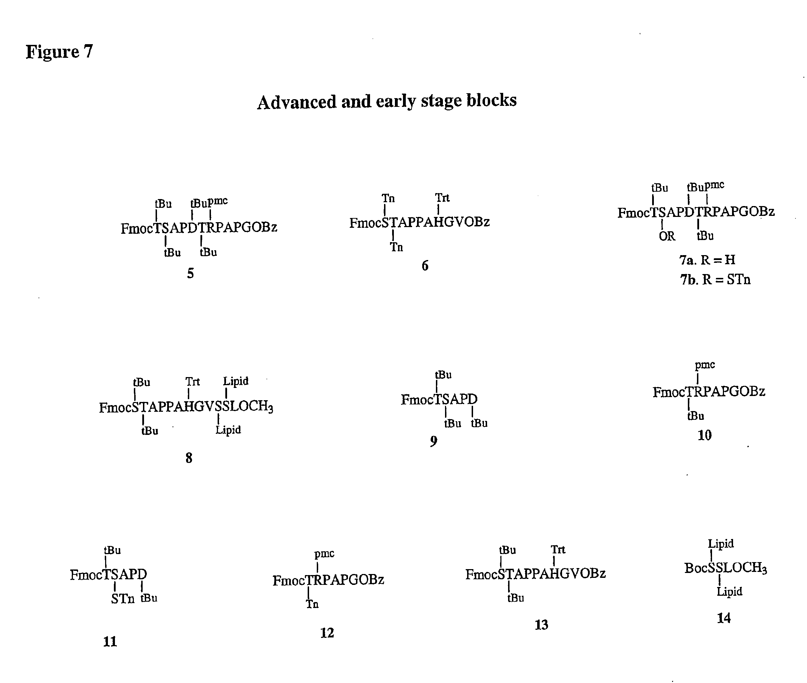

Figure 7: Primary and intermediate blocks that are used in the coupling sequence.
   All blocks of Figures 6 and 7 have peptide sequences which are subsequences of SEQ ID NO:2, as follows: 3 (AAs 1-20), 4 (AAs 21-43), 5 (AAs 1-11), 6 (AAs 12-20), 7a, 7b (AAs 21-31), 8 (AAs 32-43), 9 (AAs 1-5), 10 (AAs 6-11), 11 (AAs 21-25), 12 (AAs 26-31), 13 (AAs 32-40), and 14 (AAs 41-43).

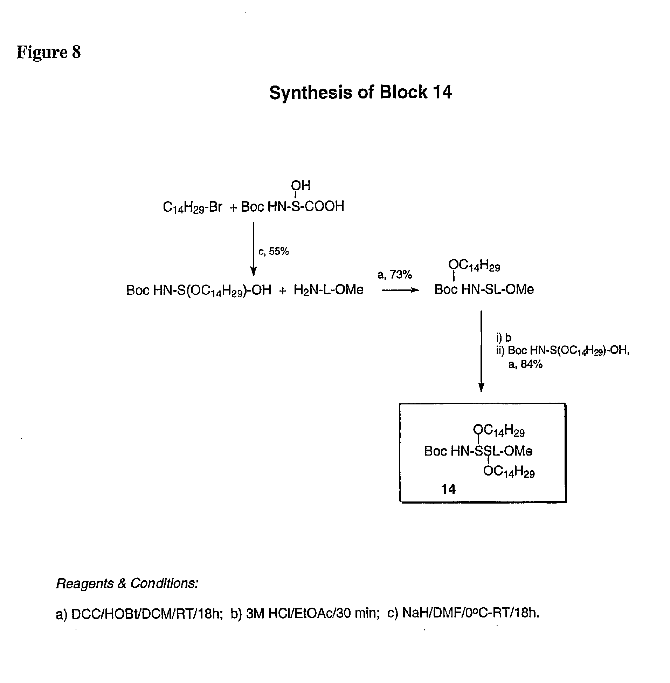

Figure 8: Scheme for the synthesis of lipid block 14.

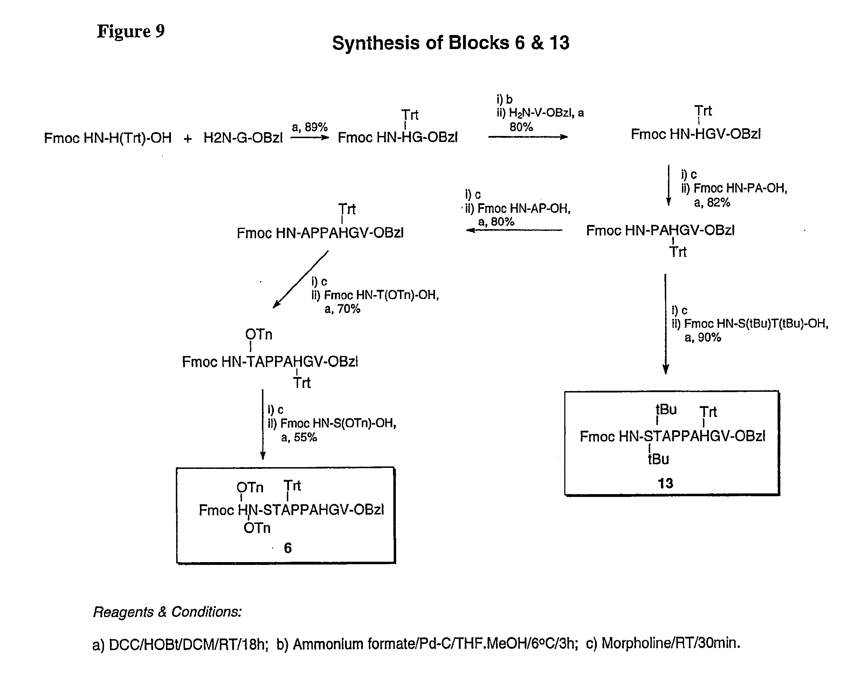

Figure 9: Scheme for the synthesis of peptide blocks 6 and 13. APPAHGV is AAs 14-20 of SEQ ID NO:2. PAHGV is AAs 16-20 of SEQ ID NO:2. TAPPAHGV is AAs 13-20 of SEQ ID NO:2. Compounds 6 and 13 were previously discussed.

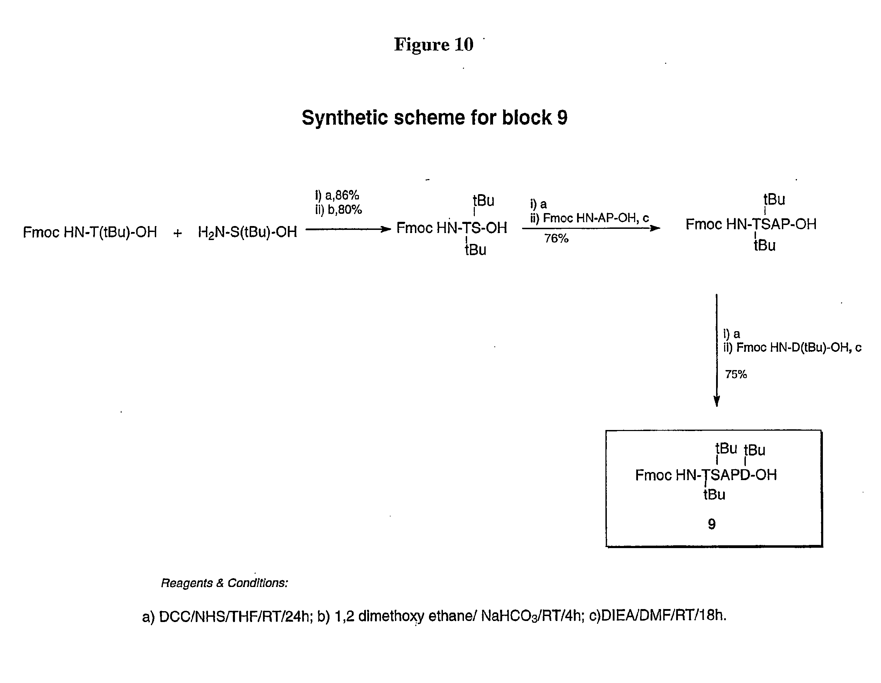

Figure 10: Synthetic scheme for block 9, AAs 1-5 of SEQ ID NO:2.

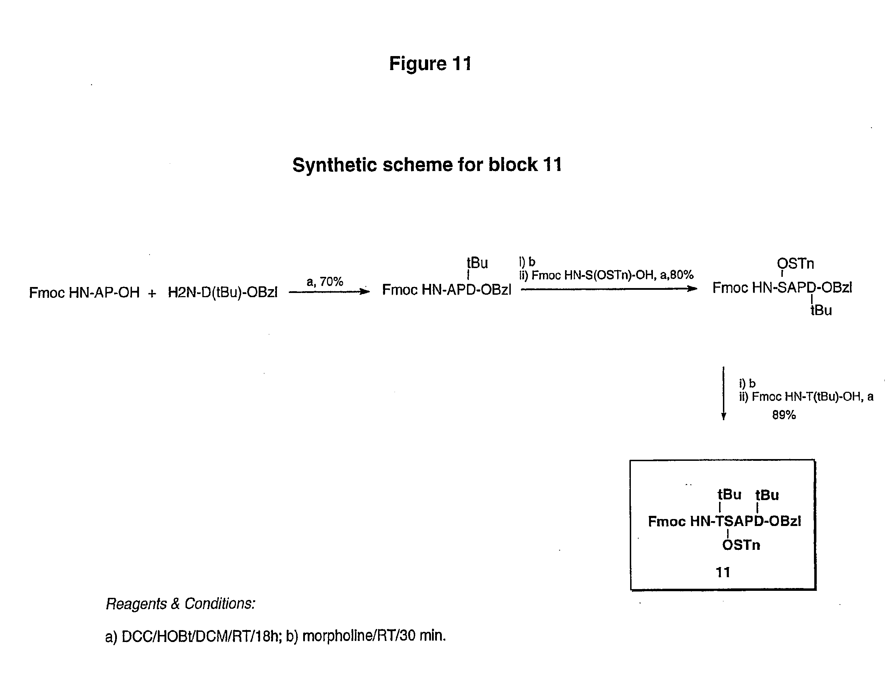

Figure 11: Synthetic scheme for block 11, As 21-25 of SEQ ID NO:2.

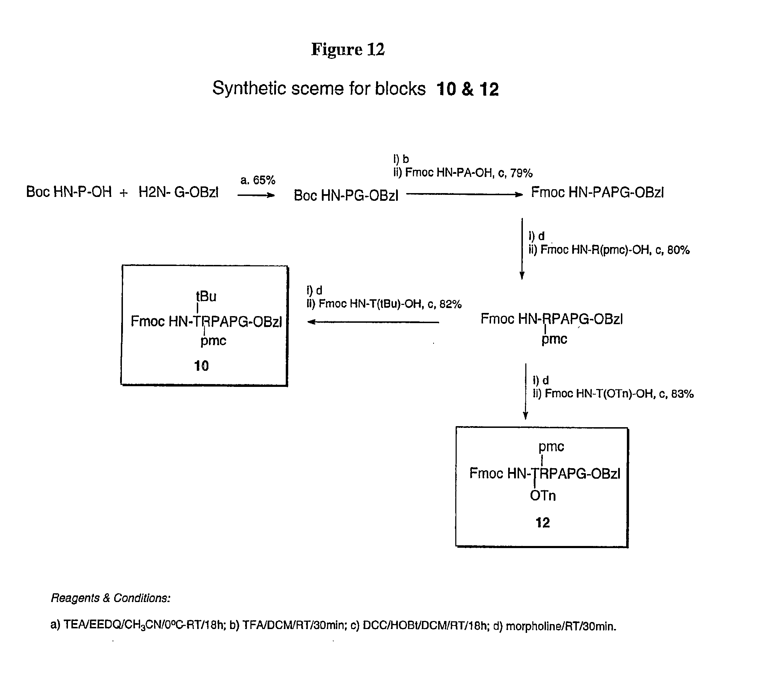

Figure 12: Synthetic scheme for blocks 10 (AAs 6-11) and 12, (AAs 26-31 of SEQ ID NO:2).

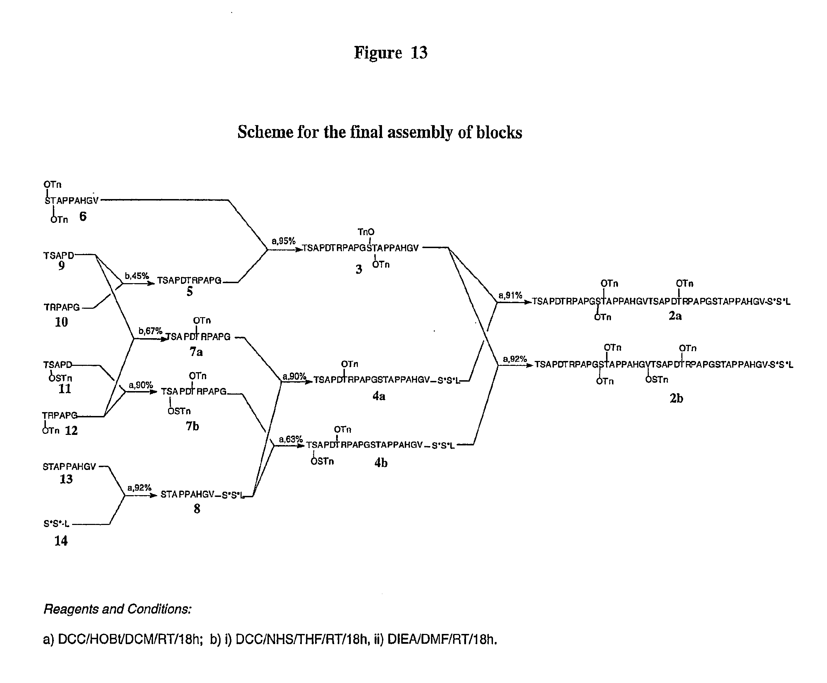

Figure 13: Scheme showing simultaneous synthesis of the two glyco-lipo-peptides 1 and 2 (SEQ ID NO:2), using common blocks strategy.

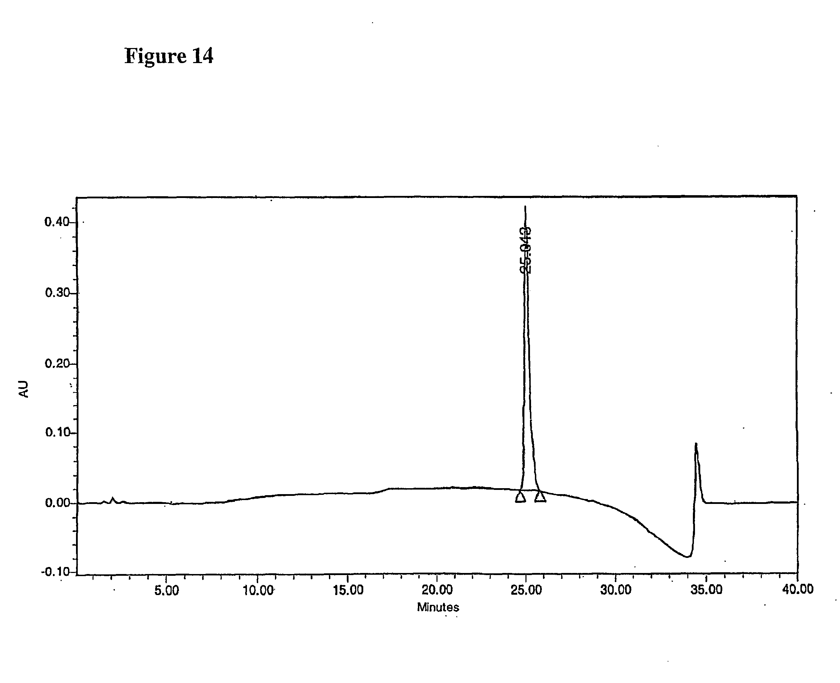

Figure 14: HPLC chromatogram of glyco-lipo-peptide 1a.

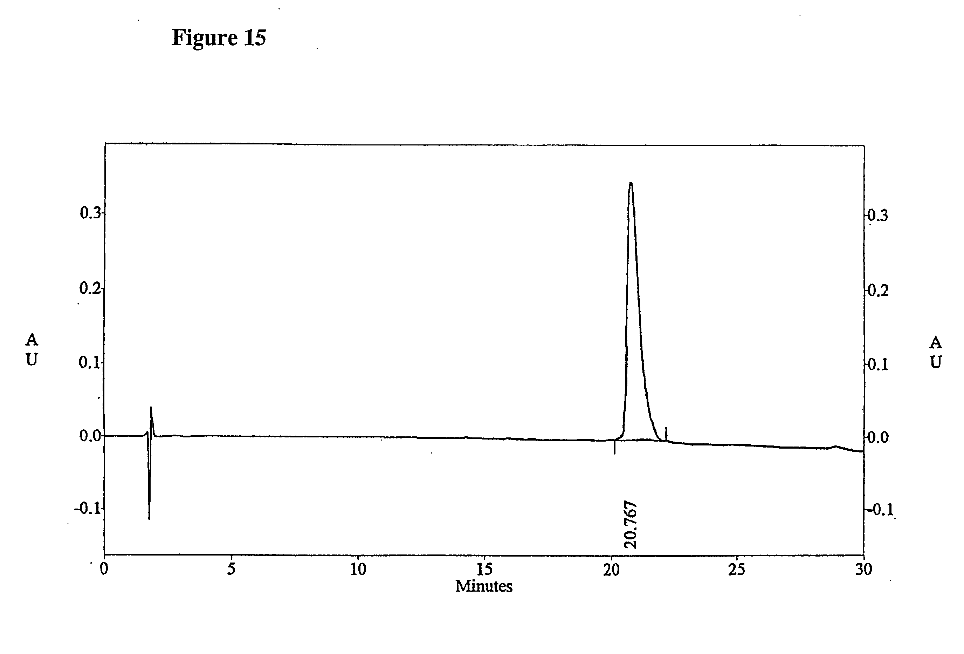

Figure 15: HPLC chromatogram of glyco-lipo-peptide 2b.

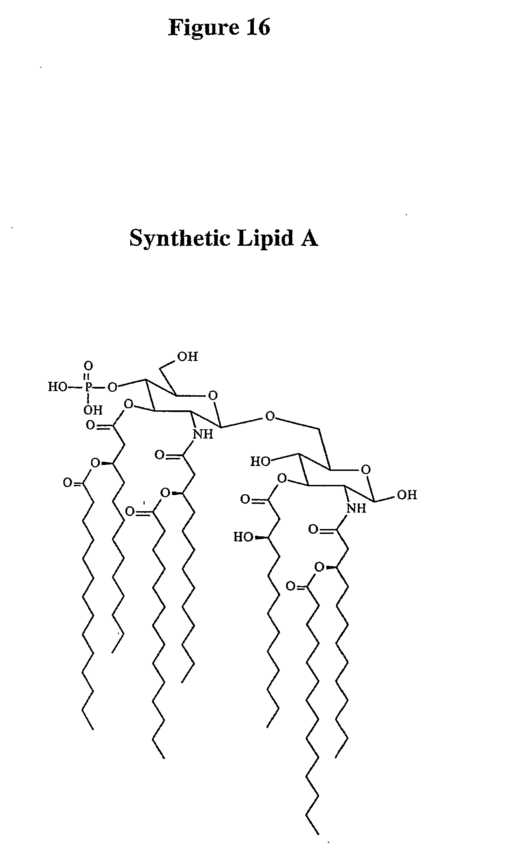

Figure 16: Structure of synthetic lipid A used in the liposome formulations.

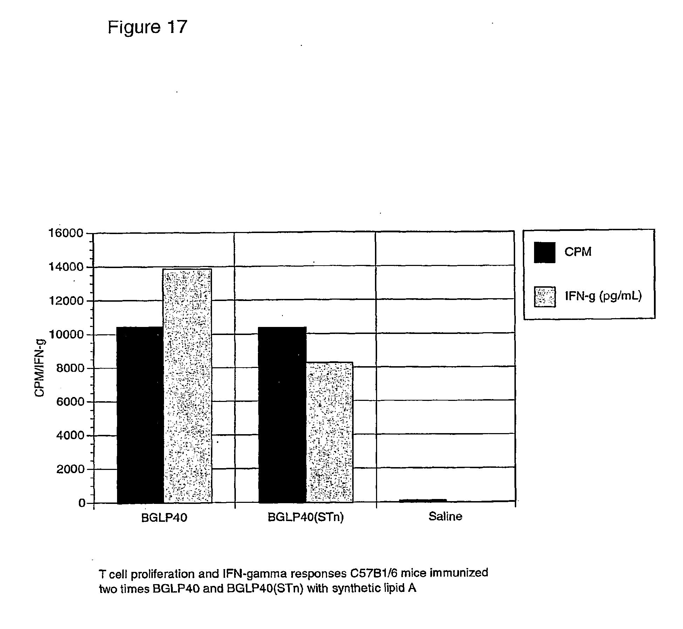

Figure 17: T-Cell proliferation and Interferon-g (IFN-g) responses in C57B1/6 mice immunised with liposomal formulations of glyco-lipo-peptide 1 and 2.

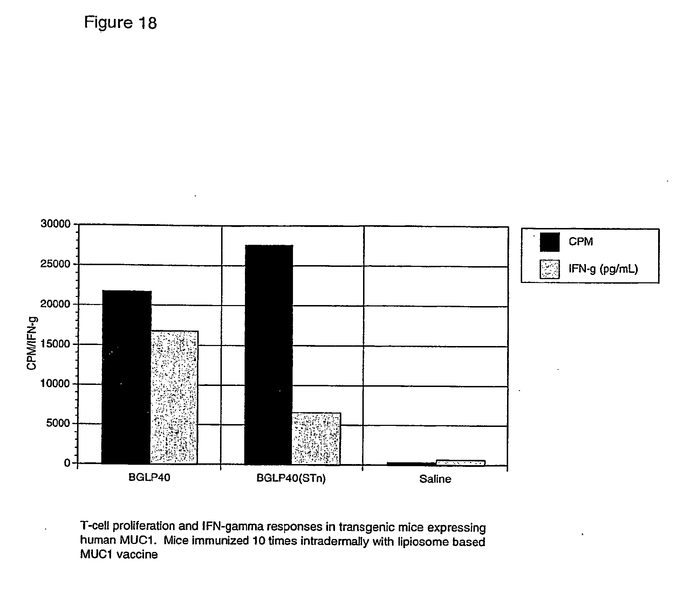

Figure 18: T-Cell proliferation and Interferon-g (IFN-g) responses in mice transfected with human MUC1 gene, immunised with liposomal formulations of glyco-lipo-peptide 1 and 2.

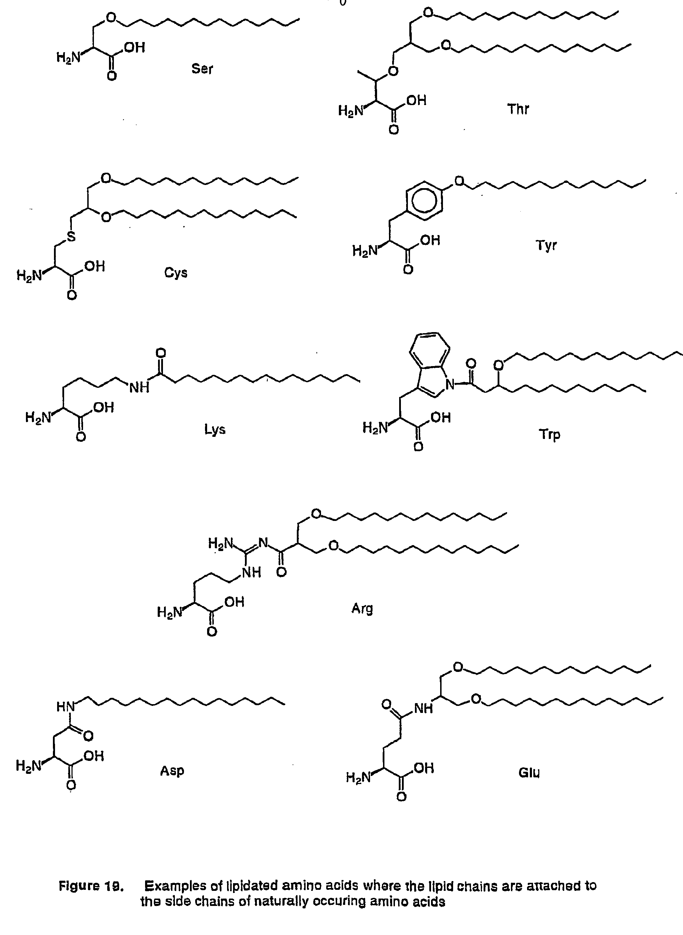

Figure 19: Examples of lipidated amino acids where the lipid chains are attached to the side chains of naturally occurring amino acids.

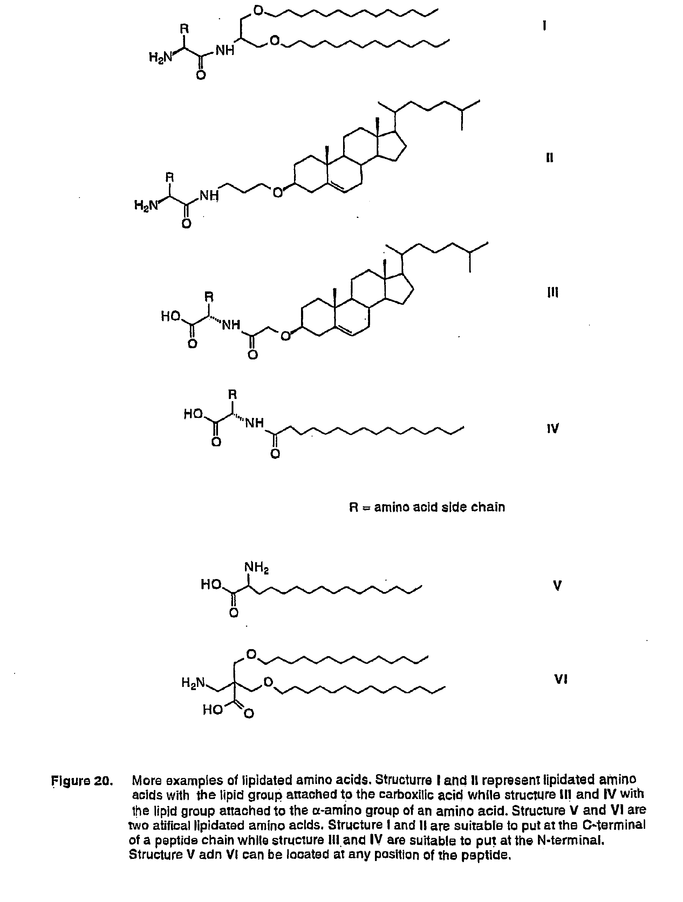

Figure 20: More examples of lipidated amino acids.
   Structures I and II represent examples where the lipids are attached to the carboxylic acid or C-terminus while II and IV are examples of lipids attached to the amino or N-terminus. Structures V and VI are lipidated non-natural amino acids. Structures I and II are designed for C-terminus, structures III and IV for N-terminus and V and VI may be located at any place of a peptide sequence.

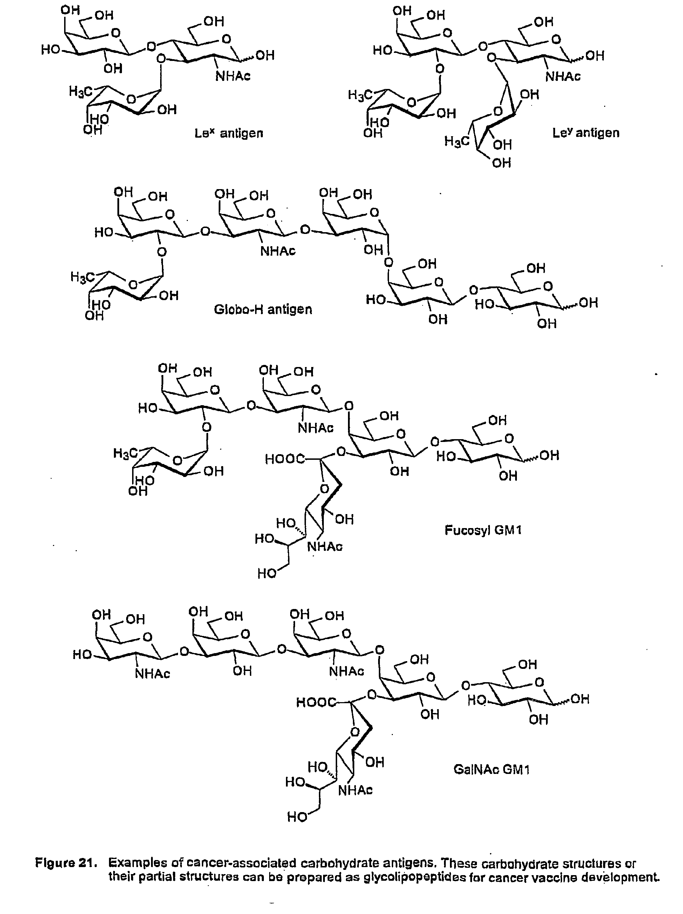

Figure 21: Examples of cancer associated carbohydrate antigens. These carbohydrate structures or their partial structures may be prepared as glycolipopeptides for cancer vaccine development.

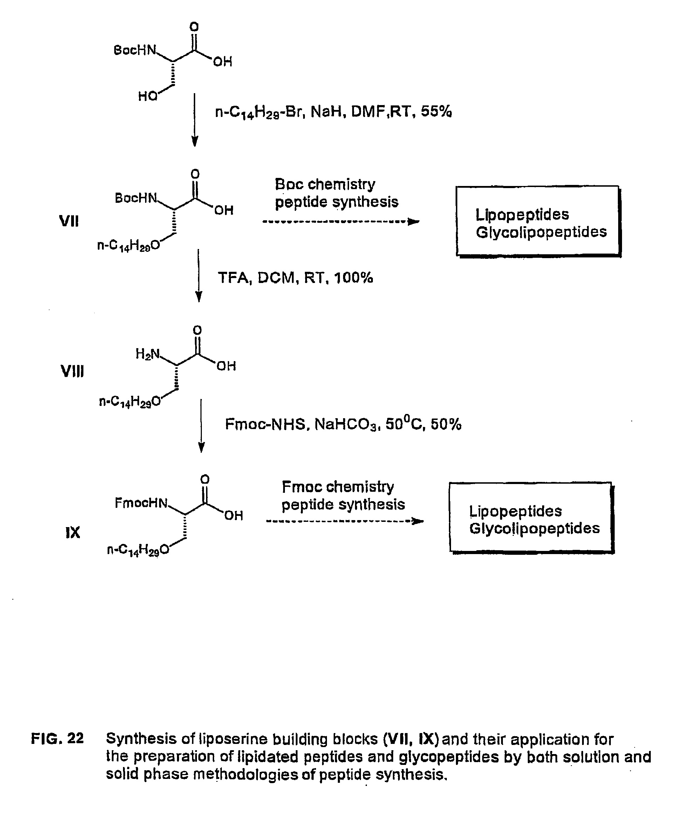

Figure 22: Synthesis of liposerine building blocks (VII, IX) and their application for the preparation of lipidated peptides and glycopeptides by both solution and solid phase methodologies of peptide synthesis.

In the figures and claims, "R" is used both as a standard symbol for the amino acid residue arginine, and as a group denoting a side chain attached to a particular residue.

### DETAILED DESCRIPTION OF THE PREFERRED EMBODIMENTS OF THE INVENTION

One aspect of the invention relates to a non-naturally occurring glycolipopeptide comprising at least one amino acid being a glycosylated amino acid and at least one amino acid being a lipidated amino acid, wherein at least one lipidated amino acid is an interior amino acid, wherein said glycolipopeptide comprises at least two MUC1 epitopes with the amino acid sequence P(D/E)(T/S)RP, and wherein
i) at least one MUC1 epitope with the amino acid sequence P(D/E)(T/S)RP comprises at least one glycosylated amino acid, and
ii) at least one MUC1 epitope with the amino acid sequence P(D/E)(T/S)RP comprises no glycosylated amino acid, and
wherein the amino acid sequence of said MUC1 epitopes may be the same or different.

Another aspect of the invention relates to the glycolipopeptide of the invention for use as a medicament.

Another aspect of the invention relates to use of the glycolipopeptide of the invention for the manufacture of a medicament for eliciting an immune response upon administration to a subject.

Another aspect of the invention relates to the glycolipopeptide of the invention for use in a method for eliciting an immune response upon administration to a subject.

Another aspect of the invention relates to a composition comprising a glycolipopeptide of the invention and a liposome.

Another aspect of the invention relates to a composition of the invention for use as a medicament.

Another aspect of the invention relates to use of a composition of the invention for the manufacture of a medicament for eliciting an immune response upon administration to a subject.

Another aspect of the invention relates to a composition of the invention for use in a method for eliciting and immune response in a subject.

### Glycolipopeptides

The glycolipopeptides of the present invention comprise at least five amino acids, at least one being glycosylated and at least one being lipidated.

Preferably, at least two amino acids are lipidated. Preferably, at least one lipidated amino acid is an interior amino acid, i.e., not the first or last amino acid of the peptide moiety. More preferably, at least two lipidated amino acids are interior amino acids. Most preferably, all of the lipidated amino acids are interior amino acids.

Preferably, at least two amino acids are glycosylated. More preferably, there are at least two glycosylated amino acids and at least two lipidated amino acids.

Preferably, the glycolipopeptide comprises at least one MUC1 peptide epitope, more preferably, PDTRP (AAs 6-10 of SEQ ID NO:10). If so, the lipidated amino acid may be a terminal amino acid or an interior amino acid. If there are two lipidated amino acids, they may both be terminal amino acids, both interior amino acids, or one of each.

Preferably, at least one glycosylated amino acid provides at least one carbohydrate epitope. More preferably, at least one carbohydrate epitope is Tn or sialyl Tn. Still more preferably, both unsialylated Tn and sialyl Tn are provided (attached to different amino acids).

Preferably, there are not more than 200 amino acids, more preferably not more than 150 amino acids, still more preferably not more than 100 amino acids, even more preferably not more than 75 amino acids, most preferably not more than 50 amino acids. In terms of molecular weight, they are preferably not more than 20,000 daltons, more preferably not more than 15,000 daltons, still more preferably not more than 10,000 daltons, even more preferably not more than 7,500 daltons, most preferably not more than 5,000 daltons.

The glycolipopeptides further comprise at least one disease-associated epitope, which may be a B-cell epitope (antibody-recognized) or a T-cell epitope. Preferably the epitope is disease-associated, more preferably disease-specific. Tumor-associated, especially, tumor-specific, epitopes are of particular interest.

Preferably, the glycolipopeptides comprise at least one B-cell epitope and at least one T-cell epitope. Preferably, at least one glycosylated amino acid is included in at least one of said epitopes.

### Lipidated Amino Acids

The glycolipopeptide, by definition, comprises one or more lipidated amino acids. A lipidated amino acid is an amino acid, other than a glycosylated amino acid, which comprises a strongly lipophilic group, as defined below. Thus, the glycolipopeptide may be a glycomonolipopeptide (one lipidated amino acid), a glycodilipopeptide (two lipidated amino acids), a glycotrilipopeptide (three lipidated amino acids), etc. Preferably, at least two of the amino acids are lipidated amino acids. Still more preferably, exactly two amino acids are lipidated amino acids.

In the case of an interior lipidated alpha amino acid, the side chain will comprise the strongly lipophilic group. In the case of a terminal lipidated alpha amino acid, either the side chain or the terminal moiety, or both, may comprise a strongly lipophilic group.

If the amino acid is not an alpha amino acid, then it may comprise more than one side chain, in which case it is possible for more than one side chain of the same amino acid to comprise a strongly lipophilic group.

If a side chain comprises a strongly lipophilic group, then, in a preferred embodiment, the side chain itself qualifies as a strongly lipophilic group.

The lipidated amino acid may be a derivative of one of the twenty genetically encoded amino acids, said derivative characterized by a lipidated side chain, especially as follows:

| Lipidated AA | Lipidated Side Chain |
|---|---|
| Ser | -CH₂OZ |
| Thr | -CH (CH₃) OZ |
| Asp | -CH₂-C(=O) OZ |
| Glu | -(CH₂)₂-C(=O) OZ |
| Cys | -CH₂SZ |
| Tyr | -CH2-Phenyl-OZ |
| Lys | - (CH₂) ₄ NHZ |
| Arg | - (CH₂) ₃-NH-C (=NH) -NHZ |
| Asn | -CH₂-C (=O) -NHZ |
| Gln | - (CH₂)_{z}-C (=O) -NHZ |

where Z is a strongly lipophilic group. (The resulting lipidated amino acid is itself, of course, a non-genetically encoded amino acid.)

The lipidated amino acid may instead be characterized by a lipidated side chain which is a derivative of the side chain of a non-genetically encoded amino acid. The latter may be a homologue or other analogue of a genetically encoded amino acid. Thus, the side chain may be, e.g.,
- (CH₂)ₘOR, m>1 (related to Ser, Thr)
- (CH₂)ₘSR, m>1 (related to Cys)
- (CH₂)ₘNHR, m≠4 (related to Lys)
- (CH₂)ₘ-C(=O)NHR, m>2 (related to Asn, Gln)
- (CH₂)ₘ-C(=O)OR, m>2 (related to Asp, Glu)

A terminal amino acid may alternatively or additionally be characterized by a- lipidated amino terminal, if an amino terminal residue (R-NH-AA), or a lipidated carboxy terminal, if a carboxy terminal residue (AA-C(=O)-OR).

The side chains of the genetically encoded amino acids may be classified as lipophilic (hydrophobic), lipophobic (hydrophilic), or neutral. The lipophilicity of these side chains, as well as the side chains of non-genetically encoded amino acids, may be determined by measuring the partition coefficient of the molecule HZ (where Z is the side chain in question) between a nonpolar solvent (e.g., ethanol, dioxane, acetone, benzene, n-octanol) and water, at STP. The lipophilicity may be defined as the logarithm of this partition coefficient; it will then be positive for molecules which prefer the nonpolar solvent. Thus, a lipophilic group is one for which logP is greater than zero.

The partition coefficient (P) is defined as the ratio of the equilibrium concentrations of a dissolved substance in a two-phase system consisting of two largely immiscible solvents. One such system is n-octanol:water; the octanol phase will contain about 20% water and the water phase about 0.008% octanol. Thus, the relevant partition coefficient (Pow) is the ratio of the molar concentration of the solute in octanol saturated with water to its molar concentration in water saturated with octanol. N-octanol is a useful surrogate for biological membranes because it, like many membrane components, is amphiphilic. (Reference hereafter to log P shall mean log Pow, unless otherwise stated.)

For the purpose of this disclosure, a strongly lipophilic group is defined as being a group, comprising at least five atoms other than hydrogen, for which the lipophilicity calculated as the log of the n-octanol:water partition coefficient, by any of the three art-recognized methods set forth below as (A) - (C) is greater than that calculated for any of the side chains of the genetically encoded amino acids (hereafter, the reference side chains). The genetically encoded amino acids with lipophilic side chains are the aliphatic amino acids alanine, valine, leucine, isoleucine, and methionine, and the aromatic amino acids tryptophan, tyrosine and phenylalanine.

It follows from the definition of "strongly lipophilic" that the glycolipopeptides of the invention must comprise at least one non-genetically encoded amino acid, e.g., a lipidated serine or threonine.

In one embodiment, the group in question is more lipophilic than any of the reference side chains when its lipophilicity, and that of the reference side chains, is determined according to method (A) below.

In a second embodiment, the group in question is more lipophilic than any of the reference side chains when its lipophilicity, and that of the reference side chains, is determined according to method (B) below.

In a third embodiment, the group in question is more lipophilic than any of the reference side chains when its lipophilicity, and that of the reference side chains, is determined according to method (C) below.

Methods (A)-(C) are as follows:
(A) for predicted log Pow values of 0 to 4, the shake flask method set forth in EPA product Properties Test Guidelines OPPTS 830.7550 EPA 712-c-96-038 (August 1996) (Note that negative log Pow values imply that the compound is not lipophilic at all.)
(B) for predicted log Pow values of 4 to 6, the liquid chromatography estimation method set forth in the EPA Product Properties Test Guidelines OPP13 830.7570, EPA 712-C-96-040 (August 1996). (This method may be used for estimating Pow values of 0 to 6.)
(C) for predicted log Pow values higher than 6, the predictive method described in Meylan, et al., Atom/fragment contribution method for estimating octanol-water partition coefficients", J. Pharm. Sci., 84: 83-92 (1995). (note that if predicted log Pow values are higher than 6, so experimental determination is necessary).

In Meylan's method, the predicted log Pow is obtained by adding weighted coefficients for each fragment (the raw coefficient multiplied by the number of copies of that fragment) to the constant 0.2290. The fragments considered include -CH3 (0.5473), -CH2- (0.4911), -CH (0.3614), -OH (-1.4086), -NH2 (-1.4148), -C(=O)N (-0.5236), -SH (-0.0001), -NH-(-1.4962), -N=C (-0.0010), -O- (-1.2566), ALDEHYDE -cho (-0.9422), -tert C so 3+ C attached (0.2676), C no H not tert (0.9723), aromatic C (0.2940), aromatic N (5 membered ring) (-0.5262), and aromatically attached -OH (-0.4802); all aliphatic or aliphatically attached unless otherwise stated.

In a fourth embodiment, the group in question is more lipophilic than any of the reference side chains when its lipophilicity, and that of the reference side chains, are determined in accordance with a preferred method of determining the partition coefficient, which method is chosen on the basis of the predicted log Pow value (this predicted value is itself determined by method (C):
(1) for predicted log Pow values of 0 to 4, method (A),
(2) for predicted low Pow values of 4 to 6, method (B), and
(3) for predicted low Pow values greater than 6, method (C).

For more information on methods of determining Pow, see Sangster, J., Octanol-Water Partition Coefficients: Fundamentals and Physical Chemistry (April 1997) (ISBN 0-471-9739).

For tabulations of octanol-water partition coefficients, see the EPA "Chemicals in the Environment: OPPT Chemicals Fact Sheets" the USDA Pesticide Properties Database, Sangster, J., "Octanol-Water Partition Coefficients of Simple Organic Compounds", J. Phys. Chem. Ref. Data, 18:1111-1230 (1989); Verbruggen, E.M.J., et al., "Physiochemical Properties of Higher Nonaromatic Hydrocarbons: Literature Study," J. Phys. Chem. Ref. Data, 29:1435-46 (2000). For more sources, see references cited at Penn State University Libraries, Physical Sciences Library, octanol-water Partition Coefficients (last updated August 21, 2001), at the URL libraries.psu.edu/crsweb/physci/coefficients.htm. It should be noted that the Pow values compiled for different compounds may have been determined by different methodologics.

The Meylan algorithm is implemented in the program LogPow (KowWin). An online version of the program, available at esc.syrres.com/interkow/kowdemo.htm accepts either CAS registry numbers or SMILES structure notations. The program also reports experimentally determined values, if in its database.

A group is expected to be a lipophilic group if its logP, as predicted by the Meylan algorithm, is greater than zero. Preferably, the logP predicted by the Meylan algorithm is at least 1, at least 2, at least 3, at least 4, at least 5, at least 6, at least 7, at least 8, at least 9, or at least 10, the higher the more preferred.

At least one lipophilic group is preferably a "highly lipophilic (Meylan) group". For the purpose of this disclosure, a "highly lipophilic (Meylan) group" is defined as one for which the lipophilicity calculated by the Meylan algorithm is at least 2.7. The highest logP predicted by the Meylan algorithm for the side chains of the genetically encoded amino acids is 2.60 (Trp); the highest experimentally determined logP for the same side chains is 2.89 (Ile). It should be noted that most "strongly lipophilic groups" will also be "highly lipophilic (Meylan) groups," and vice versa.

An alpha amino acid has one side chain, if any (glycine lacks a side chain), and hence a lipidated interior alpha amino acid necessarily comprises a single strongly lipophilic group. Non-alpha amino acids have more than one possible side chain attachment site. In the case of a lipidated interior non-alpha amino acid, if the amino acid has more than one side chain, at least one of the side chains comprises a strongly lipophilic group.

Preferably, the strongly lipophilic group will comprise not more than 100 atoms other than hydrogen, more preferably, not more than 80 such atoms, still more preferably, not more than 60 such atoms, even more preferably not more than 40 such atoms.

As noted previously, the strongly lipophilic group must comprise at least five atoms other than hydrogen (the side chains of leucine and isoleucine have four such atoms). Preferably, it comprises at least six (side chain of Lys is five), more preferably at least 8 (side chains of Arg and Phe are 7), still more preferably at least 9 (Tyr has 8), even preferably, it comprises at least 11 such atoms (tryptophan has 10), still more preferably at least 13 such atoms, most preferably at least 21 such atoms.

Preferably, the strongly lipophilic group has an elemental composition limited to the elements carbon, silicon, hydrogen, oxygen, nitrogen, sulfur, and phosphorous. Preferably, the majority of the bonds within the side chain which do not involve hydrogen are carbon-carbon bonds.

Since the presence of oxygen, nitrogen, sulfur and phosphorous tends to reduce lipophilicity, in the strongly lipophilic group, preferably more than 50%, still more preferably more than 75%, of the non-hydrogen atoms are carbon atoms.

For the same reason, the strongly lipophilic group preferably comprises at least 5, at least 6, at least 7, at least 8, at least 9, or at least 10 carbon atoms.

Preferably, the side chain is of the general form -A-Y-Z, where -A- is optional, but, if present, is an organic group of not more than 12 atoms other than hydrogen; Y is a spacer of not more than 12 atoms other than hydrogen, comprising nitrogen, oxygen, sulfur or phosphorous, and Z is a strongly lipophilic group.

The A, if present, is preferably aliphatic. More preferably, it is alkyl. Preferably, it is not more than 10, more preferably, not more than 8, atoms other than hydrogen. Most preferably, if present it is alkyl of 1-8 carbon atoms.

The spacer Y preferably comprises at least one moiety selected from the group consisting of -O-, -S-, -NH-, -NR-(where R is 1-4 alkyl), -RO₄-, C(=O), -C(=S), -C(=NH)-, and - C(=NR)-. More preferably, the spacer consists of one or two such moieties. The most preferred spacers are -O-, -S-, -NH-, -C(=O)- and -C(=S)-.

Preferably, the radicals -Y-Z and/or -A-Y-Z qualify as strongly lipophilic moieties in their own right.

The strongly lipophilic group may be entirely an aliphatic moiety or moieties, entirely an aromatic moiety or moieties, or a combination of at least one aliphatic moiety and at least one aromatic moiety. An aliphatic moiety is preferred.

Each aliphatic moiety may, independently, be linear, cyclic, a combination of linear and cyclic, branched but acyclic, or branched but with one or more branches comprising a cyclic moiety. It also may be saturated or unsaturated. If saturated, there may be one or more double and/or one or more triple bonds. The aliphatic moieties, such as those of -Z, may, independently, comprise one or more spacers, which preferably are selected from the group defined above.

The strongly lipophilic group.Z may itself comprise one or more moieties of the form -A'-Y'-Z', where A' , Y' and Z' are defined analogously to A, Y and Z.

It may be of the linear form
-A' (-Y'-Z'-)ₙ where n>1,
e.g., -A'-Y'-Z'-Y'-Z', where each Y' and each Z' is chosen independently.

In one preferred embodiment, the strongly lipophilic group Z comprises an aliphatic moiety of the form -A-O-Q, where A is optional and is an alkyl group, and Q is an alkyl group. More preferably, A is -(CH₂)ᵢ-, where i is 0 or 1, and Q is -(CH₂); Ch3, where j is 6-26.

In another preferred embodiment, the strongly lipophilic group comprises one or more fatty acid moieties. Thus, at least one Z (or Z') comprises a fatty acid group of the form -O-CO-Q, where Q is primarily alkyl but may include alkenyl, alkynyl, or ether linkages. The fatty acids are carboxylic acids, often derived from or contained in an animal or vegetable fat or oil. All fatty acids are composed of a chain of hydrocarbon groups containing from 4 to 22 carbon atoms and characterized by a terminal carboxyl radical. They may be designated by "the number of carbon atoms: number of double bonds", and optionally the locations of cis/trans isomerism. Thus, suitable fatty acids include those with designations 4:0, 6:0, 8:0, 10:0, 12:0, 14:0, 16:0, 16:1(9c), 18:0, 18:1 (9c), 18:2 (9c, 12c), 18:3 (9c, 12c, 15c), 18:4 (6c, 9c, 12c, 15c), 18:3 (9c, 11t, 13t), 18:1 (9c) 12-OH, 20:1 (9c), 20:1 (11c), 20:4 (8c, 11c, 14c, 17c), 20:5 (5c, 8c, 11c, 14c, 17c), 22:0, 22:1 (11c), 22:1 (13c), 22:5 (7c, 10c, 13c, 16c, 19c) and 22:6 (4c, 7c, 10c, 13c, 16c, 19c), all of which are found in naturally occurring glycosides.

If the strongly lipophilic group comprises a plurality of cyclic moieties, they may be fused (forming a polycyclic moiety) or unfused, and may have the same or a different number of sides. Typically, they will each have 3-6 sides. One or more of the sides may be a double or triple bond. The cyclic moieties may be heterocyclic in character. In one preferred embodiment, the side chain comprises a steroid moiety. This is a polycyclic moiety with four fused rings, one five-sided and three six-sided.

The aliphatic moieties may comprise one or more phosphoryl groups, and, if they do so, the number of such groups in the side chain is preferably not more than two. Phosphoryl groups are found in the lipids of bacterial membranes, e.g., the lipid monophosphoryl lipid A (MPLA).

The aromatic moieties may comprise one or more rings. If there is more than one ring, the rings may be fused or unfused.

The strongly lipophilic group may comprise a branched structure, preferably aliphatic in nature, such as

-B (-Y'-Z')ₙ

where B is a branched organic group with a valence of at least 2, otherwise defined similarly to A and A', n is at least 2, and Y' and Z' are as previously defined, but each Y' and each Z' may be chosen independently for each -Y'-Z' branch attached to B. More preferably n is 2 or 3.

Preferably, each branch -Y'-Z' itself qualifies as a strongly lipophilic group.

In an especially preferred embodiment, each Y' is -O- and each Z' is (CH₂)ⱼCH₃, where j = 6 to 26 (chosen independently for each branch). If n=2, then B is most preferably -CH(CH₂-)₂. If n=3, then B is more preferably -C(CH₂-)₃.

Using the program LogKow, we have calculated (see below) low Pow values for the side chains of several genetically encoded amino acids, as well as for several preferred side chains. Where LogKow also provided an experimental database, this is also given below.

| Compound | SMILES (lower case is arom) | Comments | LoqP | |
|---|---|---|---|---|
| | | | Pred | Exp |
| n-butane | CCCC | Ile side chain | 2.31 | 2.89 |
| 2-methyl propane | CC(C)C | Leu side chain | 2.23 | 2.76 |
| toluene | Cclcccccl | Phe side chain | 2.54 | 2.73 |
| p-cresol | Cclccc(O)ccl | Tyr side chain | 2.06 | 1.94 |
| 3-methyl indole | Cclcnc2ccccc12 | Trp side chain | 2.60 | 2.60 |
| n-pentane | CCCCC | one more c than Leu | 2.80 | 3.39 |
| - | COCCCCC CCCC CCCCC MW228.42 | side chain of lipidated AAs in compound 1a | 6.45 | ? |
| - | COCCCCC CCCCC CCCCC CC | 2 longer than above | 7.43 | ? |
| - | COCCCCC CCCCC CCC | 2 shorter than in 1a | 5.47 | ? |
| - | CO CCCCC CC | shortest preferred side chain of this type | 3.01 | ? |
| - | CO CCCCC CCCCC CCCCC CCCCC CCCCC CC MW410.77 | 33 longest preferred side chain of this type | 12.84 | ? |
| methyl n-butyl ether | CO CCCC | related compound with known logP; listed for comparison only | 1.54 | 1.66 |
| - | CCO CCCCC CCCCC CCCCC | as for 1a but methylated beta-carbon | 7.43 | ? |
| - | O=CC (COCCCCCCC) COCCCCCCC | -CH(=O)CH< linked diether with m=n=6 | 5.11 | ? |
| - | - MW 524.92 | -C^{H}(=O)CH< linked diether with m=n=14 | 12.96 | ? |
| - | - MW 861.57 | -CH(=0)CH< linked diether with m=n=26 | 24.75 | ? |
| - | NC (COCCCCCCC) COCCCCCCC | -NHCH< linked diether with m=n=6 | 5.23 | ? |
| - | - | -NHCH< linked diether with m=n=14 | 13.09 | ? |
| - | - MW 848.57 | -NHCH< linked diether with m=n=26 | 24.88 | ? |
| - | C (COCCCCCCC) COCCCCCCC | -CH< linked diether with m=n=6 | 6.18 | ? |
| | - | -CH< linked diether with m=n=14 | 14.03 | ? |
| - | - MW833.56 | -CH<linked diether with m=n=26 | 25.82 | ? |
| - | - | triether m=n=k=6 | 8.78 | ? |

### Relation of Lipidated Amino Acids to Liposomal Formulation

The lipidation normally will facilitate the incorporation of the immunogen into a liposome, which in turn can improve the immune presentation of the immunogen. For most efficient incorporation, the strongly lipophilic side chain of the lipidated amino acid preferably should be similar in size to at least one of the lipid components of the liposome. For example, the size should be in the range of 50%-200% of the size of the reference lipid component of the liposome. Size may be measured by counting the number of non-hydrogen atoms of each, by calculating the molecular weight of each, or by calculating (with the aid of 3D molecular models) the molecular volume or longest dimension of each.

Preferably, at least one of the lipidated amino acids comprises a lipophilic moiety which adjuvants the humoral or cellular immune response to the glycolipopeptide.

### Glycosylated Amino Acids

A glycolipopeptide, by definition, comprises one or more glycosylated amino acids. Preferably, at least one of the glycosylated amino acids comprises a disease-associated carbohydrate epitope, such as a tumor-associated carbohydrate epitope.

A common co- or post-translational modification of proteins is the glycosylation of certain amino acids. Glycosylation is the covalent attachment of one or more carbohydrate units to an amino acid.

For the purpose of the claims, a glycosylated amino acid is one whose side chain comprises at least one carbohydrate monomeric unit. It may comprise other aliphatic and/or aromatic moieties, too. A simply glycosylated amino acid is one whose side chain consists of a linker selected from the group consisting of -O-, -S-, and -NH-, and one or more carbohydrate units.

The monomer units which are most commonly attached to proteins in nature are glactose, mannose, glucose, N-acetylgucosamine, N-acetylgalactosamine, sialic acids, fucose, and xylose. The number of sugar units varies, it is typically anywhere from 1 to 20; preferably, it is 1 to 10. If an oligosaccharide chain (i.e., a chain of two or more sugar units) is attached, the chain may be linear or branched. In the case of a branched chain, the length of the longest linear sequence is usually 1 to 10 sugar units; more preferably, it is 1-5.

The most common attachments are -O- and -N- links. O-glycosylation, in nature, is of hydroxy-containing amino acids such as serine or threonine. Tyrosine can also be O-glycosylated. In collagen, there is an unusual amino acid, hyroxylysine (Hyl), that is O-glycosylated when it occurs in the sequence -Gly-Xaa-Hyl-Xaa-Arg-, where Xaa is any amino acid. Hydroxyproline can also be O-glycosylated.

N-glycosylation, in nature, is of amide-containing amino acid side chains, such as Asn, or of the amino terminal of a protein. It usually occurs at the Asn of the sequence -Asn-Xaa-Ser- or -Asn-Xaa-Thr-. In an N-link, the nitrogen may be unsubstituted (-NH-) or substituted (-NZ-).

S-glycosylation is also possible, e.g., of the thiol group of cysteine. This results in an -S- link.

Since the molecules of the present invention need not be biosynthesized, we are not limited to glycosylation of the genetically encoded amino acids.

The O-glycosylated amino acids of the present invention may be any amino acid with a side chain comprising the structure -C-O-sugar, where "sugar" denotes one or more saccharide units. The -C- may be -CH2-, or it may be further substituted. It may, but need not be, the alpha carbon of the amino acid.

Any hydroxy-containing amino acids may be O-glycosylated, including, but not limited to, serine, threonine, hydroxyproline, and hydroxylysine.

In like manner, the N-glycosylated amino acids of the present invention may be any amino acid with a side chain comprising the structure -N-sugar, where "sugar" denotes one or more saccharide units. The -N- may be -NH-, or it may be further substituted. It may, but need not be, attached to the alpha carbon of the amino acid.

Any amine-containing amino acids may be N-glycosylated, including but not limited to, asparagine. Any of the thiolcontaining amino acids may be S-glycosylated, including, but not limited to, cysteine.

The glycosylation can be performed before, during or after the synthesis of the peptide sequence. Preferably, all of the glycosylated amino acids are prepared first, and then incorporated into the peptide (or a block thereof).

A glycosylated amino acid may, but need not, comprise one or more strongly lipophilic groups.

### Amino acids and peptides

A peptide is composed of a plurality of amino acid residues joined together by peptidyl (-NHCO-) bonds. A biogenic peptide is a peptide in which the residues are all genetically encoded amino acid residues; it is not necessary that the biogenic peptide actually be produced by gene expression.

Amino acids are the basic building blocks with which peptides and proteins are constructed. Amino acids possess both an amino group (-NH₂) and a carboxylic acid group (-COOH). Many amino acids, but not all, have the alpha amino acid structure NH₂-CHR-COOH, where R is hydrogen, or any of a variety of functional groups.

Twenty amino acids are genetically encoded: Alanine, Arginine, Asparagine, Aspartic Acid, Cysteine, Glutamic Acid, Glutamine, Glycine, Histidine, Isoleucine, Leucine, Lysine, Methionine, Phenylalanine, Proline, Serine, Threonine, Tryptophan, Tyrosine, and Valine. Of these, all save Glycine are optically isomeric, however, only the L-form is found in humans. Nevertheless, the D-forms of these amino acids do have biological significance; D-Phe, for example, is a known analgesic.

Technically speaking, proline is not an amino acid at all, but rather, a cyclic imino acid, wherein the side chain - (CH₂) ₃- is linked, not only to the alpha carbon, but also to the peptide bond's amide nitrogen, forming a five-membered pyrrolidine ring. There is no amide hydrogen for proline residues. Following convention, if we refer to an "amino acid", it should be assumed to include proline (and substituted forms such as 3-hydroxyproline) unless these are expressly excluded.

Many other amino acids are also known, including: 2-Aminoadipic acid; 3-Aminoadipic acid; beta-Aminopropionic acid; 2-Aminobutyric acid; 4-Aminobutyric acid (Piperidinic acid);6-Aminocaproic acid; 2-Aminoheptanoic acid; 2-Aminoisobutyric acid, 3-Aminoisobutyric acid; 2-Aminopimelic acid; 2,4-Diaminobutyric acid; Desmosine; 2,2'-Diaminopimelic acid; 2,3-Diaminopropionic acid; N-Ethylglycine; N-Ethylasparagine; Hydroxylysine; allo-Hydroxylysine; 3-Hydroxyproline; 4-Hydroxyproline; Isodesmosine; allo-Isoleucine; N-Methylglycine (Sarcosine); N-Methylisoleucine; N-Methylvaline; Norvaline; Norleucine; and Ornithine.

Peptides are constructed by condensation of amino acids and/or smaller peptides. The amino group of one amino acid (or peptide) reacts with the carboxylic acid group of a second amino acid (or peptide) to form a peptide (-NHCO-) bond, releasing one molecule of water. Therefore, when an amino acid is incorporated into a peptide, it should, technically speaking, be referred to as an amino acid residue. The core of that residue is the moiety which excludes the -NH and -CO linking functionalities which connect it to other residues. This moiety consists of one or more main chain atoms (see below) and the attached side chains.

The main chain moiety of each AA consists of the -NH and -CO linking functionalities and a core main chain moiety. Usually the latter is a single carbon atom. However, the core main chain moiety may include additional carbon atoms, and may also include nitrogen, oxygen or sulfur atoms, which together form a single chain. In a preferred embodiment, the core main chain atoms consist solely of carbon atoms.

The side chains are attached to the core main chain atoms. For alpha amino acids, in which the side chain is attached to the alpha carbon, the C-1, C-2 and N-2 of each residue form the repeating unit of the main chain, the word "side chain" refers to the C-3 and higher numbered carbon atoms and their substituents. It also includes H atoms attached to the main chain atoms.

Amino acids may be classified according to the number of carbon atoms which appear in the main chain inbetween the carbonyl carbon and amino nitrogen atoms which participate in the peptide bonds. Among the 150 or so amino acids which occur in nature, alpha, beta, gamma and delta amino acids are known. These have 1-4 intermediary carbons. Only alpha amino acids occur in proteins. Proline is a special case of an alpha amino acid; its side chain also binds to the peptide bond nitrogen.

For beta and higher order amino acids, there is a choice as to which main chain core carbon a side chain other than H is attached to. The preferred attachment site is the C-2 (alpha) carbon, i.e., the one adjacent to the carboxyl carbon of the -CO linking functionality. It is also possible for more than one main chain atom to carry a side chain other than H. However, in a preferrred embodiment, only one main chain core atom carries a side chain other than H.

A main chain carbon atom may carry either one or two side chains; one is more common. A side chain may be attached to a main chain carbon atom by a single or a double bond; the former is more common.

### Immunogen

The immunogen of the present invention is a glycolipopeptide, as defined above, comprising at least one disease-associated B or T cell epitope, as defined below, and which, when suitably administered to a subject (which, in some cases, may mean associated with a liposome or with an antigen-presenting cell), elicits a humoral and/or cellular immune response which is protective against the disease.

### Epitope

The epitopes of the present invention may be B-cell or T-cell epitopes, and they may be of any chemical nature, including without limitation, peptides, carbohydrates, lipids, glycopeptides and glycolipids. The epitope is at least substantially the same as a naturally occurring epitope. It may be identical to a naturally occurring epitope, or a modified form of a naturally occurring epitope.

A term such as "MUC1 epitope", without further qualification, is intended to encompass, not only a native epitope of MUC1, but also a mutant epitope which is substantially identical to a native epitope. Such a mutant epitope must be cross-reactive with a native MUC1 epitope. Likewise, a term such as "tumor-associated epitope" includes both native and mutant epitopes, but the mutant epitope must be cross-reactive with a native tumor-associated epitope.

### B-cell epitopes

B-cell epitopes are epitopes recognized by B-cells and by antibodies.

B-cell peptide epitopes are typically at least five amino acids, more often at least six amino acids, still more often at least seven or eight amino acids in length, and may be continuous ("linear") or discontinuous ("conformational") (the latter being formed by the folding of a protein to bring noncontiguous parts of the primary amino acid sequence into physical proximity).

B-cell epitopes may also be carbohydrate epitopes.

### T cell Epitopes

The T cell epitope, if any, may be any T cell epitope which is at least substantially the same as a T-cell epitope of an antigen including a hapten) which is associated with a disease or adverse condition to a degree such that it could be prophylactically or therapeutically useful to stimulate or enhance a cellular immune response to that epitope. Such diseases and conditions include, but are not limited to parasitic diseases such as schistosomiasis and leishmania, fungal infections such as candidiasis, bacterial infections such as leprosy, viral infections such as HIV infections, and cancers, especially solid tumors. Of course, the greater the degree of specificity of the epitope for the associated disease or adverse condition, the more likely it is that the stimulation of an immune response to that epitope will be free of adverse effects.

The epitope must, of course, be one amenable to recognition by T-cell receptors so that a cellular immune response can occur. For peptides, the T-cell epitopes may interact with class I or class II MHC molecules. The class I epitopes usually 8 to 15, more often 9-11 amino acids in length. The class II epitopes are usually 5-24 (a 24 mer is the longest peptide which can fit in the Class II groove), more often 8-24 amino acids. If the immunogen is larger than these sizes, it will be processed by the immune system into fragments of a size more suitable for interaction with MHC class I or II molecules.

The carbohydrate T-cell epitopes may be as small as a single sugar unit (e.g., Tn). They are preferably no larger than five sugars.

Many T-cell epitopes are known. Several techniques of identifying additional T-cell epitopes are recognized by the art. In general, these involve preparing a molecule which potentially provides a T-cell epitope and characterizing the immune response to that molecule. Methods of characterizing the immune response are discussed in a later section.

The reference to a CTL epitope as being "restricted" by a particular allele of MHC Class I molecules, such as HLA-A1, indicates that such epitope is bound and presented by the allelic form in question. It does not mean that said epitope might not also be bound and presented by a different allelic form of MHC, such as HLA-A2, HLA-A3, HLA-B7, or HLA-B44.

### Disease-Associated and Disease-Specific Epitopes

A disease is an adverse clinical condition caused by infection or parasitization by a virus, unicellular organism, or multicellular organism, or by the development or proliferation of cancer (tumor) cells.

The unicellular organism may be any unicellular pathogen or parasite, including a bacteria, fungus or protozoan. The multicellular organism may be any pathogen or parasite, including a protozoan, worm, or arthropod. Multicellular organisms include both endoparasites and ectoparasites. Endoparasites are more likely to elicit an immune response, but, to the extent they can elicit a protective immune response, ectoparasites and their antigens are within the purview of the present invention.

An epitope may be said to be directly associated with a viral disease if it is presented by a virus particle, or if it is encoded by the viral genome and expressed in an infected cell.

An epitope may be said to be directly associated with a disease caused by a unicellular or multicellular organism if it presented by an intracellular, surface, or secreted antigen of the causative organism.

An epitope may be said to be directly associated with a particular tumor if it is presented by an intracellular, surface or secreted antigen of said tumor. It need not be presented by all cell lines of the tumor type in question, or by all cells of a particular tumor, or throughout the entire life of the tumor. It need not be specific to the tumor in question. An epitope may be said to be "tumor associated" in general if it is so associated with any tumor (cancer, neoplasm).

Tumors may be of mesenchymal or epithelial origin. Cancers include cancers of the colon, rectum, cervix, breast, lung, stomach, uterus, skin, mouth, tung, lips, larynx, kidney, bladder, prostate, brain, and blood cells.

An epitope may be indirectly associated with a disease if the epitope is of an antigen which is specifically produced or overproduced by infected cells of the subject, or which is specifically produced or overproduced by other cells of the subject in specific, but non-immunological, response to the disease, e.g., an angiogenic factor which is overexpressed by nearby cells as a result of regulatory substances secreted by a tumor.

The term "disease associated epitope" also includes any non-naturally occurring epitope which is sufficiently similar to an epitope naturally associated with the disease in question so that antibodies or T cells which recognize the natural disease epitope also recognize the similar non-natural epitope. Similar comments apply to epitopes associated with particular diseases or classes of diseases.

An epitope may be said to be specific to a particular source (such as a disease-causing organism, or, more particular, a tumor), if it is associated more frequently with that source than with other sources, to a detectable and clinically useful extent. Absolute specificity is not required, provided that a useful prophylactic, therapeutic or diagnostic effect is still obtained.

In the case of a "specific tumor-specific" epitope, the epitope is more frequently associated with that tumor that with other tumors, or with normal cells. Preferably, there should be a statistically significant (p=0.05) difference between its frequency of occurrence in association with the tumor in question, and its frequency of occurrence in association with (a) normal cells of the type from which the tumor is derived, and (b) at least one other type of tumor. An epitope may be said to be "tumor-specific" in general is it is associated more frequently with tumors (of any or all types) than with normal cells. It need not be associated with all tumors.

The term "tumor specific epitope" also includes any non-naturally occurring epitope which is sufficiently similar to a naturally occurring epitope specific to the tumor in question (or as appropriate, specific to tumors in general) so that antibodies or T cells stimulated by the similar epitope will be essentially as specific as CTLs stimulated by the natural epitope.

In general, tumor-versus-normal specificity is more important than tumor-versus-tumor specificity as (depending on the route of administration and the particular normal tissue affected), higher specificity generally leads to fewer adverse effects. Tumor-versus-tumor specificity is more important in diagnostic as opposed-to therapeutic uses.

The term "specific" is not intended to connote absolute specificity, merely a clinically useful difference in probability of occurrence in association with a pathogen or tumor rather than in a matched normal subject.

### Parasite-Associated Epitopes

In one embodiment, the epitope is a parasite-associated epitope, such as an epitope associated with leishmania, malaria, trypanosomiasis, babesiosis, or schistosomiasis. Suitable parasite-associated epitopes include, but are not limited to, the following.

| Parasite | Epitope | References |
|---|---|---|
| Plasmodium Falciparum (Malaria | (NANP) 3 (SEQ ID NO:3) | Good et al (1986) J. Exp. Med. 164:655 |
| | Circumsporoz. protein AA 326-343 | Good et al (1987 Science 235:1059 |
| Leishmania donovani | Repetitive peptide | Liew et al (1990) J. Exp. Med. 172:1359 |
| Leishmania major | EAEEAARLQA (SEQ ID NO:4) | Longenecker, et al., 08/229,606 |
| Toxoplasma gondii | P30 surface protein | Darcy et al (1992) J. Immunolog. 149:3636 |
| Schistosoma mansoni | Sm-28GST antigen | Wolowxzuk et al (1991) J. Immunol 146:1987 |

### Virus-Associated Epitopes

In another embodiment, the epitope is a viral epitope, such as an epitope associated with human immunodeficiency virus (HIV), Epstein-Barr virus (EBV), or hepatitis. Suitable viral epitopes include, but are not limited to:

| Virus | Epitope | Reference |
|---|---|---|
| HIV gp120 | V3 loop, 308-331 | Jatsushita, S. et al (1988) J. Viro. 62:2107 |
| HIV GP120 | AA 428-443 | Ratner et al (1985) Nature 313:277 |
| HIV gp120 | AA 112-124 | Berzofsky et al (1988) Nature 334:706 |
| HIV | Reverse transcriptase | Hosmalin et al (1990) PNAS USA 87:2344 |
| Flu | nucleoprotein AA 335-349, 366-379 | Townsend et al (1986) Cell 44:959 |
| Flu | haemagglutinin AA48-66 | Mills et al (1986) J. Exp. Med. 163:1477 |
| Flu | AA111-120 | Hackett et al (1983) J. Exp. Med 158:294 |
| Flu | AA114-131 | Lamb, J. and Green N. (1983) Immunology 50:659 |
| Epstein-Barr | LMP43-53 | Thorley-Lawson et al (1987) PNAS USA 84:5384 |
| Hepatitis B | Surface Ag AA95-109; AA 140-154 | Milich et al (1985) J. Immunol. 134:4203 |
| | Pre-S antigen AA 120-132 | Milich, et al. (1986) J. Exp. Med. 164:532 |
| Herpes simplex | gD protein AA5-23 | Jayaraman et al (1993) J. Immunol. 151:5777 |
| | gD protein AA241-260 | Wyckoff et al (1988) Immunobiology 177:134 |
| Rabies | glycoprotein AA32-44 | MacFarlan et al (1984) J. Immunol 133:2748 |

### Bacteria-Associated Epitopes

The epitope may also be associated with a bacterial antigen. Suitable epitopes include, but are not limited to:

| Bacteria | Epitope ID | Reference |
|---|---|---|
| Tuberculosis | 65Kd protein AA112-126 AA163-184 AA227-243 AA242-266 AA437-459 | Lamb et al (1987) EMBO J. 6:1245 |
| Staphylococcus | nuclease protein AA61-80 | Finnegan et al (1986) J. Exp. Med 164:897 |
| E. coli | heat stable enterotoxin | Cardenas et al (1993) Infect Immunity 61:4629 |
| | heat labile entertoxin | Clements et al (1986) Infect. Immunity 53:685 |
| Shigella sonnei | form I antigen | Formal et al (1981) Infect. Immunity 34:746 |

### Cancer-Associated Epitopes

In another embodiment, the epitope is associated with a cancer (tumor), including but not limited to cancers of the respiratory system (lung, trachea, larynx), digestive system (mouth, throat, stomach, intestines) excretory system (kidney, bladder, colon, rectum), nervous system (brain), reproductive system (ovary, uterus, cervix), glandular system (breast, liver, pancreas, prostate), skin, etc. The two main groups of cancers are sarcomas, which are of mesenchymal origin and affect such tissues as bones end muscles, and carcinomas, which are of epithelial origin and make up the great majority of the glandular cancers of breasts, stomach, uterus, skin and tongue. The sarcomas include fibrosarcomas, lymphosarcomas, osteosarcomas, chondrosarcomas, rhabdosarcomas and liposarcomas. The carcinomas include adenocarcinomas, basal cell carcinomas and squamous carcinomas.

Cancer-associated epitopes include, but are not limited to, peptide epitopes such as those of mutant p53, the point mutated Ras oncogene gene product, her 2/neu, c/erb2, and the MUC1 core protein, and carbohydrate epitopes such as sialyl Tn (STn), TF, Tn, CA 125, sialyl Le^{x}, sialyl Le^{a} and P97.

### Carbohydrate Epitopes

Carbohydrate epitopes are also of interest. For example, any of three types of tumor-associated carbohydrate epitopes which are highly expressed in common human cancers may be presented. These particularly include the lacto series type 1 and type 2 chains, cancer associated ganglio chains, and neutral glycosphingolipids. Examples of the lacto series Type 1 and Type 2 chains are as follows: Lewis a, dimeric Lewis a, Lewis b, Lewis b/Lewis a, Lewis x, Lewis, y, Lewis a/Lewis x. dimeric Lewis x, Lewis y/Lewis x, trifucosyl Lewis y, trifucosyl Lewis b, sialosyl Lewis x, sialosyl Lewis y, sialosyl dimeric Lewis x, Tn, sialosyl Tn, sialosyl TF, TF. Examples of cancer-associated ganglio chains are as follows: GM3. GD3, GM2, GM4, GD2, GM1, GD-1a, GD-1b. Neutral sphingolipids include globotriose, globotetraose, globopentaose, isoglobotriose, isoglobotetraose, mucotriose, mucotetraose, lactotriose, lactotetraose, neolactotetraose, gangliotriose, gangliotetraose, galabiose, and 9-O-acetyl-GD3.

Numerous antigens of clinical significance bear carbohydrate determinants. One group of such antigens comprises the tumor-associated mucins (Roussel, et al., Biochimie 70, 1471, 1988).

Generally, mucins are glycoproteins found in saliva, gastric juices, etc., that form viscous solutions and act as lubricants or protectants on external and internal surfaces of the body. Mucins are typically of high molecular weight (often > 1,000,000 Dalton) and extensively glycosylated. The glycan chains of mucins are O-linked (to serine or threonine residues) and may amount to more than 80% of the molecular mass of the glycoprotein. Mucins are produced by ductal epithelial cells and by tumors of the same origin, and may be secreted, or cell-bound as integral membrane proteins (Burchell, et al., Cancer Res., 47, 5476, 1987; Jerome, et al., Cancer Res., 51, 2908, 1991).

Cancerous tissues produce aberrant mucins which are known to be relatively less glycosylated than their normal counter parts (Hull, et al., Cancer Commun., 1, 261, 1989). Due to functional alterations of the protein glycosylation machinery in cancer cells, tumor-associated mucins typically contain short, incomplete glycans. Thus, while the normal mucin associated with human milk fat globules consists primarily of the tetrasaccharide glycan, gal β1-4 glcNAcpl-6(gal β1-3) gal NAc-αand its sialylated analogs (Hull, et al.), the tumor-associated Tn hapten consists only of the monosaccharide residue, α-2-acetamido-3-deoxy-D-galactopyranosyl, and the T-hapten of the disaccharide β-D-galactopyranosyl-(1-3)α-acetamido-2-deoxy-D-galactopyranosyl. Other haptens of tumor-associated mucins, such as the sialyl-Tn and the sialyl-(2-6)T haptens, arise from the attachment of terminal sialyl residues to the short Tn and T glycans (Hanisch, et al., Biol . Chem. Hoppe-Seyler, 370, 21, 1989; Hakormori, Adv. Cancer Res., 52:257, 1989; Torben, et al., Int. J. Cancer, 45 666, 1980; Samuel, et al., Cancer Res., 50, 4801, 1990).

The T and Tn antigens (Springer, Science, 224, 1198, 1984) are found in immunoreactive form on the external surface membranes of most primary carcinoma cells and their metastases (>90% of all human carcinomas). As cancer markers, T and Tn permit early immunohistochemical detection and prognostication of the invasiveness of some carcinomas (Springer). Recently, the presence of the sialyl-Tn hapten on tumor tissue has been identified as an unfavorable prognostic parameter (Itzkowitz, et al. Cancer, 66, 1960, 1990; Yonezawa, et al., Am. J. Clin. Pathol., 98 167, 1992). Three different types of tumor-associated carbohydrate antigens are highly expressed in common human cancers. The T and Tn haptens are included in the lacto series type, and type 2 chains. Additionally, cancer-associated ganglio chains and glycosphingolipids are expressed on a variety of human cancers.

The altered glycan determinants displayed by the cancer associated mucins are recognized as non-self or foreign by the patient's immune system (Springer). Indeed, in most patients, a strong autoimmune response to the T hapten is observed. These responses can readily be measured, and they permit the detection of carcinomas with greater sensitivity and specificity, earlier than has previously been possible. Finally, the extent of expression of T and Tn often correlates with the degree of differentiation of carcinomas. (Springer).

An extensive discussion of carbohydrate haptens appears in Wong, USP 6,013,779. A variety of carbohydrates can be incorporated into a synthetic glycolipopeptide immunogen, according to the present invention, for use particularly in detecting and treating tumors. The Tn, T, sialyl Tn and sialyl (2-->6)T haptens are particularly preferred.

In particular, for detecting and treating tumors, the three types of tumor-associated carbohydrate epitopes which are highly expressed in common human cancers are conjugated to aminated compounds. These particularly include the lacto series type 1 and type 2 chain, cancer associated ganglio chains, and neutral glycosphingolipids.

Examples of the lacto series Type 1 and Type 2 chains are as follows:

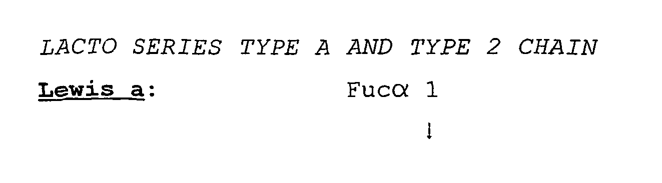

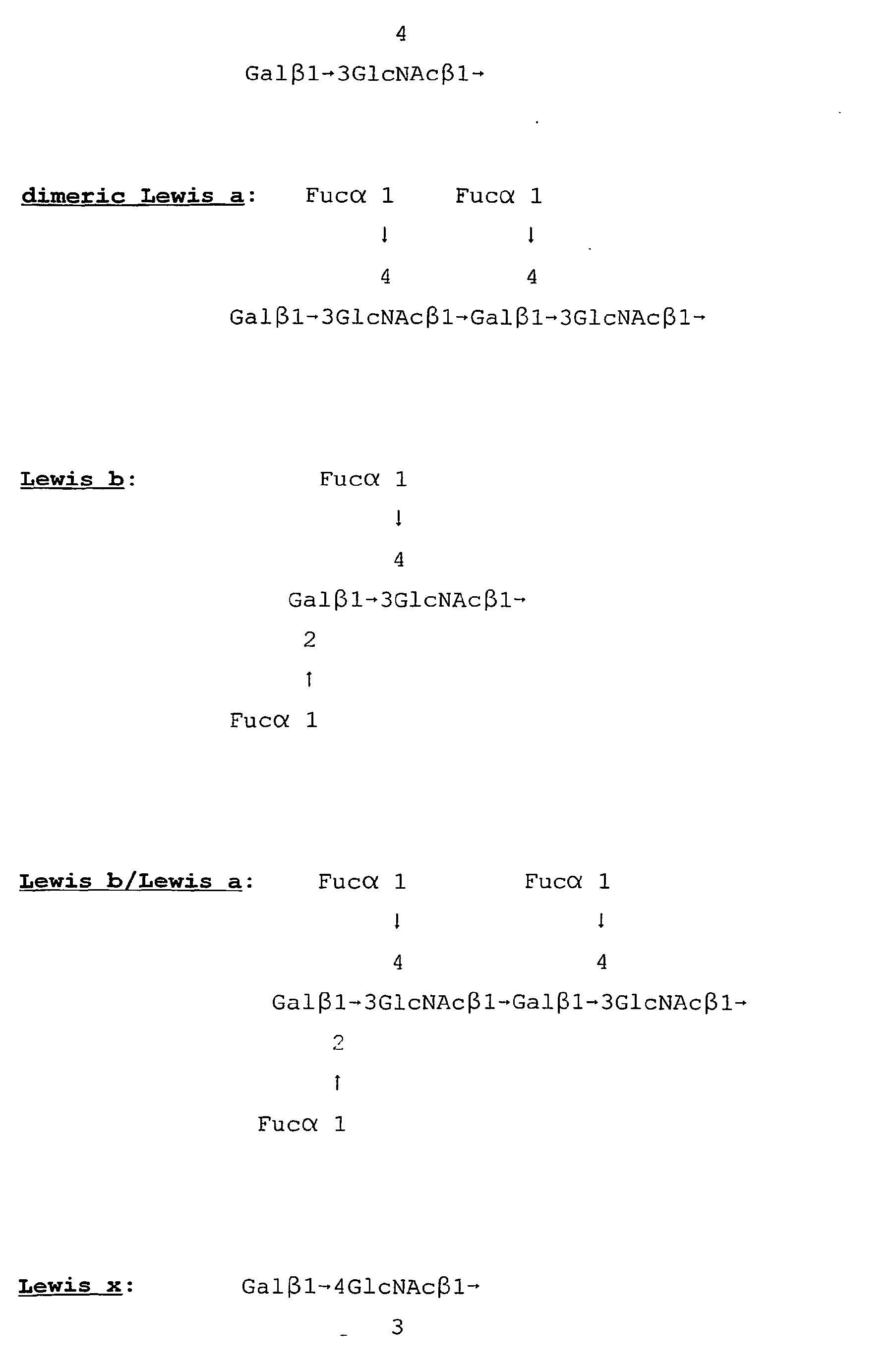

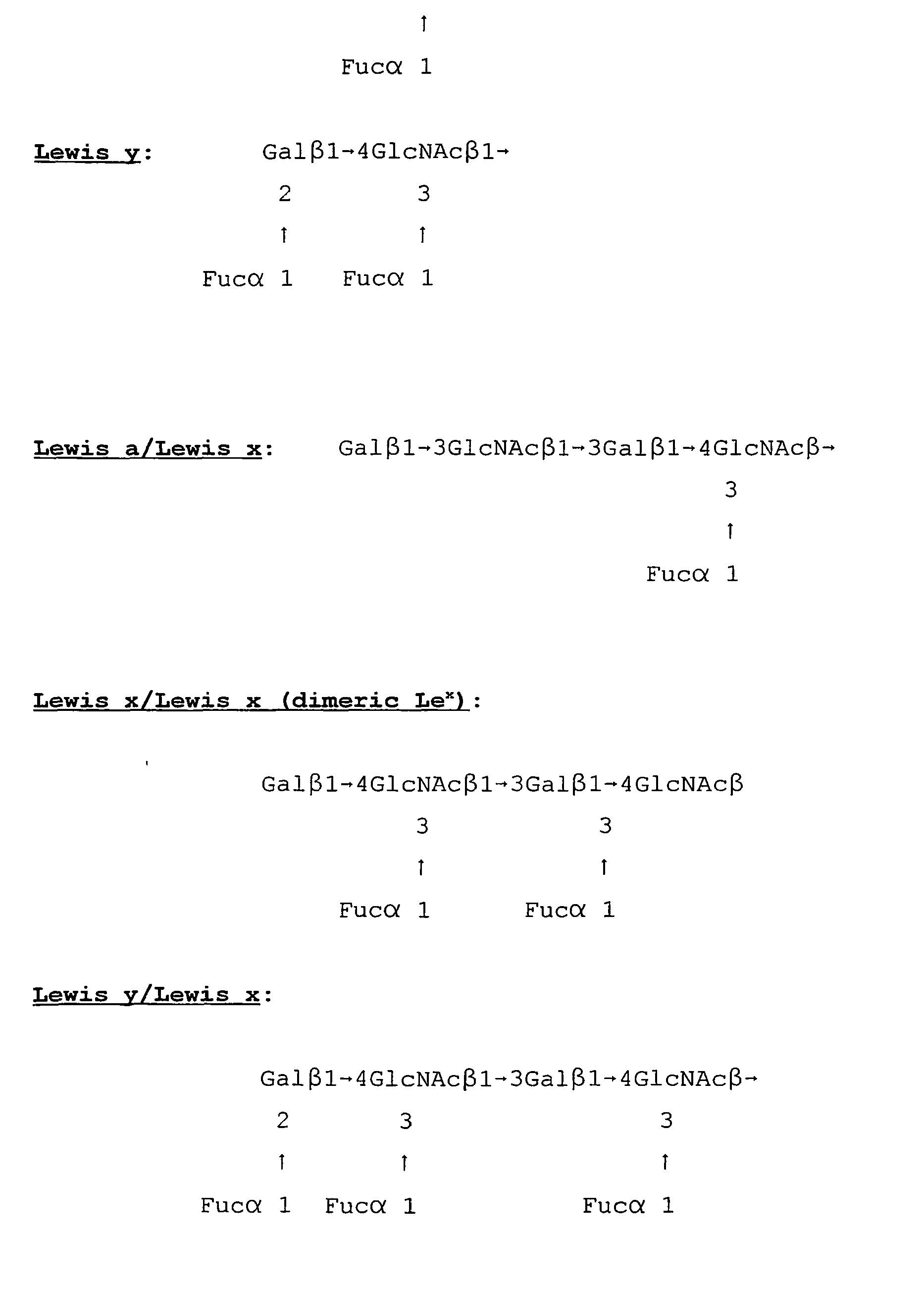

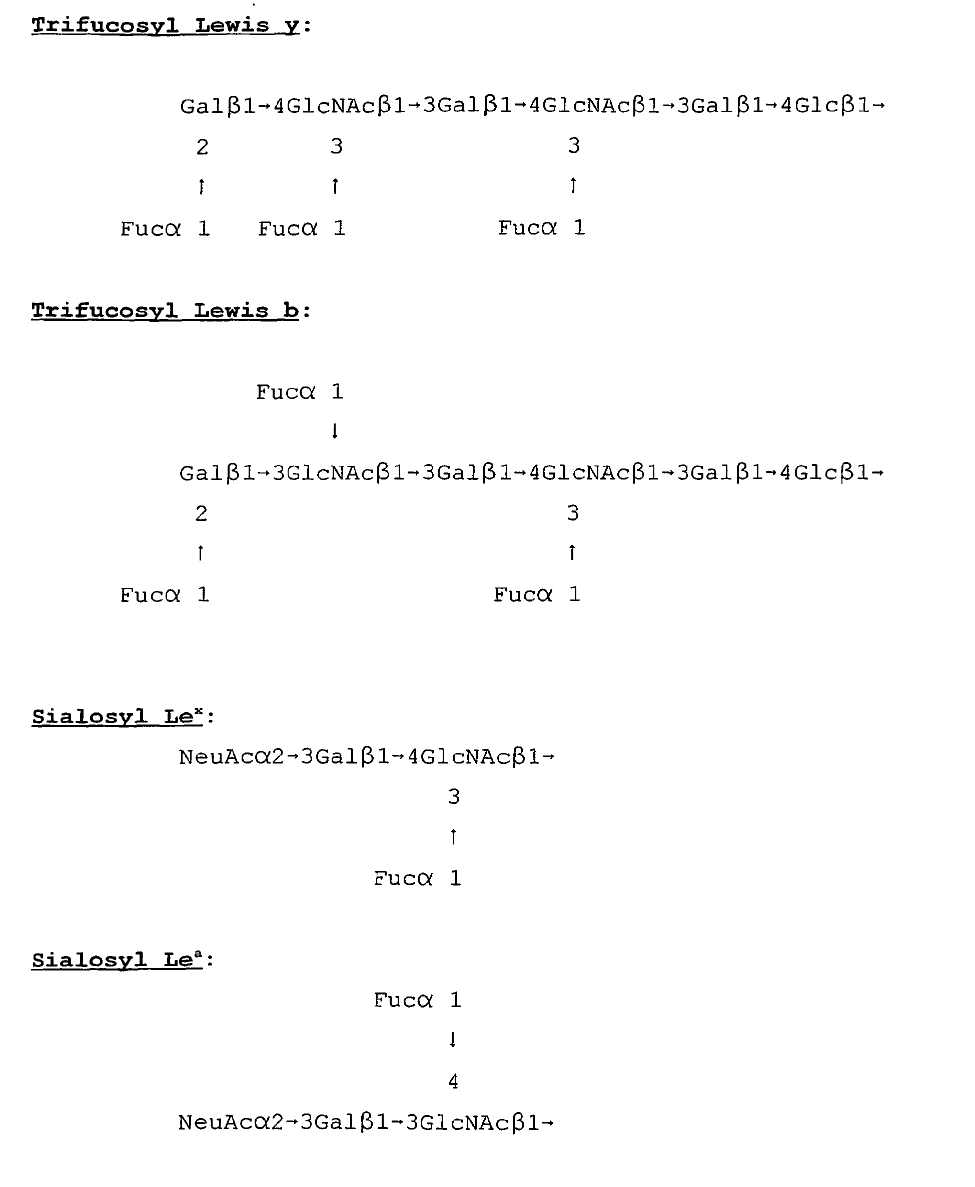

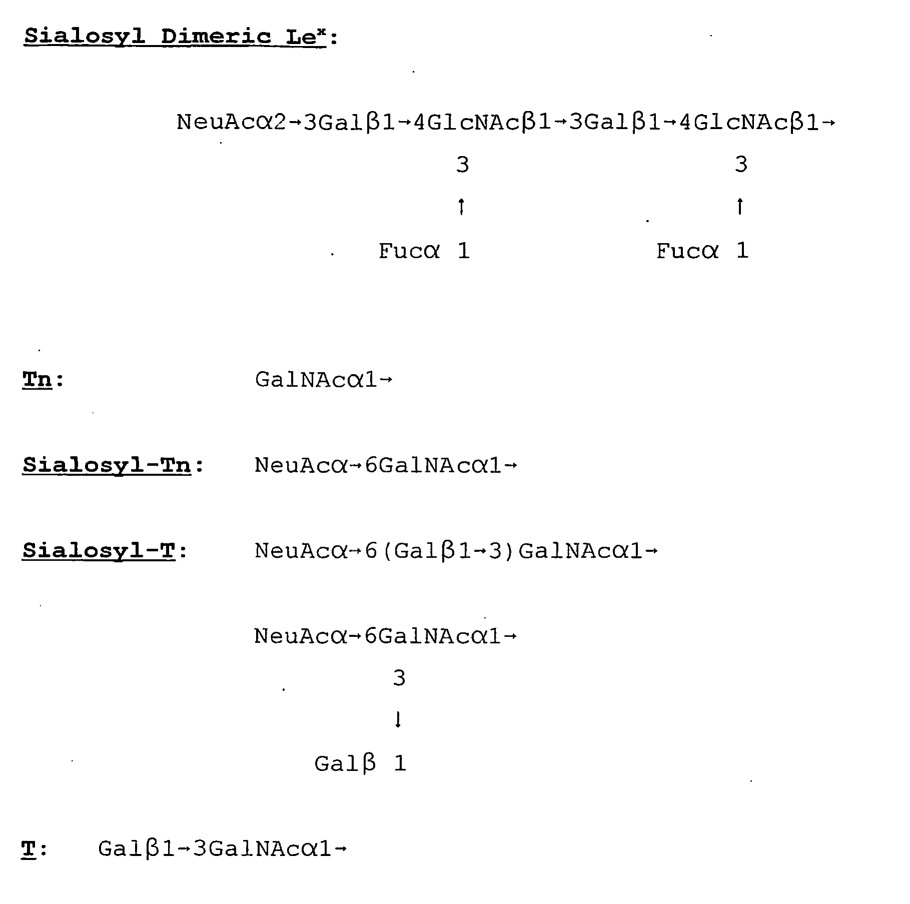

Examples of cancer-associated ganglio chains that can be conjugated to aminated compounds according to the present invention are as follows:

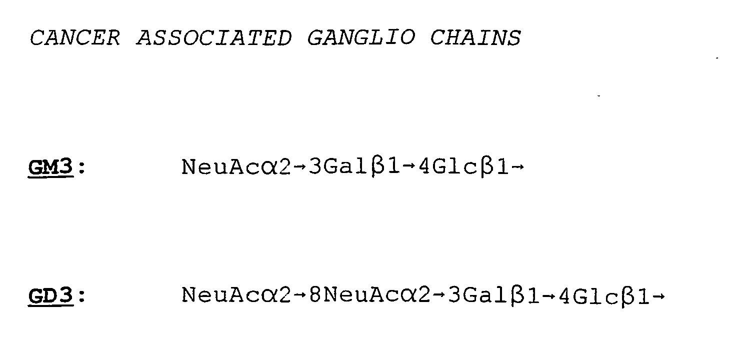

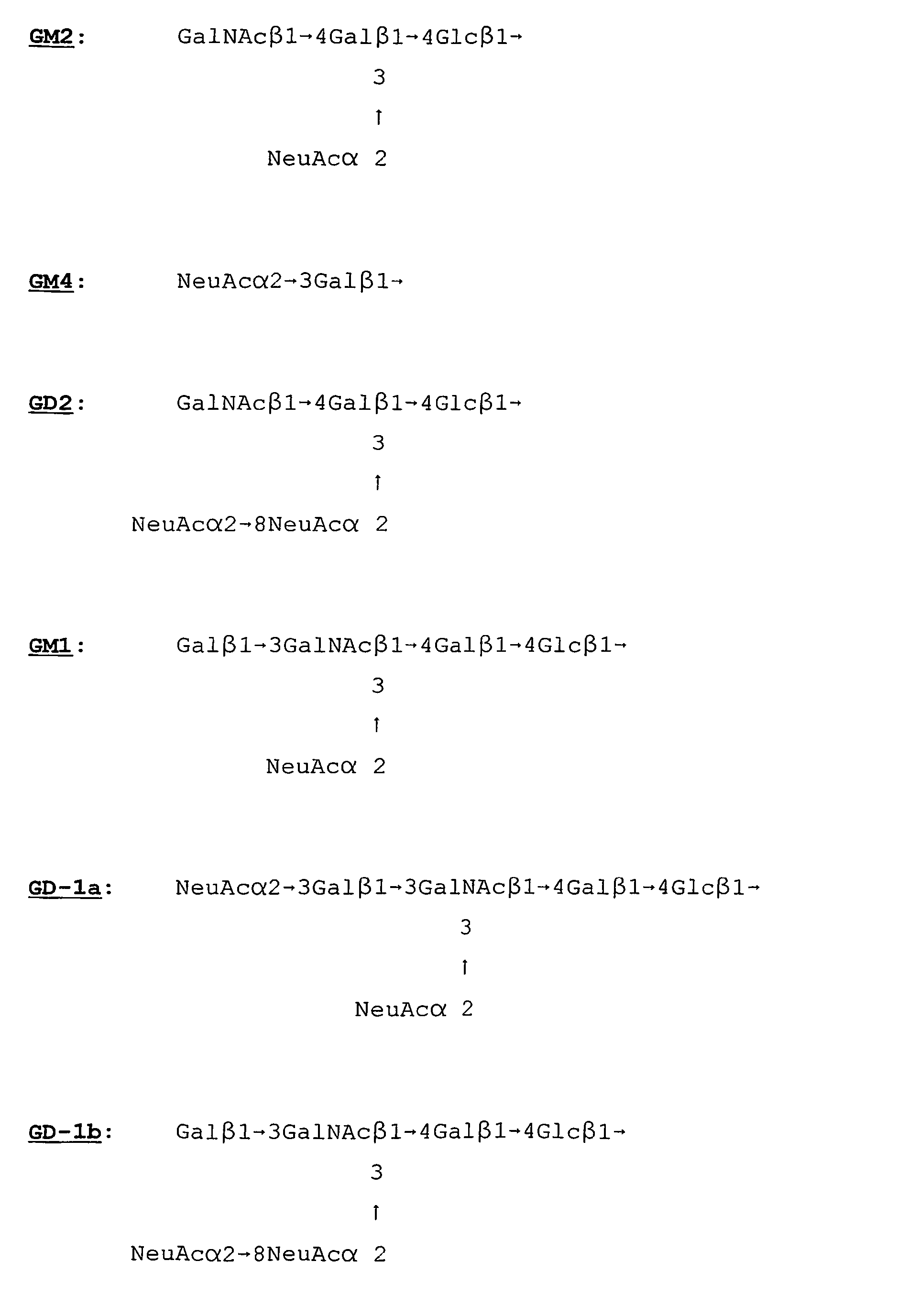

In addition to the above, neutral glycosphingolipids can also be conjugated to aminated compounds according to the present invention:

### SELECTED NEUTRAL GLYCOSPHINGOLIPIDS

| | |
|---|---|
| **Globotriose:** | Galα→4Galβ1→4Glcβ1→ |
| **Globotetraose:** | GalNAcβ1→3Galα→4Galβ1→4Glcβ1→ |
| **Globopentaose:** | GalNAcα1→3GalNAcβ1→3Galα→4Galβ1-4Glcβ1→ |
| **Isoglobotriose:** | Galα→3Galβ1→4Glcβ1→ |
| **Isoglobotetraose:** | GalNAcβ1→3Galα1→3Galβ1→4Glcβ1→ |
| **Mucotriose:** | Galβ1→4Galβ1→4Glcβ1→ |
| **Mucotetraose:** | Galβ1→3Galβ1→4Galβ1→4Glcβ→ |
| **Lactotriose:** | GalNAcβ1→3Galβ1→4Glcβ1→ |
| **Lactotetraose:** | GalNAcβ1→3GalNAcβ1→3Galβ1→4Glcβ1→ |
| **Neolactotetraose:** | Galβ1→4GlcNAcβ1→3Galβ1-4Glcβ1→ |
| **Gangliotriose:** | GalNAcβ1→4Galβ1→4Glcβ1→ |
| **Gangliotetraose:** | Galβ1→GlcNAcβ1→4Galβ1→4Glcβ1→ |
| **Galabiose:** | Galα→4Galβ1→ |
| **9-0-Acetyl-GD3:** | 9-0-Ac-NeuAcα2→8NeuAcα2→3Galβ1→4Glcβ1- |

### Mucin epitope

In a preferred embodiment, the epitope is an epitope of a cancer-associated mucin. Mucins are glycoproteins characterized by high molecular weight (>1,000,000 daltons) and extensive glycosylation (over 80%). Mucins may be expressed extracellularly, or as an integral cell membrane glycoprotein with distinct external, transmembrane, and cytoplasmic domains. Cell membrane mucins exist as flexible rods and protrude relatively great distances from the cell surface forming an important component of the glycocalyx (Jentoff, 1990) and the terminal carbohydrate portions thereof are probably the first point of contact with antibodies and cells of the immune system.

Abberant or cancer-associated mucins are known to be relatively less glycosylated (Hull et al, 1989) and hence antigenically different from their normal cell counterpart mucins exposing normally cryptic carbohydrate- (Hanish et al, 1989; Torben et al,1990; Samuel et al, 1990), peptide-(Burchell et al, 1987) and perhaps even glycopeptide-epitopes. Therefore, because cell surface mucins protrude, they themselves may serve as targets for immune attack (Henningson, et al., 1987; Fung, et al., 1990; Singhal, et al., 1991; Jerome et al., 1991; Oncogen, EP 268,279; Biomembrane Institute, W089/08711; Longenecker, USP 4,971,795). Under some circumstances, cancer-associated cell membrane mucins can actually "mask" other cell surface antigens and protect cancer cells from immune attack (Codington et al, 1983; Friberg, 1972; Miller et al, 1977).

The mucin epitope may be a core peptide, a carbohydrate, or a glycopeptide. Non-limiting examples of mucins which may carry epitopes are the human tumor associated Thomsen-Friedenreich antigen, (MacLean, 1992), epiglycanin-related glycoprotein (Codington, 1984) ovine submaillary mucin, bovine submaxillary mucin, breast tumor mucins (e.g., human polymorphic epithelial mucin, including breast tumor mucins, Gendler, 1988, 1990; breast cancer epithelial tumor antigen, Hareuveni, 1990, breast carcinoma, Hull, 1989), mammary tumor mucins (e.g., such as murine mammary adenocarcinoma, Fung, 1990) carcinoma mucins such as mucins arising from the kidney (e.g., renal cell carcinoma), ovary (e.g., ovarian carcinoma-associated sebaceous gland antigen, Layton, 1990), bladder, colon (e.g., Sialosyl-Tn in colorectal cancer, Itzkowitz, 1990) pancreatic tumor mucin (Lan, 1990), gallbladder, bladder, colon (e.g., malignant colon mucosa mucins, Torbin, 1980) and some lung tissues, melanoma mucins (e.g., melanoma-associated antigen, Kahn, 1991) epithelial tumor cell mucins, leukemia associated mucins, carcinoembryonic antigen, or any other mucin associated with abnormal cells according to known characteristics of cancer associated mucins or abnormal mucins, such as abberant glycosylation (Hakomori, 1989, and Singhal, 1990) .

### MUC1 epitopes

The human MUC1 gene product has been referred to by various names, including MAM6, milk mucin; human milk fat globule antigen (HMFG); human mammary epithelial antigen, CA 15-3, CA 27.29; episialin; and polymorphic epithelial mucin (PEM) (reviewed in Taylor-Papadimitriou et al, 1988) (for complete cites to the incompletely cited references in this section, see Longenecker, et al., 08/229,606). This mucin is strongly expressed on human breast (Gendler et al, 1988), pancreatic (Lan et al, 1990) and certain ovarian cancer cells (Layton et al, 1990). Although the MUC1 encoded mucins expressed on various cancers contain the same tandem repeat core peptide sequence, glycosylation differences do exist (Gendler et al, 1988; Lan et al, 1990). Because of underglycosylation in cancer cells, MUC-1 molecules on cancer cells express cryptic epitopes which are not expressed (i.e, are cryptic) on normal epithelial cells.

MUC1 is the first cancer-associated mucin gene to be cloned and mapped (Gendler et al, 1990), and has recently been transfected into a murine mammary cell line, 410.4 (Lalani et al, 1991). MUC1 transfected 410.4 cells express the MUC1 gene product on the cell surface.

The pattern of glycosylation is similar to, but different from, malignant cell derived mucins expressing the same cryptic peptide epitopes as expressed by human cancer associated MUC1 (Taylor-Papadimitriou et al, 1988). Lalani and co-workers (1991) have examined the immunogenicity of the 410.4 transfectants in mice. These workers demonstrated that mice which rejected a low dose of transfected 410.4 cells did not develop tumors after a subsequent transplant of a high dose of transfected 410.4 cells although no effect on tumor development of untransfected wild type 410.4 cells was seen (Taylor-Papadimitriou et al, 1988). (For complete cites, see Longenecker5-USA, and see also refs 4-11 thereof).

It has been shown that cancer vaccines composed of synthetic peptide antigens which mimic cryptic MUC-1 peptide sequences on cancer cells are able to induce effective anticancer immunotherapy against MUC-1 expressing tumor cells in a murine model · Finn and co-workers have shown that cancer patients are able to produce specific non-MHC restricted cytotoxic T-lymphocytes (CTL) which recognize peptide epitopes expressed on MUC-1 molecules on cancer cells. (See refs. 12 and 53-55 of Longenecker5-USA). Indeed the MUC1 sequence SAPDTRP (AAs 4-10 of SEQ ID NO:10) has been shown to be both a T-and a B- cell epitope. It has been demonstrated that the immunization of chimpanzees with synthetic MUC-1 antigens induces the development of specific antibodies and CMI against MUC-1.

The human epithelial mucin MUC1 is over-expressed in more than 90% of carcinomas of the breast, ovary and pancreas, and in those tumors it is aberrantly glycosylated. The SM3 antibody binds the core protein of MUC1; it also binds the tumor glycoproteins, presumably because the SM3 epitope is exposed as a result of the aforementioned aberrant glycosylation.

The amino acid sequence of Human MUC1 is available in the SWISS-PROT database as P15941. The number of repeats is highly polymorphic. It varies from 21 to 125 in the northern european population. The most frequent alleles contains 41 and 85 repeats. The tandemly repeated icosapeptide underlies polymorphism at three positions, as shown by brackets: PAPGSTAP[P/A/Q/T]AHGVTSAP[D/E][T/S]R (SEQ ID NO:5). The common polymorphisms are the coordinated double mutation DT -> ES and the single replacements P -> A, P -> Q and P-> T. The most frequent replacement DT > ES occurs in up to 50% of the repeats. For Mouse MUC1, see SWISS-PROT Q02496.

Moller, et al., Eur. J. Biochem. 269:1444-55 (Mar. 2002) has used NMR spectroscopy to study the binding of the SM3 antibody to the pentapeptide MUC1 epitope PDTRP and to the related glycopentapeptide in which the threonine is O-lined to alpha-d-GalNAc. Moller found that the PDT interacted with the SM3 antibody more strongly than did the RP, suggesting that the RP would be more tolerant of mutation. In contrast, the glycopeptide interacted with SM3 using all of its amino acids, although the strongest effect was with the Pro1. Docking studies were conducted; these could be performed with mutant peptides for which 3D structures are deducible or determined.

Hiltbold, et al., Cancer Res., 58:5066-70 (1998) showed that CD4+ T-cells primed in vitro with a synthetic MUC1 peptide of 100 amino acids, representing five unglycosylated tandem repeats, and presented by dendritic cells, produced IFN-gamma and had moderate cytolytic activity. They also identified a core peptide sequence, PGSTAPPAHGVT (SEQ ID NO:6), which elicits this response when it is presented by HLA-DR3.

Heukamp, et al., Int. J. Cancer, 91:385-92 (2001) eleicted peptide-specific CTL immunity in A2/K(b) transgenic mice with three MUC1-derived peptides that map outside the variable number tandem repeat region. These peptides were MUC(79-87)(TLAPATEPA)(SEQ ID NO:7), MUC(167-175) (ALGSTAPPV) (SEQ ID NO:8) and MUC (264-72) (FLSFHISNL) (SEQ ID NO:9). All comply with the peptide binding motif for HLA-A*0201.

Engelmann, et al., J. Biol. Chem. 276:27764-9 (Jul. 2001) report that there are three sequence variants in the tandem repeat region of MUC1. Variant 1 replaced DT with ES.

Soares et al., J. Immunol. 166: 6555-63 (Jun. 2001) used a seven tandem repeat MUC1 peptide to elicit an immune response. If the peptide was delivered on dendritic cells, it only elicited T cell immunity. If injected together with soluble peptide, Ab production was also triggered.

Von Mensdorff-Pouilly et al., J. Clin. Oncol. 18:574-83 (Feb. 2000) used a MUC1 triple tandem repeat peptide conjugated to BSA in an immunoassay of anti-MUC1 antibody levels in breast cancer patients.

Denton, et al., Pept. Res. 7:258-64 (Sept./Oct. 1994), colinearly liked a MUC1 mucin B cell peptide epitope to a known murine T cell epitope in both T-B and B-T orientations. Brossart et al., Blood, 93:4309-17 (June 1999) analyzed the MUC1 amino acid sequence and identified two novel peptides with a high binding probability to the HLA-A2 molecule. One was from the variable tandem repeat region, and the other from outside it.

Carmon, et al., Int. J. Cancer, 85:391-7 (Feb. 2000) evaluated the anti-tumor potential of HLA-A2.1 motif-selected peptides from non-tandem repeat regions of the molecule. See also Pietersz et al., Vaccine, 18:2059-71 (Apr. 2000).

Keil, et al. Angew. Chem. Int. Ed. Engl. 40:366-9(Jan. 2001) conjugated a MUC1 epitope to a tetanus toxin epitope.

Von Mensdorff-Pouilly et al., Int. J. Cancer, 86:702-12 (Jun. 2000) reported that the most frequent minimal epitopic sequences of natural MUC1 IgG and IgM antibodies were RPAPGS (AAs 9-14 of SEQ ID NO:10), PPAHGVT (AAs 4-10 of SEQ ID NO:11; equivalent to AAs 17-20 followed by AAs 1-3 of SEQ ID NO:10) and PDTRP (AAs 6-10 of SEQ ID NO:10). MUC1 peptide vaccination induced high titers of IgM and IgG antibodies predominantly directed, respectively, to the PDTRPAP (AAs 6-12 of SEQ ID NO:10) and the STAPPAHGV (AAs 1-9 of SEQ ID NO:2) sequences of the tandem repeat. Natural MUC Abs from breast cancer patients reacted more strongly with GalNac-glycosylated peptides than with unglycosylated peptides.

See also EP Appl 1,182,210; Sandrin, USP 6,344,203; Finn, USP 5,744,144.

See also, Petrakou, et al., "Epitope Mapping of Anti-MUC1 Mucin protein Core Monoclonal Antibodies" (21-29); Imai, et al., "Epitope Characterization of MUC1 Antibodies" (30-34), Schol, et al., "Epitope Fingerprinting Using Overlapping 20-mer peptides of the MUC1 Tandem repeat sequence" (35-45), and Blockzjil, "Epitope characterization of MUC1 Antibodies" (46-56), all in ISOBM TD-4 International Workshop on Monoclonal Antibodies against MUC1 Nov. 1996), reprinted in Tumor Biology, 19 Suppl. 1: 1-152 (1998).

See also Von Mensdorff-Pouilly, et al., "Human MUC1 mucin: a multifacted glycoprotein," Int J. Biol. Markers, 15:343-56 (2000)

The present invention therefore contemplates glycolipopeptides which comprise at least one native B and/ or T cell epitope of MUC1, or at least one mutant epitope substantially identical to such a native epitope. It may further comprise additional MUC1 sequence which is not part of an epitope.

Preferably, the glycolipopeptide comprises both a B cell epitope and a T cell epitope of MUC1 (which, in each case, may be a natural epitope or an allowed mutant thereof), and these epitopes may be identical, overlapping, or distinct. T and B cell epitopes of an antigen may overlap. For example, in the case of MUC-1, SAPDTRP (AAs 4-10 of SEQ ID NO:10) is a T-cell epitope, while PDTRP (AAs 6-10 of SEQ ID NO:10) is merely a B-cell epitope.

It may further comprise additional B cell epitopes, and/or additional T cell epitopes. The B cell epitopes may be the same or different, and likewise the T cell epitopes may be the same or different.

If the immunogen of the present invention comprises a MUC1-related sequence at least substantially identical to a MUC1 sequence of at least five amino acids, the MUC1-related sequence may comprise one or more glycosylation sites found in the corresponding MUC1 sequence. It may differ from the corresponding MUC1 sequence in the number of potential glycosylation sites, as a result of mutation, or it may have the same number of potential glycosylation sites.

The potential glycosylation sites may be (1) sites actually glycosylated in the MUC1-derived tumor glycoprotein, (2) sites potentially glycosylatable but not actually glycosylated in that tumor glycoprotein, and/or (3) sites foreign to said glycoprotein. Likewise, the actual glycosylation sites may be (1) sites actually glycosylated in the MUC1-derived tumor glycoprotein, (2) sites potentially glycosylatable but not actually glycosylated in that tumor glycoprotein, and/or (3) sites foreign to said glycoprotein. None, one, some or all of the glycosylation sites normally glycosylated in the MUC1-derived tumor glycoprotein may be glycosylated in the immunogen of the present invention.

MUC1 is a polymorphic antigen characterized by a variable number (typically 21-125, especially 41 or 85) of perfect and imperfect repeats of the following sequence:

### GVTSAPDTRPAPGSTAPPAH (SEQ ID NO:10)

Since there are multiple repeats of this sequence, the starting point shown is arbitrary, and an epitope may bridge two repeats.

Consequently, the immunogens of the present invention may comprise the aforementioned complete repeat sequence or a cyclic permutation thereof. Moreover, they may comprise two or more copies of the aforementioned repeat or a cyclic permutation thereof. Thus, in compounds 1a and 1b, there are two copies of a cyclic permutation (starting at TSA... and ending with HGV) of the above sequence, followed by the unrelated SSL sequence.

Each MUC1 epitope in question may correspond to an epitope of the variable tandem repeat region, or to an epitope outside that region. The former include RPAPGS (AAs 9-14 of SEQ ID NO:10), PPAHGVT (AAs 4-10 of SEQ ID NO:11) and PDTRP (AAs 6-10 of SEQ ID NO:10). The sequence PDTRPAPGS (AAs 6-14 of SEQ ID NO:10) is of particular interest, as it includes two overlapping epitopes. The PDTRP sequence forms the tip of a protruding knob exposed to solvents and forming a stable type II beta-turn.

The non- VNTR region epitopes include MUC(79-87)(TLAPATEPA)(SEQ ID NO:7), MUC(167-175) (ALGSTAPPV) (SEQ ID NO:8) and MUC(264-72)(FLSFHISNL)(SEQ ID NO:9).

Preferably, the glycolipopeptide comprises the polymorphic epitope P[D/E][T/S]RP or a substantially identical mutant thereof. More preferably it comprises PDTRP or a substantially identical mutant thereof.

In some embodiments, the glycolipopeptide comprises at least one 20 amino acid sequence (an effective tandem repeat) which differs solely by one or more conservative substitutions and/or a single nonconservative substitution from a tandem repeat of MUC1, and comprises an epitope of the variable tandem repeat region of MUC1 (either identically, or an allowed mutant). Preferably, it differs solely, if at all, by conservative substitutions, more preferably, by no more than a single conservative substitutions, and most preferably, is identical to such a tandem repeat. It should be noted that the tandem repeats of MUC1 are imperfect and hence the sequence could be identical to one repeat but not to another. Also, there are allelic variations in these repeats, and so the sequence could be identical to the sequence for one allele and not for another.

In a subset of these embodiments, the glycolipopeptide comprises a plurality of nonoverlapping effective tandem repeats, such as two (for a total of 40 amino acids), three (for a total of 60 amino acids), four, five, six, seven or eight. These effective tandem repeats may, but need not be, identical to each other. (In contrast, note that in the natural human MUC1 mucin, the number of repeats is typically 21-125.)

Besides one or more effective tandem repeats, the peptide portion of the glycolipopeptide may comprise additional amino acid subsequences. If so, these subsequences may comprise additional epitopes, which may be MUC1 variable tandem repeat region epitopes (falling short of a effective tandem repeat), MUC1 epitopes from outside that region, or epitopes of other cancer antigens. It may also include an immunomodulatory element, see Longenecker, et al., 08/229,606.

Preferably, one or more of the serines and/or threonines of the MUC1 tandem repeat are glycosylated, preferably with Tn or sialyl Tn. In the natural human MUC1 mucin, there are five normal glycosylation sites per repeat. In normal MUC1, an average of 2.6 of these five sites us ub fact occupied. The average number of glycosylated amino acids per repeat may be less than, the same as, or greater than the "natural" value.

Preferably, the glycolipopeptide comprises, in its C-terminal region, the sequence SSL, where both serines are lipidated.

### Identification of Naturally Occurring Epitopes

Naturally occurring epitopes may be identified by a divide-and-test process. One starts with a protein known to be antigenic or immunogenic. One next tests fragments of the protein for immunological activity. These fragments may be obtained by treatment of the protein with a proteolytic agent, or, if the peptide sequence is known, one may synthetically prepare smaller peptides corresponding to subsequences of the protein. The tested fragments may span the entire protein sequence, or just a portion thereof, and they may be abutting, overlapping, or separated.

For example, one may prepare a series of twenty-mer peptides with progressive overlap of 5 amino acids, e.g., residues 1-20, 16-35, 31-50, etc., of original polypeptide. The length of the peptides, and the degree of overlap, is up to the practitioner. The overlap is preferably at least five amino acids, or, more generally, the length of the smallest epitope of interest.

Fragments are readily prepared if the amino acid sequence of the peptide is known; a coding sequence may then be constructed for any desired fragment, and the fragment produced by recombinant DNA techniques. If the fragment is small, it may also be prepared by liquid or solid phase peptide synthesis.

If no sequence information is available, a polypeptide antigen may be fragmented with site-specific cleavage agents, such as cyanogen bromide, iodosobenzoic acid, and trypsin. Larger fragments may be obtained by using agents with rarer substrates, or by using the agents in low concentrations, at lower temperatures, or shorter reaction times. Smaller fragments may be obtained by using combinations of agents simultaneously, or by using high concentrations, higher temperatures, or longer reaction times, and optionally using chaotropic agents to help unfold the peptide. The fragments, large or small, are screened. Positive fragments may be fragmented further to localize the epitope.

If any of the fragments are immunologically active, the active fragments may themselves be subjected to a divide-and-test analysis, and the process may be continued until the minimal length immunologically active sequences are identified. This approach may be used to identify either B-cell or T-cell epitopes, although the assays will of course be different. Geysen teaches systematically screening all possible oligopeptide (pref. 6-10 a.a.) abutting or overlapping fragments of a particular protein for immunological activity in order to identify linear epitopes. See WO 84/03564.

The number of fragments to be screened may be reduced, if the amino acid sequence is known, by using the amino acid sequence to predict which fragments are likely to act as humoral or T-cell epitopes. In general, these predictive methods work by assembling a database of known humoral or T cell antigenic sites (and perhaps a second database of sequences known not to be humoral or T cell epitopes) and comparing these sites with (a) known 3-D structures of the proteins in question, and/or (b) the known amino acid sequence, especially in the vicinity of the site.

When testing a large number of fragments for humoral or T cell epitope activity, it is possible to use a divide-and-conquer strategy to minimize the number of test animals or cultures required. The fragments may be divided into two or more known groups and all fragments of a group administered to a single animal or culture. If no immune response is observed then all fragments if the mixture is positive, it may then be divided into smaller subgroups and the process repeated.

The fragments may be tested against T cells of a single haplotype, or of several different haplotypes.

Naturally occurring T-cell epitopes may be recovered by dissociating them from their complexes with MHC class I molecules and then sequencing them, e.g., by mass spectroscopic techniques.

Once an epitope is identified, functionally equivalent epitopes may be identified by a combination of knowledge of amino acid similarities and systematic variation of the sequence of the epitope.

### Prediction of B-cell (humoral) and T-cell epitopes

It is possible to predict the location of B-cell or T-cell peptide epitopes if an amino acid sequence is available. B-cell epitopes tend to be in regions of high local average hydrophilicity. See Hopp and Wood, Proc. Nat. Acad. Sci. (USA) 78: 3824 (1981); Jameson and Wolf, CABIOS, 4: 181 (1988).

T-cell epitopes can be predicted on the basis of known consensus sequences for the peptides bound to MHC class I molecules of cells of a particular haplotype. See e.g., Slingluff, WO98/33810, especially pp. 15-16; Parker, et al., "Scheme for ranking potential HLA-A2 binding peptides based on independent binding of individual peptide side chains", J. Immunol. 152: 163 (1994).

Margalit, et al., "Prediction of Immunodominant Helper T Cell Antigenic Sites from the Primary Sequence", J. Immunol., 138=2213-29 (1987) has identified (using the AMPHI program), a subset of T cell epitopes which are characterized by the presence of an amphipathic structure. In the Margalit algorithm, the amino acid sequence was converted into sequence of hydrophobicity values (the preferred scale was that of Fauchere-Pliska) and this sequence was divided into overlapping blocks (preferably of length 11). The blocks were examined for periodicity in hydrophobicity consistent with a regular amphipathic helical structure; the preferred power spectrum procedure is a least squares fit of a sinusoid. Margalit preferred to look for a segment of several conservative blocks which has an amphipathic score (the sum of the amphipathic indices of the blocks) of greater than 4.

Other algorithms may be developed by study of the T-cell epitope database.

### Mutant Epitopes

Generally speaking, in addition to epitopes which are identical to the naturally occurring disease- or tumor-specific epitopes, the present invention embraces epitopes which are different from but substantially identical with such epitopes, and therefore disease- or tumor-specific in their own right. It also includes epitopes which are not substantially identical to a naturally occurring epitope, but which are nonetheless cross-reactive with the latter as a result of a similarity in 3D conformation.

One class of allowable modifications of the amino acid sequence of a peptide moiety are amino acid substitutions. Conservative substitutions replace an amino acid with another of like size, charge and polarity; these are less likely to substantially alter the conformation of the peptide. The types of substitutions which may be made in the protein or peptide molecule of the present invention may be based on analysis of the frequencies of amino acid changes between a homologous protein of different species, such as those presented in Table 1-2 of Schulz et al., supra and Figs. 3-9 of Creighton, supra. Based on such an analysis, conservative substitutions are defined herein as exchanges within one of the following five groups:

**TABLE V**

| | |
|---|---|
| 1. | Small aliphatic, nonpolar or slightly polar residues: Ala, Ser, Thr (Pro, Gly); |
| 2. | Polar, negatively charged residues and their amides: Asp, Asn, Glu, Gln; |
| 3. | Polar, positively charged residues: His, Arg, Lys; |
| 4. | Large aliphatic, nonpolar or slightly polar |
| residues: | |
| | Met, Leu, Ile, Val (Cys); and |
| 5. | Large aromatic residues: Phe, Tyr, Trp. |
| Groups 1-3 are somewhat related and mutations within that set may be considered semi-conservative. Similarly, mutations within groups 4-5 may be considered semi-conservative. | |

Residues Pro, Gly and Cys are parenthesized because of their special role in protein architecture. Pro imparts rigidity to the peptide chain, and has a tendency to interfere with alpha helix formation. Gly imparts flexibility to the peptide chain, and is often found in "loops" between alpha helices or beta strands. The thiol groups of cysteine residues can be oxidized to form disulfide bonds between nonadjacent cysteinyl residues.

Within the foregoing groups, the following substitutions are considered "highly conservative":
Asp/Glu
His/Arg/Lys
Phe/Tyr/Trp
Met/Leu/Ile/Val

Semi-conservative substitutions are defined to be exchanges between two of groups (I)-(V) above which are limited to supergroup (A), comprising (I), (II) and (III) above, or to supergroup (B), comprising (IV) and (V) above. Also, Ala is considered a semi-conservative substitution for all non group I amino acids.

An epitope is considered substantially identical to a reference epitope (e.g., a naturally occurring epitope) if it has at least 10% of an immunological activity of the reference epitope and differs from the reference epitope by no more than one non-conservative substitution (except as provided below). Preferably, any non-conservative substitution is a semi-conservative substitution. Preferably, there are no non-conservative substitutions.

There may be any number of conservative substitutions. Preferably, there are no more than three such substitutions, more preferably, not more than two, and still more preferably, not more than one.

If it is a CTL epitope, it may incorporate further nonconservative substitutions which are suggested by a known binding motif of the pertinent MHC molecule. Kast, et al., J. Immunol, 152:3904-12 (1994) sets forth HLA-A specific peptide binding motifs for the HLA molecules A1, A2.1, A3, A11 and A24. Engelhard, et al., in Sette, ed., Naturally Processed Peptides, 57:39-62 (1993) explored the features that determined binding to HLA-A2.1 and HLA-B7. See also Hobohim et al; Eur. J. Immunol., 23:1271-6 (1993); Kawakami, et al., J. Immunol., 154:3961-8 (1995). Based on these and other sources, the preferred and tolerated AAs for various HLA molecules include (but are not limited to) the following:

**Table A**

| Molecule | Position | Preferred AA | tolerated AA |
|---|---|---|---|
| A1 | 2 | T, S, M | |
| | 3 | D, E | A, S |
| | 9 | Y | |
| A2.1 | 2 | L, M | I, V, A, T |
| | 9 | L, V, I | A, M, T |
| A3 | 2 | L, M, I, V, S | C, G, D |
| | | A, T, F | |
| | 9 | K, R, Y, H, F | A |
| A11 | 2 | M, L, I, V, S | C, D, F |
| | | A, T, G, N | |
| | 9 | K | R, H, Y |
| A24 | 2 | Y, F, W | M |
| | 9 | F, L, I, W | |
| B7 | 1 | A | M, S, R, L |
| | 2 | P | V |
| | 3 | R | A, K, S, M |
| | 9 | L | I, A, V |
| B8 | 3 | K | not known |
| | 5 | K | not known |
| | 9 | L | not known |
| B27 | 2 | R | not known |
| | 9 | R, K, H | not known |
| B35 | 2 | P | not known |
| | 9 | Y | not known |
| B53 | 2 | P | not known |

If a position is not listed, studies revealed a greater variability of AAs than for the listed positions. For listed positions, AAs not listed may be tolerated, especially if they are conservative or semi-conservative substitutions for "preferred" or "tolerated" Aas.

It will be appreciated that highly conservative substitutions are less likely to affect activity than other conservative substitutions, conservative substitutions are less likely to affect activity than merely semi-conservative substitutions, and semi-conservative substitutions less so than other non-conservative substitutions. In addition, single substitutions are less likely to affect activity than are multiple mutations.

Although a substitution mutant, either single or multiple, of the peptides of interest may not have quite the potency of the original peptide, such a mutant may well be useful.

Substitutions are not limited to the genetically encoded, or even the naturally occurring amino acids. When the epitope is prepared by peptide synthesis, the desired amino acid may be used directly. Alternatively, a genetically encoded amino acid may be modified by reacting it with an organic derivatizing agent that is capable of reacting with selected side chains or terminal residues.

A non-genetically encoded amino acid is considered a conservative substitution for a genetically encoded amino acid if it is more similar in size (volume) and hydrophobicity (lipophilicity) to the original amino acid, and to other amino acids in the same exchange group, than it is to genetically encoded amino acids belonging to other exchange groups.

Substantially identical peptide epitopes may be identified by a variety of techniques, some of which do not depend on preexisting knowledge of the binding motif. Thus, it is known in the art that one may synthesize all possible single substitution mutants of a known peptide epitope. For a nonpeptide, there are (20x9-1=179) such mutants. Geysen, et al., Proc Nat. Acad. Sci. (USA), 81:3998-4002 (1984). While the effects of different substitutions are not always additive, it is reasonable to expect that two favorable or neutral single substitutions at different residue positions in the epitope can safely be combined in most cases.
It is also known that one may randomly mutate one or more residues of a peptide so that any of the twenty possible amino acids, or a selected set (such as all conservative replacements), can occur at that residue position, and screen for mutants with a desired immunological activity. Parmley and Smith, Gene, 73:305-18 (1988); Devlin, et al., Science, 25:49:404-6 (1990); Scott and Smith, Science, 249:386-90 (1990); Greenwood, et al., J. Mol. Biol., 220:821-7 (1991); Cwirla, et al., Proc. Nat. Acad. Sci. (USA), 87:6378-82 (1990); Stephen and Lane, J. Mol. Biol., 225:577-83 (1992); Barrett, et al., Anal. Biochem., 204:357-64 (1992); Ladner, USP 5,223,409.

One might, for example, explore all possible conservative substitutions of a nonapeptide. There are 3-5 possibilities at each positions; assuming an average of 4, the sequence space would be 4^9, or about 250,000. A combinatorial library can readily exploring a sequence space of 10^8 or more.

Both naturally occurring and non-naturally occurring peptide epitopes may be identified, if a suitable antibody or other receptor is available, by screening a peptide combinatorial library for peptides bound by the target.

Humoral peptide epitopes may be identified by screening a combinatorial peptide phage library for specific binding to a target monoclonal antibody known to recognize the antigen of interest. Preferably, the library is prescreened to eliminate peptides which bind the antibody other than at the epitope binding site of the antibody; this can be done by eliminating phage which bind to a second, control antibody of the same isotype.

Similarly, to identify CTL peptide epitopes, one may synthesize a family of related single or multiple substitution mutants, present the mixture to the HLA-A2.1 positive lymphoblastoid cell line T2 (or other cell line capable of presenting specific CTL epitopes), and expose the T2 cells to CTLs of the desired specificity. If the T2 cells are lysed, the effective epitopes may be identified either by direct recovery from the T2 cells or by a progressive process of testing subsets of the effective peptide mixtures. Methods for the preparation of degenerate peptides are described in Rutter, USP 5,010,175, Haughten, et al., Proc. Nat. Acad. Sci. (USA), 82:5131-35 (1985), Geysen, et al., Proc. Nat. Acad. Sci. (USA), 81:3998-4002 (1984); W086/06487; W086/00991.

A multiple mutagenesis strategy was applied by Gavin, et al., Eur. J. Immunol., 24:2124-33 (1994), in their quest for peptides that mimic an H-Y epitope.

Multiple mutagenesis may be used to screen a few residue positions intensely or a larger number of positions more diffusely. One approach is to explore at least a representative member of each a.a. type at each position, e.g., one representative of each of exchange groups I-V as hereafter defined. Preferably, Gly and Pro are screened in addition to one other group I residue. Preferably, at least one screened residue is an H-bonding residue. If a positive mutant features a particular representative, like amino acids can be explored in a subsequent library. If, for example, a Phe substitution improves binding, Tyr and Trp can be examined in the next round.

The person of ordinary skill in the art, in determining which residues to vary, may also make comparisons of the sequences of the naturally processed MHC associated peptides, and may obtain 3D structures of the MHC: peptide: TCR complexes, in order to identify residues involved in MHC or TCR binding. Such residues may either be left alone, or judiciously mutated in an attempt to enhance MHC or TCR binding.

The peptide moiety may be a consensus peptide, e.g., a peptide, distinct from known cancer associated mucin core peptide sequences, but derived from a combination of known cancer associated mucin core peptide sequences. Such consensus peptides, may be derived by molecular modeling, optionally combined with hydrophobicity analysis and/or fitting to model helices, as non-limiting examples. Such modeling can be accomplished according to known method steps using known modeling algorithms, such as, but not limited to, ECEPP, INSIGHT, DISCOVER, CHEM-DRAW, AMBER, FRODO and CHEM-X. Such algorithms compare peptides to determine probable suitable alternative consensus polypeptide fragments.

### Liposome Formulations

Liposomes are microscopic vesicles that consist of one or more lipid bilayers surrounding aqueous compartments. See e.g., Bakker-Woudenberg et al., Eur. J. Clin. Microbiol. Infect. Dis. 12 (Suppl.1): S61 (1993) and Kim, Drugs, 46: 618 (1993). Because liposomes can be formulated with bulk lipid molecules that are also found in natural cellular membranes, liposomes generally can be administered safely and are biodegradable.

Liposomes are globular particles formed by the physical self-assembly of polar lipids, which define the membrane organization in liposomes. Liposomes may be formed as unilamellar or multi-lamellar vesicles of various sizes. Such liposomes, though constituted of small molecules having no immunogenic properties of their own, behave like macromolecular particles and display strong immunogenic characteristics.

Depending on the method of preparation, liposomes may be unilamellar or multilamellar, and can vary in size with diameters ranging from about 0.02 microm to greater than about 10 microm. A variety of agents can be encapsulated in liposomes. Hydrophobic agents partition in the bilayers and hydrophilic agents partition within the inner aqueous space(s). See e.g., Machy et al., Liposomes in Cell Biology and Pharmacology (John Libbey, 1987), and Ostro et al., American J. Hosp. Pharm. 46: 1576 (1989).

Liposomes can adsorb to virtually any type of cell and then release an incorporated agent. Alternatively, the liposome can fuse with the target cell, whereby the contents of the liposome empty into the target cell. Alternatively, a liposome may be endocytosed by cells that are phagocytic. Endocytosis is followed by intralysosomal degradation of liposomal lipids and release of the encapsulated agents. Scherphof et al., Ann. N.Y. Acad. Sci., 446: 368 (1985).

Other suitable liposomes that are used in the methods of the invention include multilamellar vesicles (MLV), oligolamellar vesicles(OLV), unilamellar vesicles (UV), small unilamellar vesicles (SUV),medium-sized unilamellar vesicles (MUV), large unilamellar vesicles (LUV), giant unilamellar vesicles (GUV), multivesicular vesicles (MVV), single or oligolamellar vesicles made by reverse-phase evaporation method (REV), multilamellar vesicles made by the reverse-phase evaporation method (MLV-REV), stable plurilamellar vesicles (SPLV), frozen and thawed MLV (FATMLV), vesicles prepared by extrusion methods (VET), vesicles prepared by French press (FPV), vesicles prepared by fusion (FUV),dehydration-rehydration vesicles (DRV), and bubblesomes (BSV). The skilled artisan will recognize that the techniques for preparing these liposomes are well known in the art. See Colloidal Drug Delivery Systems, vol. 66 (J. Kreuter, ed., Marcel Dekker, Inc., 1994).

A "liposomal formulation"is an in vitro-created lipid vesicles in which an antigen of the present invention can be incorporated. Thus, "liposomally-bound" refers to a antigen that is partially incorporated or attached to a liposome. The immunogen of the present invention may be a liposomally-bound antigen which, but for said liposome, would not be an immunogen, or it may be immunogenic even in a liposome-free state.

Several different glycolipopeptides may be incorporated into the same liposome, or each into a different liposome and the liposomes administered together or separately to a subject.

A glycolipopeptide can be incorporated into a liposome because the lipid portion of the molecule will spontaneously integrate into the lipid bilayer. Thus, a glycolipopeptide may be presented on the "surface" of a liposome. Alternatively, a peptide may be encapsulated within a liposome. Techniques for preparing liposomes and formulating them with molecules such as peptides are well known to the skilled artisan.

Formation of a liposome requires one or more lipids. Any lipids may be used which, singly or in combination, can form a liposome bilayer structure. Usually, these lipids will include at least one phospholipid. The phospholipids may be phospholipids from natural sources, modified natural phospholipids, semisynthetic phospholipids, fully synthetic phospholipids, or phospholipids (necessarily synthetic) with nonnatural head groups. The phospholipids of greatest interest are phosphatidyl cholines, phosphatidyl phosphatidyl ethanolamines, phosphatidyl serines, phosphatidyl glycerols, phosphatidic acids, and phosphatidyl inositols.

The liposome may include neutral, positively charged, and/or negatively charged lipids. Phosphatidyl choline is a neutral phospholipid. Phosphatidyl glycerol is a negatively charged glycolipid. N-[1-(2,3-dioleylox)propyl]-N,N,N-trimethylammonium chloride is a positively charged synthetic lipid. Another is 3beta-[N-(N',N"-dimethylaminoethane)-carbamoyl]-cholesterol.

Usually, the lipids will comprise one or more fatty acid groups. These may be saturated or unsaturated, and vary in carbon number, usually from 12-24 carbons. The phospholipids of particular interest are those with the following fatty acids: C12:0, C14:0, C16:0, C18:0, C18:1, C18:2, C18:3 (alpha and gamma), C20:0, C20:1, C20:3, C20:4, C20:5, C22:0, C22:5, C22:6, and C24:0, where the first number refers to the total number of carbons in the fatty acids chain, and the second to the number of double bonds. Fatty acids from mammalian or plant sources all have even numbers of carbon atoms, and their unsaturations are spaced at three carbon intervals, each with an intervening methylene group.

Cholesterol reduces the permeability of "fluid-crystalline state" bilayers.

A liposome may include lipids with a special affinity for particular target cells. For example, lactosylceramide has a specific affinity for hepatocytes (and perhaps also for liver cancer cells).

In a preferred liposome formulation, the component lipids include phosphatidyl choline. More preferably they also include cholesterol, and still more preferably, also phosphatidyl glycerol.

Taking advantage of the self-assembling properties of lipids, one or more immunogens may be attached to the polar lipids that in turn become part of the liposome particle. Each immunogen comprises one or more antigenic determinants (epitopes). These epitopes may be B-cell epitopes (recognized by antibodies) or T-cell epitopes (recognized by T-cells). The liposome can act to adjuvant the immune response elicited by the associated immunogens. It is likely to be more effective than an adjuvant that is simply mixed with an immunogen, as it will have a higher local effective concentration.

Moreover, a hapten may be attached in place of the aforementioned immunogen. Like an immunogen, a hapten comprises an antigenic determinant, but by definition is too small to elicit an immune response on its own (typically, haptens are smaller than 5,000 daltons). In this case, the lipid moiety may act, not only as an adjuvant, but also as an immunogenic carrier, the conjugate of the hapten and the lipid acting as a synthetic immunogen (that is, a substance against which humoral and/or cellular immune responses may be elicited).

Even if the lipid does not act as an immunogenic carrier, the liposome borne hapten may still act as a synthetic antigen (that is, a substance which is recognized by a component of the humoral or cellular immune system, such as an antibody or T-cell). The term "antigen" includes both haptens and immunogens.

### Adjuvants

It is generally understood that a synthetic antigen of low molecular weight can be weakly immunogenic, which is the biggest obstacle to the success of a fully synthetic vaccine. One way to improve the imunogenicity of such a synthetic antigen is to deliver it in the environment of an adjuvant. As conventionally known in the art, adjuvants are substances that act in conjunction with specific antigenic stimuli to enhance the specific response to the antigen. An ideal adjuvant is believed to non-specifically stimulate the immune system of the host, which upon the subsequent encounter of any foreign antigen can produce strong and specific immune response to that foreign antigen. Such strong and specific immune response, which is also characterized by its memory, can be produced only when T-lymphocytes (T-cells) of the host immune system are activated.

T-cell blastogenesis and IFN-g production as two important parameters for measuring the immune response. Experimentally, T-cell blastogenesis measures DNA synthesis that directly relates to T-cell proliferation, which in turn is the direct result of the T-cell activation. On the other hand, IFN-g is a major cytokine secreted by T-cells when they are activated. Therefore, both T-cell blastogenesis and IFN-g production indicate T-cell activation, which suggests the ability of an adjuvant in helping the host immune system to induce a strong and specific immune response to any protein-based antigen.

The compound is considered an adjuvant if it significantly (p=0.05) increases the level of either T-cell blastogenesis or of interferon gamma production in response to at least one liposome/immunogen combination relative to the level elicited by the immunogen alone. Preferably, it does both. Preferably, the increase is at least 10% , more preferably at least 50%, still more preferably, at least 100%.

A large number of adjuvants are known in the art, including Freund's complete adjuvant, saponin, DETOX (Ribi Immunochemicals), Montanide ISA-51, -50 and -70, QS-21, monophosphoryl lipid A and analogues thereof. A lipid adjuvant can be presented in the context of a liposome.

The present liposomal vaccines may be formulated advantageously with an adjuvant. Monophosphoryl lipid A (MPLA), for example, is an effective adjuvant that causes increased presentation of liposomal antigen to specific T Lymphocytes. Alving, C.R., Immunobiol., 187:430-446 (1993). The skilled artisan will recognize that lipid-based adjuvants, such as Lipid A and derivatives thereof, are also suitable. A muramyl dipeptide (MDP), when incorporated into liposomes, has also been shown to increase adjuvancity (Gupta RK et al., Adjuvants-A balance between toxicity and adjuvancity, " Vaccine, 11, 293-306 (1993)).

Use of an adjuvant is not required.

### Synthetic Strategy

Complex peptides and glycopeptides of pharmaceutical grade must adapt innovative processes that assures both quality and process controls from start to finish. Not every amino acid couples with another with same facility. Coupling points between some amino acids are sterically more hindered than others. Protected glycosylated amino acids (Figure 4) for glyco-peptide synthesis are bulkier than normal amino acids with protecting groups. The current strategy is to examine the peptide to be synthesised and identify the linkages that may be formed with relatively higher efficiency when it comes to linking two blocks of peptides. Such linkages are preserved till late stages of the assembly to enhance the efficiency of block coupling.

### Advantages:

Synthesis of peptides or glycopeptides of length and complexity has not been very feasible through conventional solid phase methods. Synthesis of appropriately protected small blocks in solution phase and their purification at every step is very feasible. Though this approach is labour intensive and time consuming in the beginning, scale-up production of pharmaceutical grade peptides is perhaps best handled through such strategy. At manufacturing scale it is also cost effective since expensive resins are dispensed with, while regulatory compliance is made a lot easier and quality and process controls are installed wherever needed. Such controls are nearly impossible with solid phase methods.

### Retro synthetic analysis:

Since the sequence and the structure of the final product is known, theoretically breaking down of the product into a minimum number of convenient blocks of smaller peptides so that their synthesis and assembly into final product is both convenient and efficient (figure 5). The following criteria are taken into consideration in dividing the product into smaller blocks in order to accomplish both coupling efficiency and cost effectiveness.

Peptide linkages that may be formed with relative ease in the sequence are identified so that smaller blocks may make use of such linkages of high efficiency in block coupling reactions.
Common blocks that repeat at two or more places in the sequence will reduce the burden of synthesis.

The coupling of expensive glycosylated amino acids is deferred, as much as possible, to the latter stages of the synthesis in order to minimize their loss through multi step coupling. N-terminal and C-terminal protecting groups are carefully chosen for their independent selective removal at will. The blocks are purified and tested for integrity using mass spectral and nuclear magnetic resonance spectral analysis. Deblocking of final product in acidic medium is maintained whenever possible, particularly in the case of carbohydrates. Final purification of the deblocked material is carried out on reverse phase column chromatography.

### Characterizing the Immune Response

The cell-mediated immune response may be assayed in vitro or in vivo. The conventional in vitro assay is a T cell proliferation assay. A blood sample is taken from an individual who suffers from the disease of interest, associated with that disease, or from a vaccinated individual. The T cells of this individual should therefore be primed to respond to a new exposure to that antigen by proliferating. Proliferation requires thymidine because of its role in DNA replication.

Generally speaking, T cell proliferation is much more extensive than B cell proliferation, and it may be possible to detect a strong T cell response in even an unseparated cell population. However, purification of T cells is desirable to make it easier to detect a T cell response. Any method of purifying T cells which does not substantially adversely affect their antigen-specific proliferation may be employed. In our preferred procedure, whole lymphocyte populations would be first obtained via collection (from blood, the spleen, or lymph nodes) on isopycnic gradients at a specific density of 10.7, ie Ficoll-Hypague or Percoll gradient separations. This mixed population of cells could then be further purified to a T cell population through a number of means. The simplest separation is based on the binding of B cell and monocyte/macrophage populations to a nylon wool column. The T cell population passes through the nylon wool and a >90% pure T population can be obtained in a single passage. Other methods involve the use of specific antibodies to B cell and or monocyte antigens in the presence of complement proteins to lyse the non-T cell populations (negative selection). Still another method is a positive selection technique in which an anti-T cell antibody (CD3) is bound to a solid phase matrix (such as magnetic beads) thereby attaching the T cells and allowing them to be separated (e.g., magnetically) from the non-T cell population. These may be recovered from the matrix by mechanical or chemical disruption.

Once a purified T cell population is obtained it is cultured in the presence of irradiated antigen presenting cells (splenic macrophages, B cells, dendritic cells all present). (These cells are irradiated to prevent them from responding and incorporating tritiated thymidine). The viable T cells (100,000-400,000 per well in 100µl media supplemented with IL2 at 20 units) are then incubated with test peptides or other antigens for a period of 3 to 7 days with test antigens at concentrations from 1 to 100µg/mL.

At the end of the antigen stimulation period a response may be measured in several ways. First the cell free supernatants may be harvested and tested for the presence of specific cytokines. The presence of α-interferon, IL2 or IL12 are indicative of a Th helper type 1 population response. The presence of IL4, IL6 and IL10 are together indicative of a T helper type 2 immune response. Thus this method allows for the identification of the helper T cell subset.

A second method termed blastogenesis involves the adding tritiated thymidine to the culture (e.g., 1µcurie per well) at the end of the antigen stimulation period, and allowing the cells to incorporate the radiolabelled metabolite for 4-16 hours prior to harvesting on a filter for scintillation counting. The level of radioactive thymidine incorporated is a measure of the T cell replication activities. Negative antigens or no antigen control wells are used to calculated the blastogenic response in terms of a stimulation index. This is CPM test/CPM control. Preferably the stimulation index achieved is at least 2, more preferably at least 3, still more preferably 5, most preferably at least 10.
CMI may also be assayed in vivo in a standard experimental animal, e.g., a mouse. The mouse is immunized with a priming antigen. After waiting for the T cells to respond, the mice are challenged by footpad injection of the test antigen. The DTH response (swelling of the test mice is compared with that of control mice injected with, e.g., saline solution.

Preferably, the response is at least .10 mm, more preferably at least .15 mm, still more preferably at least .20 mm, most preferably at least .30 mm.

The humoral immune response, in vivo, is measured by withdrawing blood from immunized mice and assaying the blood for the presence of antibodies which bind an antigen of interest. For example, test antigens may be immobilized and incubated with the samples, thereby capturing the cognate antibodies, and the captured antibodies then measured by incubating the solid phase with labeled anti-isotypic antibodies.

Preferably, the humoral immune response, if desired, is at least as strong as that represented by an antibody titer of at least 1/100, more preferably at least 1/1000, still more preferably at least 1/10.000.

### Subjects

The recipients of the vaccines of the present invention may be any vertebrate animal which can acquire specific immunity via a humoral or cellular immune response.

Among mammals, the preferred recipients are mammals of the Orders Primata (including humans, apes and monkeys), Arteriodactyla (including horses, goats, cows, sheep, pigs), Rodenta (including mice, rats, rabbits, and hamsters), and Carnivora (including cats, and dogs). Among birds, the preferred recipients are turkeys, chickens and other members of the same order. The most preferred recipients are humans.

The preferred animal subject of the present invention is a primate mammal. By the term "mammal" is meant an individual belonging to the class Mammalia, which, of course, includes humans. The invention is particularly useful in the treatment of human subjects, although it is intended for veterinary uses as well. By the term "non-human primate" is intended any member of the suborder Anthropoidea except for the family Hominidae. Such non-human primates include the superfamily Ceboidea, family Cebidae (the New World monkeys including the capuchins, howlers, spider monkeys and squirrel monkeys) and family Callithricidae (including the marmosets); the superfamily Cercopithecoidea, family Cercopithecidae (including the macaques, mandrills, baboons, proboscis monkeys, mona monkeys, and the sacred hunaman monkeys of India); and superfamily Hominoidae, family Pongidae (including gibbons, orangutans, gorillas, and chimpanzees). The rhesus monkey is one member of the macaques.

### Pharmaceutical Compositions

Pharmaceutical preparations of the present invention, comprise at least one immunogen in an amount effective to elicit a protective immune response. The response may be humoral, cellular, or a combination thereof. The composition may comprise a plurality of immunogens.

At least one immunogen will be either a glycolipopeptide which is immunogenic per se, or a glycolipopeptide which is immunogenic as a result of its incorporation into a liposome.

The composition preferably further comprises a liposome. Preferred liposomes include those identified in Jiang,et al., PCT/US00/31281, filed Nov. 15, 2000 (our docket JIANG3A-PCT), and Longenecker, et al., 08/229,606, filed April 12, 1994 (our docket LONGENECKER5-USA, and PCT/US95/04540, filed April 12, 1995 (our docket LONGENECKER5-PCT).

The composition may comprise antigen-presenting cells, and in this case the immunogen may be pulsed onto the cells, prior to administration, for more effective presentation.

The composition may contain auxiliary agents or excipients which are known in the art. See, e.g., Berkow et al, eds., The Merck Manual, 15th edition, Merck and Co., Rahway, N.J., 1987; Goodman et al., eds., Goodman and Gilman's The Pharmacological Basis of Therapeutics, 8th edition, Pergamon Press, Inc., Elmsford, N.Y., (1990); Avery's Drug Treatment: Principles and Practice of Clinical Pharmacology and Therapeutics, 3rd edition, ADIS Press, LTD., Williams and Wilkins, Baltimore, MD. (1987), Katzung, ed. Basic and Clinical Pharmacology, Fifth Edition, Appleton and Lange, Norwalk, Conn. (1992), which references and references cited therein, are entirely incorporated herein by reference.

A composition may further comprise an adjuvant to nonspecifically enhance the immune response. Some adjuvants potentiate both humoral and cellular immune response, and other s are specific to one or the other. Some will potentiate one and inhibit the other. The choice of adjuvant is therefore dependent on the immune response desired.

A composition may include immunomodulators, such as cytokines which favor or inhibit either a cellular or a humoral immune response, or inhibitory antibodies against such cytokines.

A pharmaceutical composition according to the present invention may further comprise at least one cancer chemotherapeutic compound, such as one selected from the group consisting of an anti-metabolite, a bleomycin peptide antibiotic, a podophyllin alkaloid, a *Vinca* alkaloid, an alkylating agent, an antibiotic, cisplatin, or a nitrosourea. A pharmaceutical composition according to the present invention may further or additionally comprise at least one viral chemotherapeutic compound selected from gamma globulin, amantadine, guanidine, hydroxybenzimidazole, interferon-α, interferon-β, interferon-γ, thiosemicarbarzones, methisazone, rifampin, ribvirin, a pyrimidine analog, a purine analog, foscarnet, phosphonoacetic acid, acyclovir, dideoxynucleosides, or ganciclovir. See, e.g., Katzung, *supra,* and the references cited therein on pages 798-800 and 680-681, respectively, which references are herein entirely incorporated by reference.

Anti-parasitic agents include agents suitable for use against arthropods, helminths (including roundworns, pinworms, threadworms, hookworms, tapeworms, whipworms, and Schistosomes), and protozoa (including amebae, and malarial, toxoplasmoid, and trichomonad organisms). Examples include thiabenazole, various pyrethrins, praziquantel, niclosamide, mebendazole, chloroquine HCl, metronidazole, iodoquinol, pyrimethamine, mefloquine HCl, and hydroxychloroquine HCl.

### Pharmaceutical Purposes

A purpose of the invention is to protect subjects against a disease. The term "protection", as in "protection from infection or disease", as used herein, encompasses "prevention," "suppression" or "treatment." "Prevention" involves administration of a Pharmaceutical composition prior to the induction of the disease. "Suppression" involves administration of the composition prior to the clinical appearance of the disease. "Treatment" involves administration of the protective composition after the appearance of the disease. Treatment may be ameliorative or curative.

It will be understood that in human and veterinary medicine, it is not always possible to distinguish between "preventing" and "suppressing" since the ultimate inductive event or events may be unknown, latent, or the patient is not ascertained until well after the occurrence of the event or events. Therefore, it is common to use the term "prophylaxis" as distinct from "treatment" to encompass both "preventing" and "suppressing" as defined herein. The term "projection," as used herein, is meant to include "prophylaxis." See, e.g., Berker, *supra,* Goodman, *supra*, Avery, *supra* and Katzung, *supra,* which are entirely incorporated herein by reference, including all references cited therein.

The "protection" provided need not be absolute, i.e., the disease need not be totally prevented or eradicated, provided that there is a statistically significant improvement (p=0.05) relative to a control population. Protection may be limited to mitigating the severity or rapidity of onset of symptoms of the disease. An agent which provides protection to a lesser degree than do competitive agents may still be of value if the other agents are ineffective for a particular individual, if it can be used in combination with other agents to enhance the level of protection, or if it is safer than competitive agents.

The effectiveness of a treatment can be determined by comparing the duration, severity, etc. of the disease posttreatment with that in an untreated control group, preferably matched in terms of the disease stage.

The effectiveness of a prophylaxis will normally be ascertained by comparing the incidence of the disease in the treatment group with the incidence of the disease in a control group, where the treatment and control groups were considered to be of equal risk, or where a correction has been made for expected differences in risk.

In general, prophylaxis will be rendered to those considered to be at higher risk for the disease by virtue of family history, prior personal medical history, or elevated exposure to the causative agent.

### Pharmaceutical Administration

At least one protective agent of the present invention may be administered by any means that achieve the intended purpose, using a pharmaceutical composition as previously described.

Administration may be oral or parenteral, and, if parenteral, either locally or systemically. For example, administration of such a composition may be by various parenteral routes such as subcutaneous, intravenous, intradermal, intramuscular, intraperitoneal, intranasal, transdermal, or buccal routes. Parenteral administration can be by bolus injection or by gradual perfusion over time. A preferred mode of using a pharmaceutical composition of the present invention is by subcutaneous, intramuscular or intravenous application. See, e.g., Berker, *supra*, Goodman, *supra*, Avery, *supra* and Katzung, *supra,* which are entirely incorporated herein by reference, including all references cited therein.

A typical regimen for preventing, suppressing, or treating a disease or condition which can be alleviated by an immune response by active specific immunotherapy, comprises administration of an effective amount of a pharmaceutical composition as described above, administered as a single treatment, or repeated as enhancing or booster dosages, over a period up to and including between one week and about 24 months.

It is understood that the effective dosage will be dependent upon the age, sex, health, and weight of the recipient, kind of concurrent treatment, if any, frequency of treatment, and the nature of the effect desired. The ranges of effective doses provided below are not intended to limit the invention and represent preferred dose ranges. However, the most preferred dosage will be tailored to the individual subject, as is understood and determinable by one of skill in the art, without undue experimentation. This will typically involve adjustment of a standard dose, e.g., reduction of the dose if the patient has a low body weight. See, e.g., Berkow et al, eds., The Merck Manual, 15th edition, Merck and Co., Rahway, N.J., 1987; Goodman et al., eds., Goodman and Gilman's The Pharmacological Basis of Therapeutics, 8th edition, Pergamon Press, Inc., Elmsford, N.Y., (1990); Avery's Drug Treatment: Principles and Practice of Clinical Pharmacology and Therapeutics, 3rd edition, ADIS Press, LTD., Williams and Wilkins, Baltimore, MD. (1987), Ebadi, Pharmacology, Little, Brown and Co., Boston, (1985); Chabner et al., *supra;* De Vita et al., *supra;* Salmon, *supra;* Schroeder et al., *supra;* Sartorelli et al., *supra;* and Katsung, *supra,* which references and references cited therein, are entirely incorporated herein by reference.

Prior to use in humans, a drug will first be evaluated for safety and efficacy in laboratory animals. In human clinical studies, one would begin with a dose expected to be safe in humans, based on the preclinical data for the drug in question, and on customary doses for analogous drugs (if any). If this dose is effective, the dosage may be decreased, to determine the minimum effective dose, if desired. If this dose is ineffective, it will be cautiously increased, with the patients monitored for signs of side effects. See, e.g., Berkow, et al., eds., The Merck Manual, 15th edition, Merck and Co., Rahway, N.J., 1987; Goodman, et al., eds., Goodman and Gilman's The Pharmacological Basis of Therapeutics, 8th edition, Pergamon Press, Inc., Elmsford, N.Y., (1990); Avery's Drug Treatment: Principles and Practice of Clinical Pharmacology and Therapeutics, 3rd edition, ADIS Press, LTD., Williams and Wilkins, Baltimore, MD. (1987), Ebadi, Pharmacology, Little, Brown and Co., Boston, (1985), which references and references cited therein, are entirely incorporated herein by reference.

The total dose required for each treatment may be administered in multiple doses (which may be the same or different) or in a single dose, according to an immunization schedule, which may be predetermined or ad hoc. The schedule is selected so as to be immunologically effective, i.e., so as to be sufficient to elicit an effective immune response to the antigen and thereby, possibly in conjunction with other agents, to provide protection. The doses adequate to accomplish this are defined as "therapeutically effective doses." (Note that a schedule may be immunologically effective even though an individual dose, if administered by itself, would not be effective, and the meaning of "therapeutically effective dose" is best interpreted in the context of the immunization schedule.) Amounts effective for this use will depend on, e.g., the peptide composition, the manner of administration, the stage and severity of the disease being treated, the weight and general state of health of the patient, and the judgment of the prescribing physician.

Typically, the daily dose of an active ingredient of a pharmaceutical, for a 70 kg adult human, is in the range of 10 nanograms to 10 grams. For immunogens, a more typical daily dose for such a patient is in the range of 10 nanograms to 10 milligrams, more likely 1 microgram to 10 milligrams. However, the invention is not limited to these dosage ranges.

It must be kept in mind that the compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

The doses may be given at any intervals which are effective. If the interval is too short, immunoparalysis or other adverse effects can occur. If the interval is too long, immunity may suffer. The optimum interval may be longer if the individual doses are larger. Typical intervals are 1 week, 2 weeks, 4 weeks (or one month) , 6 weeks, 8 weeks (or two months) and one year. The appropriateness of administering additional doses, and of increasing or decreasing the interval, may be reevaluated on a continuing basis, in view of the patient's immunocompetence (e.g., the level of antibodies to relevant antigens).

A variety of methods are available for preparing liposomes, as described in, e.g., Szoka et al., Ann. Rev. Biophys. Bioeng. 9:467 (1980), U.S. Patent Nos. 4,235,871, 4,501,728, 4,837,028, and 5,019369, incorporated herein by reference.

The appropriate dosage form will depend on the disease, the immunogen, and the mode of administration; possibilities include tablets, capsules, lozenges, dental pastes, suppositories, inhalants, solutions, ointments and parenteral depots. See, e.g., Berker, supra, Goodman, supra, Avery, supra and Ebadi, supra, which are entirely incorporated herein by reference, including all references cited therein.

The antigen may be delivered in a manner which enhance, e.g., delivering the antigenic material into the intracellular compartment such that the "endogenous pathway" of antigen presentation occurs. For example, the antigen may be entrapped by a liposome (which fuses with the cell), or incorporated into the coat protein of a viral vector (which infects the cell).

Another approach, applicable when the antigen is a peptide, is to inject naked DNA encoding the antigen into the host, intramuscularly. The DNA is internalized and expressed.

It is also possible to prime autologous PBLs with the compositions of the present invention, confirm that the PBLs have manifested the desired response, and then administer the PBLs, or a subset thereof, to the subject.

### Examples:

Two glycolipopeptides **1a** and **1b** are synthesized by block coupling method in solution phase. Compound **1a** (figure 6) contains two Tn-threonines and one Tn-serine where as the compound **1b** (figure 6) contains two Tn-threonines, one Tn-serine and one STn-serine (Tn: aGalNAc-O-; STn: SialylTn, Neu5Acα(2-6)αGalNAc-O-) . The strategy for the synthesis of **1a** and **1b** is presented in the retro synthetic plan (Figure 5). The final glycopeptides would be obtained by deblocking the corresponding precursors **2a** and **2b,** which could be prepared by coupling of the two blocks, 20-mer **3** and 23-mer **4a** or **4b.** The 20-mer was further dissected into 11-mer **5** and 9-mer **6.** Similarly, the 23 mer **4a** and **4b** were also made into 11-mer **7** and 12-mer **8.** Blocks **5** and **7** were further divided into primary blocks **9, 10** and **9/11, 12.** The block **8** was further divided into block **14** and the block **13,** which is similar to **6.** The block **14** is the serine-serine-leucine (S*S*L) triad in which serines were attached to lipid chains and was designed to serve as lipid carrier in the final glycolipopeptide. The primary blocks **5, 6, 9, 10, 11, 12, 13** and **14** (Figure 7) are synthesized from the individual glycosylated and unglycosylated protected amino acids.

### Synthesis of the primary blocks:

The lipo-amino acid triad, **14** (Figure 8) was synthesized from the Boc protected lipo serine, which in turn was synthesized from Boc protected serine and bromo tetradecane in 55% yield by treating with NaH in DMF. The lipo-serine was coupled to leucine methyl ester by DCC/HOBt method. The Boc group was deprotected with HCl from the resulted Boc-lipo-serine-leucine methyl ester and was coupled to another Boc lipo-serine to give **14** in 84% yield.

The blocks **6** and **13** (Figure 9) were synthesized from a common block APPAHGV which was synthesized from the individual protected amino acids. Trityl protected Fmoc-histidine was coupled to glycine benzyl ester by DCC/HOBt method in 89% yield to give protected HG block from which benzyl ester was cleaved by treating with ammonium formate in presence of palladium charcoal in THF-methanol mixture at 6°C. The free acid was coupled to the free amine of valine benzyl ester by DCC/HOBt method. The Fmoc group was deblocked by treating with morpholine at room temperature and coupled to Fmoc-PA-OH and subsequently with Fmoc-AP-OH to give the common block of Fmoc APPAH(Trt)GV benzyl ester. This block was treated first with morpholine to remove Fmoc protection and then coupled to Fmoc protected S(tBu)T(tBu)-OH by DCC/HOBt method to give block **13** in 91% yield. The free amine of APPAH(Trt)GV benzyl ester was coupled successively to glycosylated amino acids, Tn-thronine and Tn-serine by the same method to give block **6** in 60% overall yield.

Block **9** was synthesized (Figure 10) starting from the coupling of tBu protected threonine and serine by DCC/NHS method to give TS block which was treated with morpholine and the resulting free amine was treated with Fmoc protected AP-OH again by the same method. The resulting tetrapeptide was coupled to tBu protected aspartic acid to give block **9** in 75% yield. The similar block **11** which contains glycosylated amino acid, STn-serine, was synthesized (Figure 11) by the sequential coupling of Fmoc protected AP-OH to tBu protected aspartic acid benzyl ester and then to STn-serine and tBu-threonine by DCC/HOBt method. Finally deblocking of Fmoc protection by morpholine gave **11** in about 80% overall yield.

Blocks **10** and **12** were synthesized (Figure 12) from the common block Fmoc RPAPG benzyl ester, which in turn was synthesized starting from Boc protected proline coupled with glycine benzyl ester, which was coupled to Fmoc PA-OH block after deblocking Boc group by DCC/HOBt method. The resulted PAPG block was coupled to Pmc protected arginine to give Fmoc RPAPG benzyl ester. This after Fmoc deprotection when coupled to tBu protected threonine gave block **10** whereas when coupled to Tn-threonine gave block **12** in 82 and 83% yields respectively.

### Synthesis of glycolipopeptide (1a/1b) by block coupling:

The final steps of assembly of glycolipopeptides **1a** and **1b** were carried out using appropriate blocks at various stages in accordance with the planned strategy (Figure 13). The synthesis of the seven primary blocks starting from the individual suitably protected amino acids was described above. All these primary blocks were coupled appropriately to give the corresponding intermediate secondary blocks, which would lead to the fully blocked compounds **2a** and **2b,** precursors to the final compounds **1a** and **1b,** respectively. Block **9** was treated with DCC and NHS to make the corresponding succinimide and coupled to the free amine of **10** (treated with morpholine to deblock Fmoc group) in presence of base in DMF to give block **5** in 45% yield. Similarly, block **9** was coupled to free amine of **12** to give block **7a** in 67% yield. Free acid of **11** (treated with ammonium formate and palladium charcoal in THF-MeOH at 6°C to deblock benzyl ester) was coupled to free amine of **12** by DCC, HOBt method to give block **7b** in 90% yield. Similarly, free acid of **13** was coupled to Boc deprotected **14** by DCC/HOBt method to give block **8** in 92% yield. Further couplings of the rest of the blocks were all carried out by DCC/HOBt method. Coupling of free amine of **8** to the free acid of **7a** gave **4a** in 90% yield whereas to the free acid of **7b** resulted in 63% yield of **4b**. **6** was treated with morpholine to release free amine and then coupled with the free acid of **5** to give **3** in 95% yield.
Coupling of the free acid of **3** to the free amine of **4a** led to the precursor **2a** whereas to the free amine of **4b** led to the other precursor **2b**. Both **2a** and **2b** upon three step deblocking of all the protecting groups gave the final compounds **1a** and **1b** respectively in an average yield of 60%. After the work up the final products were desalted and purified by RP-HPLC on C4 column using acetonitrile-water system.

Thus very few protecting groups, Fmoc and Boc for amine functionalities and benzyl ester and methyl ester for acid functionalities, have been used in this peptide synthesis. All other amino acids were suitably protected with either tBu (for threonine, serine and aspartic acid) or Pmc (for arginine) or Trt (for histidine) groups. Glycosylated (Tn and STn) serines and threonines with appropriate protecting groups were synthesised and used where appropriate in the blocks. Similarly, pre-synthesised lipo-serine was used in the synthesis of appropriate blocks. Thus the entire synthesis was accomplished with a minimum diversity in chemical reactions. Just two methods of coupling, viz., DCC/NHS method and DCC/HOBt method and standard deblocking methods for Fmoc and Boc were used in the entire synthesis. Hence, a brief description of the general methods of coupling and decoupling is given.

### I. Coupling Methods:

There are two procedures used in the synthesis of the peptide blocks or in the block coupling, viz., DCC/NHS method and DCC/HOBt method. Both methods gave very good yields.

### DCC/NHS Method:

Anhydrous THF was added to the flask containing Fmoc protected amino acid or peptide block **A** (14.33 mmol), N-hydroxy-succinimide (17.14 mmol) and DCC (17.15 mmol) under nitrogen atmosphere and stirred for overnight. The urea formed as white solid was filtered off and washed with THF. The solvent was removed from the combined filtrate and washings under reduced pressure. The succinimide was used either as crude, or after purification by column chromatography or crystallization. The succinimide derivative was coupled to the next amino acid in two methods.

**Method A**: The succinimide derivative was dissolved in DMF (120 ml) and the suitably protected amino acid or peptide block B, with free amino and carboxylic functionalities (14.29 mmol) and DIEA (2.5 ml) were added. The suspension was stirred at RT overnight, in the mean time it became clear solution. DMF was removed and the residue was redissolved in DCM, washed with water (75 ml) and brine (75 ml) and dried over anhydrous sodium sulfate. The DCM layer was concentrated to give the crude amino acid block **AB**, which was purified by column chromatography using (3-5%) methanol in DCM with 0.5% acetic acid. The pure block **AB** was obtained as a white solid after removal of the solvent in about 75% yield.

**Method B**: The amino acid **B** (5.42 mmol) was dissolved in water and sodium bicarbonate (4.5 mmol) was added. The mixture was stirred for 30 min and the succinimide derivative of block **A** (3.0 mmol) dissolved in 1,2-dimethoxy-ethane (250 ml) was added. The reaction mixture was stirred for overnight at RT. The solvent was removed on rotavapor, a white slurry was resulted which was cooled at 0°C and acidified with 10% HCl until pH 4-5. DCM was added to this mixture and stirred for a few min and the contents were transferred into a separating funnel. The DCM layer was separated and the aqueous layer was extracted with DCM (X 2). The combined organic layer was dried over anhydrous sodium sulfate and concentrated to dryness. The residue was recrystallised with hot ethyl acetate and hexane to give pure peptide block **AB** in about 80% yield.

### DCC/HOBt Coupling:

The amino acid or block **A** with free carboxylic acid group (4.09 mmol), HOBt (4.46 mmol) and block **B** with free amine (3.72 mmol) were dissolved in about 50 ml dry DCM. DCC (7.44 mmol) was dissolved in 10 ml dry DCM and added to the above mixture at RT dropwise slowly. The reaction mixture was stirred at RT for overnight. About 0.4 ml water was added to the reaction mixture and stirred for 30 min to convert all the unreacted DCC into the urea. The urea formed was removed by filtration on anhydrous sodium sulfate bed and the DCM was removed completely and the residue was purified by silica gel column chromatography using 4-5% methanol in DCM. The pure product of the peptide block **AB** was obtained after removal of the solvent in about 85-97% yield.

### II. Deblocking Methods:

**Fmoc Deprotection:** Fmoc protected amino acid (1.0 mmol) was stirred with morpholine (10 ml) at RT for 30 min. When TLC indicated the completion of the reaction, morpholine was removed under reduced pressure, co-distilled with toluene. The residue was dried under high vacuum and used as crude in the next step, or purified by silica gel column chromatography using 5-10% methanol in DCM. The yield of free amine obtained was about 92-95%.

**Boc Deprotection:** To the ethylacetate solution of Boc protected compound (19.0 mmol) was added 10 ml conc. HCl and stirred for about 30 min. When TLC indicated the completion of the reaction, solvents were removed under reduced pressure. The residue was dissolved in 200 ml DCM and washed with aqueous sodium bicarbonate. The aqueous layer was back extracted with DCM several times. The combined organic layers were concentrated and dried under high vacuum. The crude amine was obtained in quantitative yields and can be carried out to the next step as it is.

**Benzyl ester Deprotection:** Benzyl ester (0.3 mol) was dissolved in 10 ml THF:MeOH (1:1) mixture and 100 mg of Pd carbon was added. The mixture was cooled to 6°C and ammonium formate (4.0 mmol) was added and stirred at 6°C for 40 min. If the TLC showed incomplete reaction then more quantities of Pd/C and ammonium formate were required to add and continued the reaction until TLC shows the absence of starting materials. When TLC indicated the completion of the reaction, the reaction mixture was filtered on a small celite bed to remove Pd/C and washed the catalyst successively with methanol and DCM. The combined filtrates were concentrated and the residue was dissolved in minimum amount of DCM. A white solid was precipitated out which was discarded. The filtrate was concentrated to get crude free acid and was purified by silica gel column chromatography using 7-10% methanol in DCM with 1% acetic acid. The pure compound was obtained as a white solid that was redissolved in methanol and co-distilled with toluene to remove the traces of acetic acid present. If still there was any trace of acetic acid present in the compound, then it was dissolved in excess amount of chloroform and washed with small quantities of brine (X 4). The chloroform layer was dried and concentrated to give free amino acid about 85-90% yields.

### III. Final Deblocking of the glycolipopeptide:

**Deblocking of the acid sensitive groups (with Reagent B):** Fully blocked glycolipopeptide **2a** or **2b** (0.08 mmol) was stirred with 60 ml of the cocktail Reagent B (TFA : Phenol : Triisopropyl silane : Water, 8.8:0.5:0.2:0.5) at RT for 2h. The solvent was removed under reduced pressure up to 90% and cold ether (200 ml) was added. A white precipitate was formed which was filtered through a fine sintered funnel, washed the precipitate with lot of cold ether to wash off phenol. The compound was finally dissolved in methanol, concentrated and dried under high vacuum. TLC indicated that the reaction was complete and the yield was normally about 95-98%.

**Deblocking the base sensitive groups (with sodium methoxide):** The product from the above step (0.055 mmol) was dissolved in 20 ml dry methanol and 1.5 ml of NaOMe (0.3M) solution in methanol was added slowly while checking the pH of the reaction mixture. The pH should be around 9-10 and the overall concentration of the base should be around 0.02M. The mixture was stirred at RT for 3h and acidified with 5% acetic acid in methanol until pH of the reaction mixture changes to about 6. The solvents were removed under reduced pressure and the residue was dissolved in water (20 ml) and extracted with ether (15 ml X 1). The aqueous layer was separated and lyophilized to get a white fluffy solid as a product, which also contains some salt (sodium acetate).

**Deblocking methyl ester(s)**: The product from the above step (0.06 mmol) was dissolved in 20 ml of DMF:water (7:1) mixture and cooled at 0°C. 5ml of 0.1N solution of NaOH was added dropwise to the reaction mixture to make the overall concentration of the base to 0.02M solution. The reaction mixture was stirred at 0°C for 2h and acidified with 5% acetic acid in methanol until pH changed to about 6. The solvents were removed under reduced pressure and dried under high vacuum to give crude product **1a** or **1b**.

### IV. Desalting Procedure:

The crude product is desalted prior to RP-HPLC purification. A column was packed with C4 material in about 100 ml of methanol and eluted for an hour with the same solvent. Gradually the solvent was changed from 100% methanol to 100% water (by slowly increasing the water contents in order to avoid the heat liberation with the sudden change of the solvent). The column was eluted for another hour with water. The product was desolved in water and purified by first eluting with about 500 ml water and then switched to 60% acetonitrile in water. The salts were eluted first with water followed by the impurities in 60% acetonitrile in water. Then the solvent was changed to 90% methanol in water. The product was started eluting out along with its polar impurities slowly and was completely out when the solvent was changed to 100% methanol. The fractions containing the product were all pooled together and removed the solvent under reduced pressure. The desalted crude product was further purified by RP-HPLC (Figures 14 and 15) using water and acetonitrile with 0.1% TFA on a C4 column.

### V. Spectroscopic and analytical data for selected blocks:

### Block 6:

MS m/z (%): 2203.9 [M+Na]⁺ (100), 2181 (35), 113.6(10).
¹HNMR: 300MHz (CDCl₃+CD₃OD) d 0.82 (d, J = 5 Hz, 3H, C*H*₃ (Val)), 0.85 (d, J = 5 Hz, 3H, C*H*₃ (Val)), 1.2-1.25 (m, 6H), 1.3 (d, J= 7Hz, 3H, C*H*₃ (Thr)), 1.6-1.9 (m, 8H, C*H*₂-C*H*, (Pro)), 1.75 (s, 3H, COC*H*₃), 1.85 (s, 3H, COC*H*₃), 2.2 (m, 2H), 2.95 (d, J = 6Hz, 2H, C*H*₂ (His)), 3.35-3.6 (m, 4H, N-C*H*₂ (Pro)), 3.8 (m, 5H), 3.9-4.6 (m, 20H), 4.73-4.85 (m, 2H), 5.0 (m, 1H), 5.05 (dd, J = 12, 10 Hz, 2H, C*H*₂C₆H₅), 5.2-5.3 (m, 3H), 5.45 (s, 1H, C*H*C₆H₅), 5.5 (s, 1H, C*H*C₆H₅), 6.7 (s, 1H, CH (His)), 7.1 (m, 6H, 5ArH, C*H* (His)), 7.2-7.8 (m, 43H, 8 ArH (Fmoc), 35ArH), 8.0 (m, 4H, NH). [α] _{D}²⁰ = +27.5 © 0.1, CHCl₃)

### Block 9:

MS m/z (%): 902.5 [M+Na]⁺ (100), 880 (10), 846.4 (25).
¹HNMR: 500 MHZ (CDCl₃+CD₃OD) d 1.09 (d, J = 11.5 Hz, 3H, C*H*₃ (Thr)), 1.19 (s, 9H, C(C*H*₃)₃), 1.32 (s, 9H, C(C*H*₃)₃), 1.39 (d, J = 12 Hz, 3H, C*H*₃ (Ala) ), 1.45 (s, 9H, C(C*H*₃)₃), 1.9-2.1 (m, 4H, C*H*₂-C*H*₂ (Pro)), 2.76 (dd, J = 17, 5 Hz, 1H, C*H*H (Asp)), 2.86 (dd, J = 17, 5 Hz, 1H, CH*H* (Asp)), 3,48 (dd, J = 9, 5 Hz, 1H, CH*H* (Ser)), 3.64-3.74 (m, 2H, N-C*H*₂ (Pro)), 3.85 (dd, J = 9, 4 Hz, C*H*H (Ser)), 4.12 (m, 1H, C*H* (Thr)), 4.25 (m, 2H, 2C*H* (Pro, Ala)), 4.40-4.50 (m, 4H, C*H*₂ (Fmoc), C*H* (Ser), C*H* (Thr)), 4.7 (t, J = 5 Hz, 1H, C*H*_(Fmoc)), 4.77 (m, 1H, C*H* (Asp)), 7.1-7.8 (m, 8H, ArH (Fmoc)).
[α] _{D}²⁰ = -5.5 © 0.2, CHCl₃)

### Block 10:

MS m/z (%): 1254.6 [M+Na]⁺ (100)
¹HNMR: 500 MHZ (CDCl₃) d1. 05 (d, J = 6 Hz, 3H, C*H*₃ (Thr)), 1.25 (s, 9H, C(CH₃)₃)' 1.29 (d, J = 3.5 Hz, 6H, C(C*H*₃)₂ (Pmc)), 1.35 (d, J = 7 Hz, 3H, C*H*₃ (Ala)), 1.8 (t, J = 7 Hz, 2H, C*H*₂ (Pmc)), 1.90-2.0 (m, 10H), 2.1 (s, 3H, C*H*₃ (Arg)), 2.5 (s, 3H, Ar-C*H*₃ (pmc)), 2.6 (s, 3H, Ar-C*H*₃ (Pmc)), 2.62 (t, J = 7 Hz, 2H, Ar-C*H*₂ (Pmc)), 3.05-3.30 (2m, 2H, C*H*₂ (Arg)), 3.55-3.75 (m, 5H, N-C*H*₂ (Pro)), 3.95 (m, 2H), 4.10-4.30 (m, 4H), 4.40 (m, 2H, C*H*₂ (Fmoc)), 4.53 (m, 2H, C*H*₂ (Arg)), 4.65 (m, 1H), 4.75 (m, 1H), 5.14 (d, J = 1.5 Hz, 2H, C*H*₂C₆H₅), 5.87 (d, J = 6 Hz, 1H, N*H*), 6.17 (bs, 2H, N*H*₂), 6.4 (bs, 1H, N*H*), 6.80 (d, J = 6 Hz, 1H, N*H*), 7.20-7.80 (m, 14H, Ar*H* (Fmoc, C₆H₅), 1NH), 8.20 (d, J = 6 Hz, 1H, N*H*).
[α] _{D}²⁰ = -14.5 © 0.1, CHCl₃)

### Block 11:

MS m/z (%) : 1758.7 [M+Na]⁺ (100), 1736 (7), 1398.7 (8), 891.6 (10).
¹HNMR: 500 MHZ (CDCl₃) d 1.08 (d, J = 6 Hz, 3H, C*H*₃ (Thr)), 1.32 (s, 9H, C(C*H*₃)₃), 1.34 (s, 9H, C(C*H*₃)₃), 1.42 (d, J = 7 Hz, 3H, C*H*₃ (Ala)), 1.85-2.20 (m, 25H, C*H*₂-C*H*₂ (4Pro), 7COC*H*₃ (STn)), 2.52 (dd, 1H, J = 12.5, 4.5 Hz, 1H, STn), 2.57 (dd, J = 17, 5 Hz, 1H, C*H*H (Asp)), 2.70 (dd, J = 17, 5 Hz, 1H, CH*H* (Asp)), 3.28 (dd, J = 9.5, 6.5 Hz, 1H, STn), 3.55 (m, 1H), 3.6-3.9 (m, 6H), 3.95- 4.40 (m, 8H), 4.55-4.65 (m, 3H), 4.70-4.85 (m, 3H), 5.02 (q, J = 45, 12 Hz, 2H), 5.1 (d, J = 4 Hz, 2H), 5.15-5.40 (m, 2H), 5.55 (d, J = 3 Hz, 1H, N*H*), 6.00 (d, J = 5.5 Hz, 1H, N*H*), 6.95 (d, J = 8 Hz, 1H, N*H*), 7.12 (dd, J = 15, 7 Hz, 1H, N*H*), 7.25-8.00(m, 19H, Ar*H* (Fmoc, C₆H₅), N*H*).
[α] _{D}²⁰= +4 . 0 © 0.1, CHCl₃)

### Block 12:

MS m/z (%) : 1593.7 [M+Na]⁺ (100), 1571.7 (80).
¹HNMR: 300 MHZ (CDCl₃) d 1.1-1.4 (m, 12H, C*H*₃ (Ala, Thr, Pmc)), 1.7-2.5 (m, 18H, C*H*₂ (Arg, Pro, Pmc), NHCOC*H*₃, ArC*H*₃), 2.50-2.70 (m, 8H, ArC*H*₃ (Pmc)), 2.90-3.30 (2m, 2H, C*H*₂ (Arg)), 3.50-3.70 (m, 4H), 3.70- 3.90 (m, 3H), 4.05-4.50 (m, 9H), 4.50-5.10 (m, 8H), 5.30 (m, 2H, C*H*₂C₆H₅), 5.50 (s, 1H, C*H*C₆H₅), 5.80- 6.00(bs, 2H, N*H*₂), 6.50 (bs, 1H, N*H*), 7.10-7.60 (m, 19H, Ar*H* (C₆H₅, Fmoc)), 7.7 (m, 2H, Ar*H* (Fmoc)), 8 . 10 (m, 3H, Ar*H*_(Fmoc), 1N*H*).
[α] _{D}²⁰ = +19.0 © 0 . 1, CHCl₃)

### Block 13:

MS m/z (%): 1524 [M+Na]⁺ (70), 1503.8 (100).
¹HNMR: 500 MHZ (CD₃OD) d 0.9 (d, J = 7 Hz, 6H, C(C*H*₃)₂ (Val)), 1.15 (d, J = 6 Hz, 3H, C*H*₃ (Thr)), 1.20 (s, 9H, C(C*H*₃)₃), 1.24 (s, 9H, C(C*H*₃)₃), 1.30 (m, 6H, C*H*₃ (2 Ala), 1.85-2.2 (m, 9H), 3.0 (m, 2H, C*H*₂ (His)), 3.50-4.0 (m, 8H, C*H*₂-C*H*₂ (Pro)), 4.10-4.80 (m, 13 H), 5.10 (m, 2H, C*H*₂C₆H₅), 6 . 8 (s, 1H, C*H* (His)), 7.0-8.0 (m, 29H, Ar*H* (Fmoc, C₆H₅), C*H* (His)).
[α] _{D}²⁰ = -19.75 © 0.2, CHCl₃)

### Block 14:

MS m/z (%) : 834 [M+Na]⁺ (100), 778.6 (7), 712.6 (7), 539.5 (5).
¹HNMR: 500 MHZ (CDCl₃) d 0. 85 (t, J = 6.5 Hz, 6H, C*H*₃ (2lip), 0.90 (2bs, 6H, C*H*₃ (Leu), 1.24 (s, 44H, C*H*₂ (lip)), 1.43 (s, 9H, C (C*H*₃) ₃), 1.50-1.55 (m, 4H, OCH₂C*H*₂- (lip), 1.60-1.65 (m, 3H, C*H*₂-C*H*(CH₃)₂ (Leu)), 3.40-3.50 (m, 5H, O-C*H*₂- (lip), C*H*H (Ser)), 3.55 (m, 1H, CH*H* (Ser)), 3.66 (s, 3H, COOC*H*₃), 3.750-3.78 (m, 1H, C*H*H (Ser), 3.85-3.88 (m, 1H, CH*H* (Ser)), 4.24 (bs, 1H, C*H* (Leu)), 4.45-4.48 (m, 1H, C*H* (Ser), 4.58-4.62 (m, 1H, C*H* (Ser)), 5.36 (bs, 1H, N*H*), 7. 04 (d, J = 8 Hz, 1H, N*H*), 7. 15 (d = 7.5 Hz, 1H, N*H*).
[α]_{D} = +8.75 © 0.2, CHCl₃)

### Block 5:

MS m/z (%): 1895 [M+Na]⁺ (100), 1873 (90), 931 (20).
¹HNMR: 600 MHZ (CDCl₃), d 1.03 (d, J = 6.5 Hz, 3H, C*H*₃ (Thr)), 1.07 (d, J = 6.5, 3H, C*H*₃ (Thr) ), 1.17 (s, 9H, C(C*H*₃)₃)' 1.24 (s, 9H, C (C*H*₃)₃), 1.26 (m, 3H, C*H*₂ (Pmc)), 1.29 (d, J = 5 Hz, 3H, C*H*₃ (Ala)), 1.32 (s, 9H, C(C*H*₃)₃), 1.36 (t, J = 6. 5 Hz, 6. 0 Hz, 3H), 1.39 (d, J = 5 Hz, 3H, C*H*₃ (Ala), 1.40 (s, 9H (C(C*H*₃)₃), 1.55 (bs, 1H), 1.62 (bs, 1H), 1.7) (bs, 1H), 1.8 (t, J = 6.5 Hz, 2H), 1.83-2.15 (m, 17H), 2.28 (m, 1H), 2.53-2.65 (m, 9H), 2.83 (dd, J = 11.5, 5.0 Hz, 1H, C*H*H (Asp)), 3.10-3.30 (m, 2H, C*H*₂ (Arg)), 3.42 (m, 1H), 3.50-3.70 (m, 6H, C*H*₂-C*H*₂ (Pro)), 3.88-4.05 (m, 3H), 4.10-4.18 (m, 2H), 4.20-4.30 (m, 3H), 4.35-4.42 (d, J = 7 Hz, 2H), 4.5 (m, 4H), 4.60-4.65 (p, J = 6.5 Hz, 1H), 4.7 (m, 2H), 4.78 (p, J = 7 Hz, 1H), 5.13 (d, J = 2 Hz, 2H, C*H*₂C₆H₅), 5.95 (d, J = 6.5 Hz, 1H, NH), 6.3 (bs, 2H, N*H*₂), 6.95 (bs, 1H, N*H*), 7.2-7.8 (m, 18H, Ar*H* (Fmoc), N*H*).
[α] _{D}²⁰ = +0. 5 © 0 .1, CHCl₃)

### Block 7a:

MS m/z (%): 2233.5 [M+Na]⁺ (100), 2211.5 (25), 1128.3 (35), 1105.5 (33).
¹HNMR: 500 MHZ (CDCl₃+CD₃OD), d 1.0-1.35 (m, 45H, C*H*₃ (Thr), C(C*H*₃)₃, C*H*₃ (Ala), C*H*₃ (Pmc)), 1.50-2.15 (m, 22H, C*H*₂-C*H*₂ (Pro), C*H*₂, ArC*H*₃ (Pmc), C*H*₂ (Arg), NHCOC*H*₃), 2. 35-2.85 (m, 10H), 3.1 (bs, 2H), 3.30-3.70 (m, 7H), 3.76-4.10 (m, 10H), 4.15-4.80 (m, 13H), 5. 0-5. 10 (m, 3H), 5.25 (m, 2H, (C*H*₂C₆H₅) ), 5.45 (s, 1H, CH (C₆H₅)), 7.18-8.0 (m, 24H, ArH (Fmoc, C₆H₅), NH).
[α] _{D}²⁰ = -15.0 © 0. 1, CHCl₃)

### Block 7b:

MS m/z (%): 3001 [M+Na]⁺ (25), 2979 (100).
¹HNMR: 500 MHZ (CDCl₃), d 1.08 (d, J = 3Hz, 3H, C*H*₃ (Thr)), 1.14 (d, J = 3Hz, 3H, C*H*₃ (Thr)), 1.20-1.48 (m, 30H, C*H*₃ (Ala), C(C*H*₃)₃, C(C*H*₃)₂ (Pmc)), 1.50-2.20 (m, 43H, C*H*₂ (Arg), ArC*H*₃, ArC*H*₂ (Pmc), C*H*₂-C*H*₂ (Pro), COC*H*₃), 2.40-2.80 (m, 11H), 3.10-3.40 (m, 3H), 3.40-3.65 (m, 4H), 3.65-3.80 (m, 5H), 3.80-4.18 (m, 13H), 4.18-4.60 (m, 15H), 4.60-4.90 (m, 6H), 4.90-5.0 (m, 2H), 5.08-5.24 (m, 5H), 5.24-5.42 (m, 3H), 5.45-5.6 (m, 2H), 5.95 (m, 1H), 6.9 (bs, 3H), 7.25-8.05 (m, 33H).
[α] _{D}²⁰= +4.5 © 0.1, CHCl₃)

### Block 8:

MS m/z (%): 2129 [M+Na]⁺ (35), 2107 (100).
¹HNMR: 500 MHZ (CDCl₃+CD₃OD), d 0. 88 (t, J = 7Hz, 6H, C*H*₃ (lip)), 0. 90-0. 98 (m, 12H, CH(C*H*₃)₂ (Val), CH(C*H*₃)₂ (Leu)), 1.10 (d, J = 6.5 Hz, 3H, C*H*₃ (Thr)), 1.20-1.40 (m, 68H, C*H*₃ (Ala), C*H*₂ (lip), C(C*H₃*)₃), 1.48-1.74 (m, 7H, OCH₂C*H*₂ (lip), C*H*-C*H*₂ (Leu)), 1.80-2.25 (m, 9H), 3.0 (d, J = 6 Hz, 2H, C*H*₂ (His)), 3.40-3.85 (m, 19H),7.25-7.40 (m, 12H), 7.60-7.85 (m, 6H).
[α] _{D}²⁰ = -16.0 © 0.1, CHCl₃)

### Block 3:

MS m/z (%): 3723.8 [M+H]⁺ (55), 3481.2 (100), 1862.1 (75), 1762.7 (25).
¹HNMR: 500 MHZ (CD₃OD), d 0.90 (d, J = 7 Hz, 6H, CH (C*H*₃)₂ = (Val)), 1.05-1.45 (m, 63H, C(C*H*₃)₃, C*H*₃ (Thr), C*H*₃ (Ala), C(C*H*₃)₂ (Pmc)), 1.45-2.20 (m, 35H), 2.50-2.90 (m, 10H), 2.40-2.90 (m, 10H), 3.0-3.20 (m, 4H, C*H*₂ (Arg), CH₂ (His)), 3.50-4.25 (m, 30H), 4.3-4.95 (m, 24H), 5.05-5.17 (m, 3H), 5.30-5.40 (m, 3H), 5.55 (s, 1H, C*H*C₆H₅), 5.63 (s, 1H, C*H*C₆H₅), 6.80 (s, 1H, C*H* (His), 7.13 (m, 6H, C*H* (His), ArH), 7.25-7.48 (m, 32H), 7.58-8.20 (m, 11H).
[α]_{D}²⁰ = +25 . 0 © 0.1, CHCl₃)

### Block 4a:

MS m/z (%): 2018 [M+2Na]⁺² (25), 2007 (75), 1995 (100), 1928 (25), 1885 (25).
¹HNMR: 500 MHZ (CD₃OD), d 0.9 (m, 18H), 1.05-1.30 (m, 107H), 1.4 (s, 9H), 1.50-1.70 (m, 10H), 1.75-2.30 (m, 28H), 2.50 (d, J = 4.5 Hz, 6H), 2.60-2.90 (m, 4H), 3.0-3.40 (m, 4H), 3.40-4.0 (m, 27H), 4.0-4.80 (m, 36H), 5.10 (m, 1H), 5.35 (dd, 1H, J = 11, 3Hz, 1H), 5.55 (s, 1H, C*H*C₆H₅), 6.80 (s, 1H, C*H* (His)), 7.11 (m, 6H), 7.20-8.0 (m, 28H).
[α]_{D}²⁰ = -6.5 © 0.1, CHCl₃)

### Block 4b:

MS m/z (%): 2387.5 [M+2Na]⁺² (70), 2377.5 (100).
¹HNMR: 500 MHZ (CD₃OD), d 0.8-0.9 (m, 18H), 1.10-1.15 (m, 9H, C*H*₃ (Thr)), 1.15-1.45 (m, 98H), 1.45-2.40 (m, 59H), 2.40-2.80 (m, 11H), 2.90-3.10 (m, 2H), 3.10-3.25 (m, 3H), 3.35-4.0 (m, 35H), 4.0-4.20 (m, 15H), 4.25-4.50 (m, 18H), 4.60-4.90 (m, 10H), 5.0-5.15 (m, 3H), 5.25-5.40 (m, 5H), 5.55-5.62 (m, 3H), 6.80 (s, 1H, C*H* (His)), 7.1-7.45 (m, 30H), 7.50-8.15 (m, 12H).
[α] _{D}²⁰ = +4.0 © 0.1, CHCl₃)

### Block 2a:

MS m/z (%): MALDI, 7403 [M+Na]⁺ (95), 7161.8 (100).
ES+, 3713 (30), 3592.2 (10), 2483.1
(100), 1868.3 (35).
¹HNMR: 600 MHZ (CD₃OD), d 0.85-0.95 (m, 24H), 1.05-1.34 (m, 161H), 1.40 (s, 9H), 1.41 (s, 9H), 1.48-1.70 (m, 12H), 1.75-2.0 (m, 49H), 2.06 (s, 3H), 2.08-2.20 (m, 10H), 2.50-2.56 (m, 13H), 2.59-2.64 (m, 3H), 2.66-2.75 (m, 2H), 2.76-2.90 (m, 2H), 2.94-3.06 (m, 4H), 3.08-3.25 (m, 4H), 3.32-4.0 (m, 52H), 4.0-4.24 (m, 20H), 4.24-4.90 (m, 42H), 5.10 (dd, J = 19, 5 Hz, 2H), 5.28-5.39 (m, 4H), 5.53 (s, 1H, C*H*C₆H₅), 5.57 (s, 1H, C*H*C₆H₅), 5.59 (s, 1H, C*H*C₆H₅), 6.72 (s, 1H), 6.75 (s, 1H), 6.95-7.15 (m, 13H), 7.25-7.30 (m, 12H), 7.33-7.45 (m, 40H), 7.55-8.01 (m, 14H).
[α]_{D}²⁰ = -0. 5 © 0. 1, CHCl₃)

### Block 2b:

MS m/z (%): MALDI, 8147.6 [M]⁺ (100).
ES+, 4096 (65), 2738.7 (100), 2059.6 (17). ¹HNMR: 600 MHZ (CD₃OD), d 0.85-1.0 (m, 24H), 1.05-1.45 (m, 161H), 1.45-2.40 (m, 94H), 2.45-2.90 (m, 21H), 2.90-3.30 (m, 9H), 3.40-4.30 (m, 80H), 4.30-5.0 (m, 52H), 5.02-5.15 (m, 4H), 5.25-5.40 (m, 8H), 5.50-5.65 (m, 4H), 6.75 (s, 2H), 7.10-7.20 (m, 13H), 7.20-7.65 (m, 54H), 7.70-8.10 (m, 8H).
[α] _{D}²⁰ = 0.0 © 0.1, CHCl₃)

### Compound 1a:

MS m/z (%): MALDI, 5046.89 [M+H]⁺ (100), 4824.66 (25).
ES+, 2524.2 [M+2H]⁺² (30), 1682.9 (90), 1262.6 (100).
[α] _{D}²⁰= -100 . 0 © 0 .1, H₂O)

### Compound 1b:

MS m/z (%): MALDI, 5541.34 [M+H]⁺ (100), 5249.55 (50), 5028.21 (55).
ES+, 2771 [M+2H]⁺², (25), 1848 (55), 1751 (40), 1683 (20), 1386 (100), 1313 (70), 1263 (85), 1212 (55).
[α]_{D}²⁰ = -29.5.0 © 0.1, H₂O).

### Immunological investigations:

Immune responses to the two examples of synthetic glyco-lipo-peptides (GLP) **1a** and **1b** have been thoroughly investigated. The results indicate that the structural features incorporated into these molecules are reflected in the broad spectrum of immune responses, both humoral and cell mediated. Synthetic lipid-A has been used as an adjuvant for both formulations of GLP in liposomes.

A summary of immunological data from mice after immunization with the vaccine formulations for the two glycolipopeptides la and 1b, designated as **1a** and **1b** respectively, is presented in figure 16. Groups of C57B1/6 mice immunized with either one of the glycolipopeptide formulation in liposomes with synthetic lipid-A (figure 16), produced very potent and T cell specific proliferative response and high IFN-g levels (Figures 17 and 18). The sera taken from the same mice after two immunizations were screened against various solid phases for specific IgG and IgM antibody titres. **1a** and **1b** glycolipopeptides were used as sources of synthetic solid phases and neuraminidase treated ovine sub-maxillary mucin (OSM), and human MUC1 mucin purified from ascites obtained from cancer patients, were used as sources of natural solid phases. As presented in table 3, both **1a** and **1b** elicited strong IgG antibody responses against synthetic solid phases with titres of 1/218, 700. On Muc1 mucin solid phase the titres are the range of 1/300 to 1/900.

The IgM responses are presented in table 4. Both the glyco-lipo-peptides elicited IgM titres against synthetic solid phase with median titres of 1/2700. Similar titres are observed with neuraminidase treated OSM as a solid phase, suggesting carbohydrate specific antibodies to Tn carbohydrate antigen. Some IgM titres are found to bind to the human MUC1 mucin solid phase. It has also been demonstrated that the immune serum antibodies bind to human MUC1 transfected tumor cells.

The presented data clearly demonstrated that synthetic glycolipopeptides, when formulated into liposomal delivery system, are very potent inducers of both cellular (T1) and humoral (T2) immune responses against both synthetic and natural sources of MUC1 antigen. These high responses may be the result of the normal C57B1/6 mice recognizing these synthetic glycolipopeptides, which are mimics of human MUC1 mucin, as foreign antigens. To see if these antigens are tolerated in mice transfected with human MUC1 gene, the transgenic mice expressing MUC1 mucin are immunised. As shown in Figure 18, human MUC1 transgenic mice maintain a potent T cell specific proliferative response and IFN-g production that is comparable to the immune responses observed in normal C57B1/6 mice. Immunisations of mice transfected with human MUC1 reproduced similar high titres of IgG and IgM antibodies (Tables 5 and 6) as observed in the normal C57B1/6. Potent immune responses, both T1 and T2 kind, are maintained through 10 bi-weekly immunizations. These observations indicate that synthetic glycopeptide liposomal vaccines are able to overcome the tolerance to self-antigens in mouse models. This study demonstrates the utility of synthetic self antigens, in the form of glycolipopeptides, as vaccines against cancers.

### Brief Description of Tables

Note: table 1 deleted.
Table 2: List of abbreviations used for the reagents, protecting groups and intermediates.
Table 3: IgG antibody titres in C57B1/6 mice after two immunizations.
Table 4: IgM antibody titres in C57B1/6 mice after two immunizations.
Table 5: IgG antibody titres in C57B1/6 mice after 10 immunizations.
Table 6: IgM antibody titres in C57B1/6 mice after 10 immunizations.

**Table 2**

| Abbreviations of reaqents and terms used in the text | |
|---|---|
| Boc | : t-Butyloxycarbonyl |
| DCC | : 1,3-Dicyclohexyl carbodiimide |
| DCM | : Dichloromethane |
| DIEA | : Diisopropylethyl amine |
| DMF | : Dimethylformamide |
| Fmoc | : 9-Fluorenylmethyloxycarbonyl |
| Gal | : Galactose |
| GalNAc | : N-Acetyl galactosamine |
| HOBt | : 1-Hydroxybenzotriazole hydrate |
| HPTLC | : High performance thin layer chromatography |
| Lip | : Lipid chain (-C₁₄H₂₉) |
| MeOH | : Methanol |
| MUC1 | : Mucin 1 |
| NaOMe | : Sodium methoxide |
| Neu5Ac | : N-Acetyl neuraminic acid |
| NHS | : N-Hydroxy succinimide |
| Pd/C | : Palladium over charcoal |
| Pmc | : 2,2,5,7,8-pentamethylchroman-6-sulphonyl |
| Rf | : Retention factor |
| RP-HPLC | : Reverse phase-High performance liquid |
| chromatogr aphy | |
| RT | : Room temperature |
| tBu | : tertiary Butyl |
| TFA | : Trifluoro acetic acid |
| THF | : Tetrahydrofuran |
| TLC | : Thin layer chromatography |
| Tn | : alphaN-Acetyl galactosamine |
| Trt | : Trityl |

**Table 3. IgG antibody response in C57Bl/6 mice after two immunizations with synthetic glycolipopeptide liposomal vaccines***

| | | | **IgG** |
|---|---|---|---|
| | **Antibody Titer Summary** | | |
| Injected Material | **1a** solid phase | **1b** solid phase | Human MUC1 mucin solid phase |
| | 1/218,700 | 1/218,700 | 1/300 |
| **1a** | | | |
| | 1/218,700 | 1/218,700 | 1/900 |
| **1b** | | | |
| Saline | <1/100 | <1/100 | <1/100 |

**Table 4. IgM antibody response in C57Bl/6 mice after two immunizations with synthetic glycolipopeptide liposomal vaccines***

| | | | **IgM** |
|---|---|---|---|
| | **Antibody Titer Summary** | | |
| Injected Material | **1b** solid phase | Neuraminidase treated OSM | Human MUC1 mucin solid phase |
| **1a** | 1/2700 | 1/2700 | 1/300 |
| **1b** | 1/2700 | 1/2700 | 1/900 |
| Saline | <1/100 | <1/100 | <1/100 |

| | | | |
|---|---|---|---|
| *Sera from immunized mice were screened for IgG and IgM antibodies using both synthetic and natural solid phases. The synthetic glycolipopeptides **1a** and **1b** were used as synthetic solid phase. The natural solid phases were: human MUC1 mucin purified from the ascites of ovarian cancer patient, and OSM (Ovine Submaxilliary Mucin) that is a natural source of STn carbohydrate antigen. Treatment of OSM with neuraminidaze leads to the exposure of Tn carbohydrate antigens. | | | |

**Table 5. IgG antibody response in C57Bl/6 human MUC1 transgenic mice after ten immunizations with synthetic glycolipopeptide liposomal vaccines***

| | | | **IgG** |
|---|---|---|---|
| | **Antibody Titer Summary** | | |
| Injected Material | **1a** solid phase | **1b** solid phase | Human MUC1 mucin solid phase |
| | 1/72, 900 | 1/72, 900 | <1/100 |
| **1a** | 1/72,900 | 1/24,300 | <1/100 |
| **1b** Saline | <1/100 | <1/100 | <1/100 |

**Table 6. IgM antibody response in C57Bl/6 human MUC1 transgenic mice after ten immunizations with synthetic glycolipopeptide liposomal vaccines***

| | | | **IgM** |
|---|---|---|---|
| | **Antibody Titer Summary** | | |
| Injected Material | **1b** solid phase | Neuraminidase treated OSM | Human MUC1 mucin solid phase |
| **1a** | 1/24,300 | 1/300 | 1/2700 |
| **1b** | 1/24,300 | 1/100 | 1/300 |
| Saline | <1/100 | <1/100 | <1/100 |

| | | | |
|---|---|---|---|
| *Sera from immunized mice were screened for IgG and IgM antibodies using both synthetic and natural solid phases. The synthetic glycolipopeptides **1a** and **1b** were used as synthetic solid phase. The natural solid phases were: human MUC1 mucin purified from the ascites of ovarian cancer patient, and OSM (Ovine Submaxilliary Mucin) that is a natural source of STn carbohydrate antigen. Treatment of OSM with neuraminidaze leads to the exposure of Tn carbohydrate antigens. | | | |

*Citation of documents herein is not intended as an admission that any of the documents cited herein is pertinent prior art, or an admission that the cited documents is considered material to the patentability of any of the claims of the present application. All statements as to the date or representation as to the contents of these documents is based on the information available to the applicant and does not constitute any admission as to the correctness of the dates or contents of these documents.*

*The appended claims are to be treated as a non-limiting recitation of preferred embodiments.*

*In addition to those set forth elsewhere, the following references in their most recent editions as of the time of filing of this application* may be of relevance for the understanding of the invention : *Kay, Phage Display of Peptides and Proteins: A Laboratory Manual; the John Wiley and Sons Current Protocols series, including Ausubel, Current Protocols in Molecular Biology; Coligan, Current Protocols in Protein Science; Coligan, Current Protocols in Immunology; Current Protocols in Human Genetics; Current Protocols in Cytometry; Current Protocols in Pharmacology; Current Protocols in Neuroscience; Current Protocols in Cell Biology; Current Protocols in Toxicology; Current Protocols in Field Analytical Chemistry; Current Protocols in Nucleic Acid Chemistry; and Current Protocols in Human Genetics; and the following Cold Spring Harbor Laboratory publications: Sambrook, Molecular Cloning: A Laboratory Manual; Harlow, Antibodies: A Laboratory Manual; Manipulating the Mouse Embryo: A Laboratory Manual; Methods in Yeast Genetics: A Cold Spring Harbor Laboratory Course Manual; Drosophila Protocols; Imaging Neurons: A Laboratory Manual; Early Development of Xenopus laevis: A Laboratory Manual; Using Antibodies: A Laboratory Manual; At the Bench: A Laboratory Navigator; Cells: A Laboratory Manual; Methods in Yeast Genetics: A Laboratory Course Manual; Discovering Neurons: The Experimental Basis of Neuroscience; Genome Analysis: A Laboratory Manual Series ; Laboratory DNA Science; Strategies for Protein Purification and Characterization: A Laboratory Course Manual; Genetic Analysis of Pathogenic Bacteria: A Laboratory Manual; I PCR Primer: A Laboratory Manual; Methods in Plant Molecular Biology: A Laboratory Course Manual ; Manipulating the Mouse Embryo: A Laboratory Manual; Molecular Probes of the Nervous System; Experiments with Fission Yeast: A Laboratory Course Manual; A Short Course in Bacterial Genetics: A Laboratory Manual and Handbook for Escherichia coli and Related Bacteria; DNA Science: A First Course in Recombinant DNA Technology; Methods in Yeast Genetics: A Laboratory Course Manual; Molecular Biology of Plants: A Laboratory Course Manual.*

*Reference to known method steps, conventional methods steps, known methods or conventional methods is not in any way an admission that any aspect, description or embodiment of the present invention is disclosed, taught or suggested in the relevant art.*

*The foregoing description of the specific embodiments will so fully reveal the general nature of the invention that others can, by applying knowledge within the skill of the art (including the contents of the references cited herein), readily modify and*/*or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.*

*Any description of a class or range as being useful or preferred in the pra ctice of the invention shall be deemed a description of any subclass (e.g., a disclosed class with one or more disclosed members omitted) or subrange contained therein, as well as a separate description of each individual member or value in said class or range.*

*The description of preferred embodiments individually shall be deemed a description of any possible combination of such preferred embodiments, except for combinations which are impossible (e.g, mutually exclusive choices for an element of the invention) or which are expressly excluded by this specification.*

*If an embodiment of this invention is disclosed in the prior art, the description of the invention shall be deemed to include the invention as herein disclosed with such embodiment excised.*

*The invention, as contemplated by applicant (s), includes but is not limited to the subject matter set forth in the appended claims, and presently unclaimed combinations thereof. It further includes such subject matter further limited, if not already such, to that which overcomes one or more of the disclosed deficiencies in the prior art. To the extent that any claims encroach on subject matter disclosed or suggested by the prior art, applicant(s) contemplate the invention(s) corresponding to such claims with the encroaching subject matter excised.*

## Claims

1. A non-naturally occurring glycolipopeptide, said glycolipopeptide comprising at least one amino acid being a glycosylated amino acid and at least one amino acid being a lipidated amino acid, wherein at least one lipidated amino acid is an interior amino acid, wherein said glycolipopeptide comprises at least two MUC1 epitopes with the amino acid sequence P(D/E)(T/S)RP, and wherein
i) at least one MUC1 epitope with the amino acid sequence P(D/E)(T/S)RP comprises at least one glycosylated amino acid, and
ii) at least one MUC1 epitope with the amino acid sequence P(D/E)(T/S)RP comprises no glycosylated amino acid, and
wherein the amino acid sequence of said MUC1 epitopes may be the same or different.

2. The glycopeptide of claim 1 which comprises the amino acid sequence PDTRP (AAs 6-10 of SEQ ID NO:10).

3. The glycolipopeptide of claim 1 or 2, wherein each of the epitopes (i) and (ii) comprises the amino acid sequence PDTRP.

4. The glycolipopeptide of any one of claim 1 - 3 which comprises at least one T cell epitope.

5. The glycolipopeptide of any one of claim 1-4 which comprises the amino acid sequence SAPDTRP (AAs 4-10 of SEQ ID NO:10).

6. The glycolipopeptide of any of the previous claims which comprises the amino acid sequence PDTRPAPGS.

7. The glycolipopeptide of any of the previous claims which comprises the amino acid sequence RPAPGS.

8. The glycolipopeptide of any of the previous claims which comprises the amino acid sequence PPAHGVT.

9. The glycolipopeptide of any of the previous claims which comprises the amino acid sequence STAPPAHGV.

10. The glycolipopeptide of any one of claims 1-9 which comprises at least one copy of
(a) the MUC1 consensus tandem repeat GVTSAPDTRPAPGSTAPPAH (SEQ ID NO:10),
(b) a cyclic permutation thereof, or
(c) a sequence substantially identical to (a) or (b) above.

11. The glycolipopeptide of claim 10 which comprises at least two copies of (a), (b) or (c).

12. The glycolipopeptide of claim 10 which comprises at least one copy of (a) or (b).

13. The glycolipopeptide of claim 10 which comprises at least two copies of (a) or (b).

14. The glycolipopeptide of claim 10 which comprises only two copies of (a) or (b).

15. The glycolipopeptide of any of the previous claims with the amino acid sequence:
TSAPDTRPAPGSTAPPAHGVTSAPDTRPAPGSTAPPAHGVSSL

16. The glycolipopeptide of any one of claims 1-15 where at least one glycosylated amino acid is O-glycosylated.

17. The glycolipopeptide of any one of claims 1-15 where at least one glycosylated amino acid is N-glycosylated.

18. The glycolipopeptide of any one of claims 1-15 where at least one glycosylated amino acid is S-glycosylated.

19. The glycolipopeptide of any one of claims 1-18 which comprises a tumor-associated carbohydrate epitope.

20. The glycolipopeptide of claim 19 where the carbohydrate epitope is GalNAc (Tn).

21. The glycolipopeptide of claim 19 where the carbohydrate epitope is sialyl Tn.

22. The glycolipopeptide of claim 19 where the carbohydrate epitope is Gal-GaINAc (TF).

23. The glycolipopeptide of claim 2 where the threonine of PDTRP is glycosylated.

24. The glycolipopeptide of claim 23 where the threonine of PDTRP is O-linked to Tn.

25. The glycolipopeptide of any one of claims 1-24 where at least two amino acids are glycosylated.

26. The glycopeptide of any one of claims 1-125 in which at least two amino acids are lipidated.

27. The glycolipopeptide of any one of claims 1-26 in which at least two interior amino acids are lipidated.

28. The glycolipopeptide of any one of claims 1-27 in which all of the lipidated amino acids are interior amino acids.

29. The glycolipopeptide of any one of claims 1-28 **characterized by** a carboxy terminal sequence SSL, where each of the serines is lipidated.

30. The glycolipopeptide of any one of claims 1 to 29 in which there are not more than 200 amino acids.

31. The glycolipopeptide of any one of claims 1 to 30 in which there are not more than 75 amino acids.

32. The glycolipopeptide of any one of claims 1 to 31 in which there are not more than 50 amino acids.

33. The glycolipopeptide of any one of claims 1 to 32 comprising two non-overlapping effective tandem repeats (for a total of 40 amino acids).

34. The glycolipopeptide of any one of claims 1-33 wherein at least one lipidated amino acid comprises a strongly lipophilic group comprising at least 13 atoms other than hydrogen.

35. The glycolipopeptide of any one of claims 1-34 wherein at least one lipidated amino acid comprises a strongly lipophilic group comprising at least 21 atoms other than hydrogen.

36. The glycolipopeptide of any one of claims 1-35 where said group consists of not more than 100 atoms other than hydrogen.

37. The glycolipopeptide of any one of claims 1-35 where said group consists of not more than 40 atoms other than hydrogen.

38. The glycopeptide of any one of claims 1-37 in which at least one strongly lipophilic group of at least one lipidated amino acid has a log^{p}, as predicted by the Meylan algorithm of at least 2.7.

39. The glycolipopeptide of any one of claims 1-38 in which the amino terminal amino acid comprises a strongly lipophilic group.

40. The glycolipopeptide of any one of claims 1-39 in which the carboxy terminal amino acid comprises a strongly lipophilic group.

41. The glycolipopeptide of any one of claims 1-40 in which at least one lipidated amino acid is selected from the group consisting of lipidated Ser, Thr, Asp, Glu, Cys, Tyr, Lys, Arg, Asn or Gln.

42. The glycolipopeptide of claim 41 where the lipidated amino acid is lipidated Ser or Thr.

43. The glycolipopeptide of any one of claims 1-42 where at least one lipidated amino acid comprises a side chain of the formula
-A-Y-Z i.
i) where A is optional but, if present, is an organic group of not more than 12 atoms other than hydrogen;
ii) Y is a spacer of not more than 12 atoms other than hydrogen, and comprising nitrogen, oxygen, sulfur or phosphorous, and Z is a strongly lipophilic group.

44. The glycolipopeptide of claim 43 in which A, if present, is an alkyl of 1-4 carbon atoms.

45. The glycolipopeptide of claim 44 in which A is present and is -CH₂- or -CH(CH₃)-.

46. The glycolipopeptide of any one of claims 43-45 in which Y comprises a group selected from the group consisting of -O-, -S-, -NH-, -NR-, -PO₄-, -C(=O)-, -C(=S)-, -C(= NH)-, and - C(=NR)-, where R is 1-4 alkyl.

47. The glycolipopeptide of claim 43 in which Y is -NHCO-, -OCO- or -SCO-.

48. The glycolipopeptide of claim 43 in which Y is -CONH- or -CH₂NH-.

49. The glycolipopeptide of claim 43 in which Y is -O-, -S- or -NH-.

50. The glycolipopeptide of any one of claims 43-49 in which -Y-Z is itself a strongly lipophilic group.

51. The glycolipopeptide of claim 50 in which A is present and -A-Y-Z is itself a strongly lipophilic group.

52. The glycolipopeptide of any one of claims 43-51 in which Z is at least partially aromatic.

53. The glycolipopeptide of any one of claims 43-52 in which Z is aliphatic.

54. The glycolipopeptide of any one of claims 43-53 in which Z comprises at least one moiety of the form -A'-Y'-Z', where A', Y' and Z' are defined analogously to A, Y and Z, respectively.

55. The glycolipopeptide of claim 54 where Y' is -O- and Z' is an alkyl group.

56. The glycolipopeptide of claim 55 where A is -(CH2)i-, where i is 0 or 1, or Z' is - (CH₂)jCH₃, where j is 6 to 26.

57. The glycolipopeptide of any one of claims 54-56 in which Z comprises -B(-Y'-Z')ₙ, in which B is a branched organic group of not more than 12 atoms than hydrogen, each Y' is an independently chosen spacer of not more than 12 atoms other than hydrogen, and comprising nitrogen, oxygen, sulfur or phosphorous, and each Z' is an independently chosen strongly lipophilic group, and n is at least two.

58. The glycolipopeptide of claim 57 in which n is 2 or 3.

59. The glycolipopeptide of claims 57 or 58 in which each Y' is -O- and each Z' independently is -(CH₂)ⱼCH₃, where j = 6 to 26.

60. The glycolipopeptide of claim 59 in which n=2 and B is -CH(CH₂-)₂.

61. The glycolipopeptide of claim 60 in which n=2 and B is -C(CH₂-)₃.

62. The glycolipopeptide of any of claims 34-61 where the strongly lipophilic group of at least one lipidated amino acid comprises at least one of the following structures: 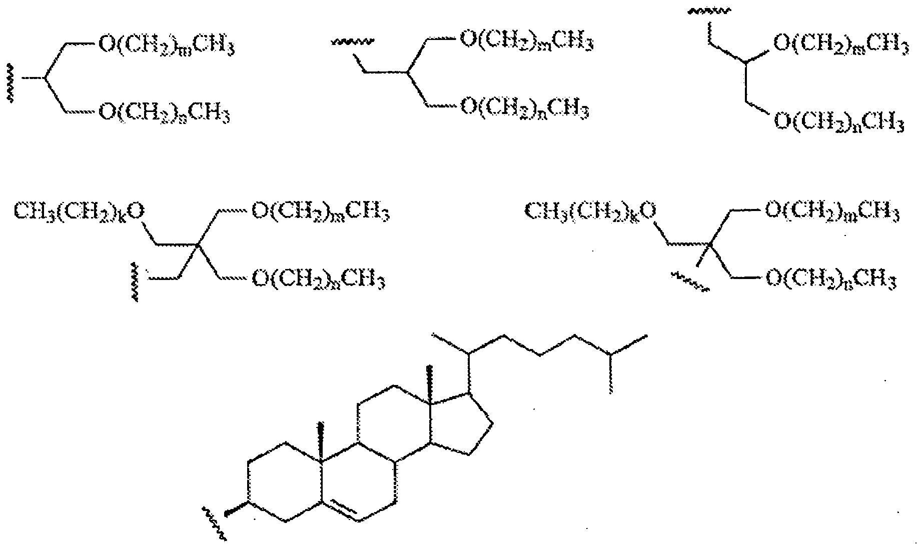 where m, n, and k are independent integers with values ranging from 3 to 30.

63. The glycolipopeptide of any of the claims 1-62, where at least one lipidated amino acid comprises the structure 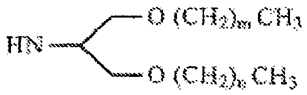 where m and n are independent integers with values ranging from 6 to 26.

64. The glycolipopeptide of any one of claims 34-63, where the strongly lipophilic group at least one lipidated amino acid comprises the structure 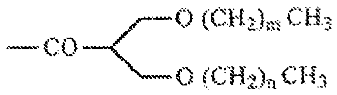 where m and n are independently chosen integers 6 to 26.

65. The glycolipopeptide of any one of the claims 1-64, having the following structure: 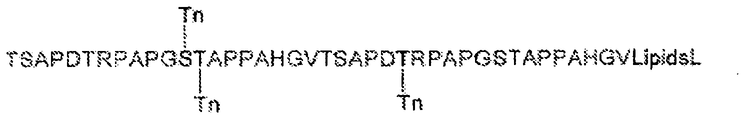 where Tn is a N-acetyl galactosamine, and "Lipids" refers to two or more consecutive lipidated amino acids.

66. The glycolipopeptide of any one of the claims 1-64, having the following structure: 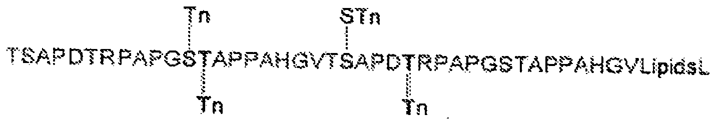 where Tn is aN-acetyl galactosamine and STn is a(2-6) sialyl, aN-acetyl galactosamine, and "Lipids" refers to two or more consecutive lipidated amino acids.

67. The glycolipopeptide of any one of claim 65 or 66, wherein the two or more consecutive lipidated amino acids are lipidated serines.

68. The glycolipopeptide of any one of the claims 1-65 and 67, having the following structure:

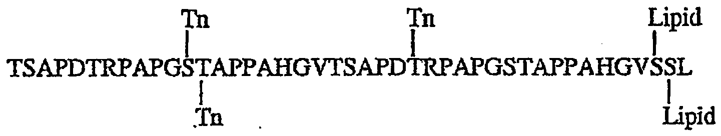

69. The glycolipopeptide of any one of the claims 1-64, 66 and 67, having the following structure:

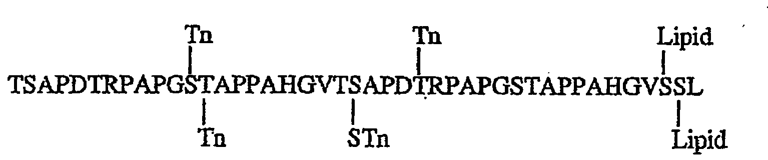

70. The glycolipopeptide of any of the previous claims, wherein at least one glycosylated amino acid comprised by said peptide epitope itself comprises a cancer associated carbohydrate epitope.

71. The glycolipopeptide of any of the previous claims for use as a medicament.

72. Use of the glycolipopeptide of any one of the claims 1-70, for the manufacture of a medicament for eliciting an immune response upon administration to a subject.

73. The glycolipopeptide of any of the previous claims 1-70 for use in a method for eliciting an immune response upon administration to a subject.

74. The use according to claim 70, wherein said subject suffers from the disease with which said cancer-associated pitope is associated.

75. The glycolipopeptide of claim 72, wherein said subject suffers from the disease with which said cancer-associated epitope is associated.

76. A composition comprising a glycolipopeptide according to any one of claims 1-70, and a liposomes.

77. The composition of claim 76, which comprises phosphatidyl choline.

78. The composition of claim 76, which comprises cholesterol.

79. The composition of claim 76, which comprises phosphatidyl glycerol.

80. The composition according to any of claims 76-79 for use as a medicament.

81. Use of the composition according to any one of the claims 76-80 for the manufacture of a medicament for eliciting an immune response upon administration to a subject.

82. The composition according to any one of the claims 76-80 for use in a method for eliciting and immune response in a subject.

## Patentansprüche

1. Ein nicht in der Natur vorkommendes Glycolipopeptid, wobei das Glycolipopeptid wenigstens eine Aminosäure, die eine glycosylierte Aminosäure ist, und wenigstens eine Aminosäure, die ein lipidierte Aminosäure ist, umfasst, wobei wenigstens eine lipidierte Aminosäure eine innere Aminosäure ist, wobei das Glycolipopeptid wenigstens zwei MUC1-Epitope mit der Aminosäuresequenz P(D/E)(T/S)RP umfasst und wobei
i) wenigstens ein MUC1-Epitop mit der Aminosäuresequenz P(D/E)(T/S)RP wenigstens eine glycosylierte Aminosäure umfasst und
ii) wenigstens ein MUC1-Epitop mit der Aminosäuresequenz P(D/E)(T/S)RP keine glycosylierte Aminosäure umfasst und
wobei die Aminosäuresequenz der MUC1-Epitope gleich oder verschieden sein kann.

2. Das Glycopeptid nach Anspruch 1, welches die Aminosäuresequenz PDTRP (AAs 6 - 10 von SEQ ID NR:10) umfasst.

3. Das Glycolipopeptid nach Anspruch 1 oder 2, wobei jedes der Epitope (i) und (ii) die Aminosäuresequenz PDTRP umfasst.

4. Das Glycolipopeptid nach irgendeinem der Ansprüche 1 - 3, welches wenigstens ein T-Zell-Epitop umfasst.

5. Das Glycolipopeptid nach irgendeinem der Ansprüche 1 - 4, welches die Aminosäuresequenz SAPDTRP (AAs 4 - 10 von SEQ ID NR:10) umfasst.

6. Das Glycolipopeptid nach irgendeinem der vorhergehenden Ansprüche, welches die Aminosäuresequenz PDTRPAPGS umfasst.

7. Das Glycolipopeptid nach irgendeinem der vorhergehenden Ansprüche, welches die Aminosäuresequenz RPAPGS umfasst.

8. Das Glycolipopeptid nach irgendeinem der vorhergehenden Ansprüche, welches die Aminosäuresequenz PPAHGVT umfasst.

9. Das Glycolipopeptid nach irgendeinem der vorhergehenden Ansprüche, welches die Aminosäuresequenz STAPPAHGV umfasst.

10. Das Glycolipopeptid nach irgendeinem der Ansprüche 1 - 9, welches wenigstens eine Kopie der folgenden umfasst:
(a) des MUC1 Consensus Tandem Repeats GVTSAPDTRPAPGSTAPPAH (SEQ ID NR:10),
(b) einer zyklischen Permutation von diesem oder
(c) einer Sequenz, welche im Wesentlichen zu (a) oder (b) oben identisch ist.

11. Das Glycolipopeptid nach Anspruch 10, welches wenigstens zwei Kopien von (a), (b) oder (c) umfasst.

12. Das Glycolipopeptid nach Anspruch 10, welches wenigstens eine Kopie von (a) oder (b) umfasst.

13. Das Glycolipopeptid nach Anspruch 10, welches wenigstens zwei Kopien von (a) oder (b) umfasst.

14. Das Glycolipopeptid nach Anspruch 10, welches nur zwei Kopien von (a) oder (b) umfasst.

15. Das Glycolipopeptid nach irgendeinem der vorhergehenden Ansprüche mit der Am inosäuresequenz:
TSAPDTRPAPGSTAPPAHGVTSAPDTRPAPGSTAPPAHGVSSL.

16. Das Glycolipopeptid nach irgendeinem der Ansprüche 1-15, wobei wenigstens eine glycosylierte Aminosäure O-glycosyliert ist.

17. Das Glycolipopeptid nach irgendeinem der Ansprüche 1-15, wobei wenigstens eine glycosylierte Aminosäure N-glycosyliert ist.

18. Das Glycolipopeptid nach irgendeinem der Ansprüche 1-15, wobei wenigstens eine glycosylierte Aminosäure S-glycosyliert ist.

19. Das Glycolipopeptid nach irgendeinem der Ansprüche 1-18, welches ein Tumor-assoziiertes Kohlenhydrat-Epitop umfasst.

20. Das Glycolipopeptid nach Anspruch 19, wobei das Kohlenhydrat-Epitop GalNAc (Tn) ist.

21. Das Glycolipopeptid nach Anspruch 19, wobei das Kohlenhydrat-Epitop Sialyl Tn ist.

22. Das Glycolipopeptid nach Anspruch 19, wobei das Kohlenhydrat-Epitop Gal-GalNAc (TF) ist.

23. Das Glycolipopeptid nach Anspruch 2, wobei das Threonin von PDTRP glycosyliert ist.

24. Das Glycolipopeptid nach Anspruch 23, wobei das Threonin von PDTRP mit Tn O-verknüpft ist.

25. Das Glycolipopeptid nach irgendeinem der Ansprüche 1 - 24, wobei wenigstens zwei Aminosäuren glycosyliert sind.

26. Das Glycopeptid nach irgendeinem der Ansprüche 1 - 25, bei welchem wenigstens zwei Aminosäuren lipidiert sind.

27. Das Glycolipopeptid nach irgendeinem der Ansprüche 1 - 26, bei welchem wenigstens zwei innere Aminosäuren lipidiert sind.

28. Das Glycolipopeptid nach irgendeinem der Ansprüche 1 - 27, bei welchem alle der lipidierten Aminosäuren innere Aminosäuren sind.

29. Das Glycolipopeptid nach irgendeinem der Ansprüche 1 - 28, welches durch eine carboxyterminale Sequenz SSL gekennzeichnet ist, wobei jedes der Serine lipidiert ist.

30. Das Glycolipopeptid nach irgendeinem der Ansprüche 1 bis 29, bei welchem es nicht mehr als 200 Aminosäuren gibt.

31. Das Glycolipopeptid nach irgendeinem der Ansprüche 1 bis 30, bei welchem es nicht mehr als 75 Aminosäuren gibt.

32. Das Glycolipopeptid nach irgendeinem der Ansprüche 1 bis 31, bei welchem es nicht mehr als 50 Aminosäuren gibt.

33. Das Glycolipopeptid nach irgendeinem der Ansprüche 1 bis 32, welches zwei nicht überlappende wirksame Tandem Repeats (für eine Gesamtzahl von 40 Aminosäuren) umfasst.

34. Das Glycolipopeptid nach irgendeinem der Ansprüche 1 - 33, wobei wenigstens eine lipidierte Aminosäure eine stark lipophile Gruppe umfasst, welche wenigstens 13 Atome, die von Wasserstoff verschieden sind, umfasst.

35. Das Glycolipopeptid nach irgendeinem der Ansprüche 1 - 34, wobei wenigstens eine lipidierte Aminosäure eine stark lipophile Gruppe umfasst, welche wenigstens 21 Atome, die von Wasserstoff verschieden sind, umfasst.

36. Das Glycolipopeptid nach irgendeinem der Ansprüche 1 - 35, wobei die Gruppe aus nicht mehr als 100 Atomen, die von Wasserstoff verschieden sind, besteht.

37. Das Glycolipopeptid nach irgendeinem der Ansprüche 1 - 35, wobei die Gruppe aus nicht mehr als 40 Atomen, die von Wasserstoff verschieden sind, besteht.

38. Das Glycolipopeptid nach irgendeinem der Ansprüche 1 - 37, bei welchem wenigstens eine stark lipophile Gruppe von wenigstens einer lipidierten Aminosäure einen logP, wie dieser durch den Meylan-Algorithmus vorausgesagt wird, von wenigstens 2,7 aufweist.

39. Das Glycolipopeptid nach irgendeinem der Ansprüche 1 - 38, bei welchem die aminoterminale Aminosäure eine stark lipophile Gruppe umfasst.

40. Das Glycolipopeptid nach irgendeinem der Ansprüche 1 - 39, bei welchem die carboxyterminale Aminosäure eine stark lipophile Gruppe umfasst.

41. Das Glycolipopeptid nach irgendeinem der Ansprüche 1 - 40, bei welchem wenigstens eine lipidierte Aminosäure ausgewählt ist aus der Gruppe, bestehend aus lipidiertem Ser, Thr, Asp, Glu, Cys, Tyr, Lys, Arg, Asn oder Gln.

42. Das Glycolipopeptid nach Anspruch 41, wobei die lipidierte Aminosäure lipidiertes Ser oder Thr ist.

43. Das Glycolipopeptid nach irgendeinem der Ansprüche 1 - 42, wobei wenigstens eine lipidierte Aminosäure eine Seitenkette mit der Formel
-A-Y-Z umfasst, i.
i) wobei A fakultativ ist, aber, wenn es vorliegt, eine organische Gruppe von nicht mehr als 12 Atomen, die von Wasserstoff verschieden sind, ist;
ii) Y ein Abstandhalter von nicht mehr als 12 Atomen, die von Wasserstoff verschieden sind, ist und Stickstoff, Sauerstoff, Schwefel oder Phosphor umfasst und Z eine stark lipophile Gruppe ist.

44. Das Glycolipopeptid nach Anspruch 43, bei welchem A, wenn es vorliegt, ein Alkyl mit 1 - 4 Kohlenstoffatomen ist.

45. Das Glycolipopeptid nach Anspruch 44, bei welchem A vorliegt und -CH₂- oder -CH(CH₃)- ist.

46. Das Glycolipopeptid nach irgendeinem der Ansprüche 43 - 45, bei welchem Y eine Gruppe umfasst, die ausgewählt ist aus der Gruppe, bestehend aus -O-, -S-, -NH-, -NR-, -PO₄-, -C(=O)-, -C(=S)-, -C(=NH)- und -C(=NR)-, wobei R 1-4-Alkyl ist.

47. Das Glycolipopeptid nach Anspruch 43, bei welchem Y -NHCO-, -OCO- oder -SCO- ist.

48. Das Glycolipopeptid nach Anspruch 43, bei welchem Y -CONH- oder -CH₂NH-ist.

49. Das Glycolipopeptid nach Anspruch 43, bei welchem Y -O-, -S- oder -NH- ist.

50. Das Glycolipopeptid nach irgendeinem der Ansprüche 43 - 49, bei welchem -Y-Z selbst eine stark lipophile Gruppe ist.

51. Das Glycolipopeptid nach Anspruch 50, bei welchem A vorliegt und -A-Y-Z selbst eine stark lipophile Gruppe ist.

52. Das Glycolipopeptid nach irgendeinem der Ansprüche 43 - 51, bei welchem Z wenigstens teilweise aromatisch ist.

53. Das Glycolipopeptid nach irgendeinem der Ansprüche 43 - 52, bei welchem Z aliphatisch ist.

54. Das Glycolipopeptid nach irgendeinem der Ansprüche 43 - 53, bei welchem Z wenigstens einen Anteil in der Form -A'-Y'-Z' umfasst, wobei A', Y' und Z' analog zu A, Y bzw. Z definiert sind.

55. Das Glycolipopeptid nach Anspruch 54, wobei Y' -O- ist und Z' eine Alkylgruppe ist.

56. Das Glycolipopeptid nach Anspruch 55, wobei A -(CH₂)ᵢ- ist, wobei i 0 oder 1 ist oder Z' -(CH₂)ⱼCH₃ ist, wobei j 6 bis 26 ist.

57. Das Glycolipopeptid nach irgendeinem der Ansprüche 54 - 56, bei welchem Z -B(-Y'-Z')ₙ umfasst, bei welchem B eine verzweigte organische Gruppe mit nicht mehr als 12 Atomen, die von Wasserstoff verschieden sind, ist, jedes Y' ein unabhängig ausgewählter Abstandhalter mit nicht mehr als 12 Atomen, die von Wasserstoff verschieden sind, ist und Stickstoff, Sauerstoff, Schwefel oder Phosphor umfasst, und jedes Z' eine unabhängig ausgewählte stark lipophile Gruppe ist und n wenigstens zwei ist.

58. Das Glycolipopeptid nach Anspruch 57, bei welchem n 2 oder 3 ist.

59. Das Glycolipopeptid nach den Ansprüchen 57 oder 58, bei welchem Y' -O- ist und jedes Z' unabhängig -(CH₂)ⱼCH₃ ist, wobei j = 6 bis 26.

60. Das Glycolipopeptid nach Anspruch 59, bei welchem n = 2 und B -CH(CH₂-)₂ ist.

61. Das Glycolipopeptid nach Anspruch 60, bei welchem n = 2 und B -C(CH₂-)₃ ist.

62. Das Glycolipopeptid nach irgendeinem der Ansprüche 34 - 61, wobei die stark lipophile Gruppe von wenigstens einer lipidierten Aminosäure wenigstens eine der folgenden Strukturen umfasst: 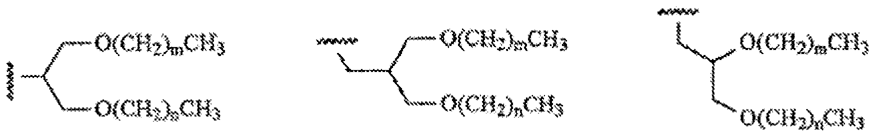 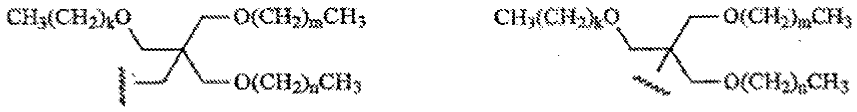 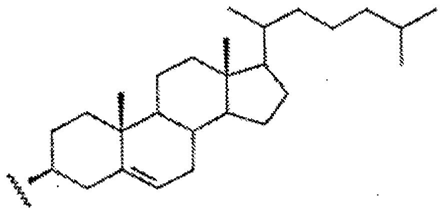 wobei m, n und k unabhängige ganze Zahlen mit Werten, die von 3 bis 30 reichen, sind.

63. Das Glycolipopeptid nach irgendeinem der Ansprüche 1 - 62, wobei wenigstens eine lipidierte Aminosäure die Struktur 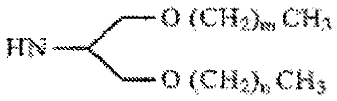 umfasst, wobei m und n unabhängige ganze Zahlen mit Werten, die von 6 bis 26 reichen, sind.

64. Das Glycolipopeptid nach irgendeinem der Ansprüche 34 - 63, wobei die stark lipophile Gruppe an wenigstens einer lipidierten Aminosäure die Struktur 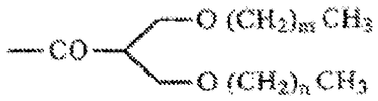 umfasst, wobei m und n unabhängig ausgewählte ganze Zahlen von 6 bis 26 sind.

65. Das Glycolipopeptid nach irgendeinem der Ansprüche 1 - 64, welches die folgende Struktur aufweist: 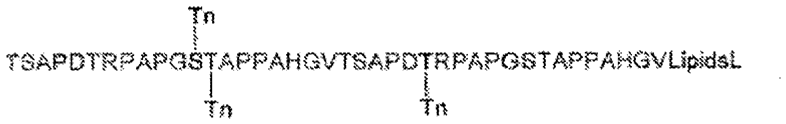 wobei Tn ein N-Acetyl-Galactosamin ist und sich "Lipids" auf zwei oder mehr aufeinander folgende lipidierte Aminosäuren bezieht.

66. Das Glycolipopeptid nach irgendeinem der Ansprüche 1 - 64, welches die folgende Struktur aufweist: 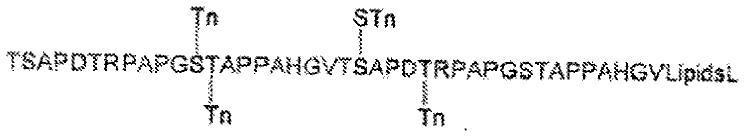 wobei Tn ein N-Acetyl-Galactosamin ist und STn ein (2-6)-Sialyl, ein N-Acetyl-Galactosamin ist und sich "Lipids" auf zwei oder mehr aufeinander folgende lipidierte Aminosäuren bezieht.

67. Das Glycolipopeptid nach irgendeinem der Ansprüche 65 oder 66, wobei die zwei oder mehr aufeinander folgenden lipidierten Aminosäuren lipidierte Serine sind.

68. Das Glycolipopeptid nach irgendeinem der Ansprüche 1 - 65 und 67, welches die folgende Struktur aufweist:

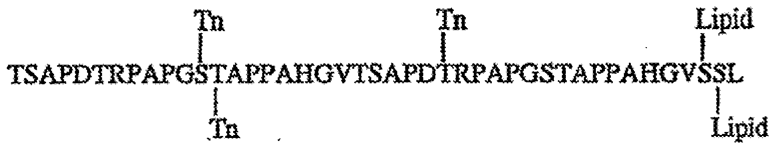

69. Das Glycolipopeptid nach irgendeinem der Ansprüche 1 - 64, 66 und 67, welches die folgende Struktur aufweist:

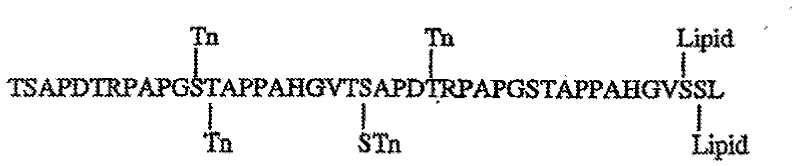

70. Das Glycolipopeptid nach irgendeinem der vorhergehenden Ansprüche, wobei wenigstens eine glycosylierte Aminosäure, die von dem Peptidepitop umfasst ist, selbst ein krebsassoziiertes Kohlenhydrat-Epitop umfasst.

71. Das Glycolipopeptid nach irgendeinem der vorhergehenden Ansprüche zur Verwendung als ein Medikament.

72. Verwendung des Glycolipopeptids nach irgendeinem der Ansprüche 1 - 70 zur Herstellung eines Medikaments zum Hervorrufen einer Immunantwort bei Verabreichung an einen Patienten (subject).

73. Das Glycolipopeptid nach irgendeinem der vorhergehenden Ansprüche 1 - 70 zur Verwendung in einem Verfahren zum Hervorrufen einer Immunantwort bei Verabreichung an einen Patienten.

74. Die Verwendung gemäß Anspruch 70, wobei der Patient an der Krankheit leidet, mit welcher das krebsassoziierte Epitop assoziiert ist.

75. Das Glycolipopeptid nach Anspruch 72, wobei der Patient an der Krankheit leidet, mit welcher das krebsassoziierte Epitop assoziiert ist.

76. Eine Zusammensetzung, welche ein Glycolipopeptid gemäß irgendeinem der Ansprüche 1 - 70 und ein Liposom umfasst.

77. Die Zusammensetzung nach Anspruch 76, welche Phosphatidylcholin umfasst.

78. Die Zusammensetzung nach Anspruch 76, welche Cholesterin umfasst.

79. Die Zusammensetzung nach Anspruch 76, welche Phosphatidyglycerol umfasst.

80. Die Zusammensetzung gemäß irgendeinem der Ansprüche 76 - 79 zur Verwendung als ein Medikament.

81. Verwendung der Zusammensetzung gemäß irgendeinem der Ansprüche 76 - 80 zur Herstellung eines Medikaments zum Hervorrufen einer Immunantwort bei Verabreichung an einen Patienten.

82. Die Zusammensetzung gemäß irgendeinem der Ansprüche 76 - 80 zur Verwendung in einem Verfahren zum Hervorrufen einer Immunantwort in einem Patienten.

## Revendications

1. Glycolipopeptide non naturel, lequel glycolipopeptide comprend au moins un acide aminé qui est un acide aminé glycosylé et au moins un acide aminé qui est un acide aminé lipidé, dans lequel au moins un acide aminé lipidé est un acide aminé intérieur, lequel glycolipopeptide comprend au moins deux épitopes MUC1 avec la séquence d'acides aminés P(D/E)(T/S)RP, et dans lequel
(i) au moins un épitope MUC1 avec la séquence d'acides aminés P(D/E)(T/S)RP comprend au moins un acide aminé glycosylé, et
ii) au moins un épitope MUC1 avec la séquence d'acides aminés P(D/E)(T/S)RP comprend au moins un acide aminé non glycosylé, et
dans lequel les séquences d'acides aminés desdits épitopes MUC1 peuvent être identiques ou différentes.

2. Glycopeptide selon la revendication 1, qui comprend la séquence d'acides aminés PDTRP (AA 6-10 de la SEQ ID NO : 10).

3. Glycolipopeptide selon la revendication 1 ou 2, dans lequel chacun des épitopes (i) et (ii) comprend la séquence d'acides aminés PDTRP.

4. Glycolipopeptide selon l'une quelconque des revendications 1 à 3, qui comprend au moins un épitope de cellule T.

5. Glycolipopeptide selon l'une quelconque des revendications 1 à 4, qui comprend la séquence d'acides aminés SAPDTRP (AA 4-10 de la SEQ ID NO : 10).

6. Glycolipopeptide selon l'une quelconque des revendications précédentes, qui comprend la séquence d'acides aminés PDTRPAPGS.

7. Glycolipopeptide selon l'une quelconque des revendications précédentes, qui comprend la séquence d'acides aminés RPAPGS.

8. Glycolipopeptide selon l'une quelconque des revendications précédentes, qui comprend la séquence d'acides aminés PPAHGVT.

9. Glycolipopeptide selon l'une quelconque des revendications précédentes, qui comprend la séquence d'acides aminés STAPPAHGV.

10. Glycolipopeptide selon l'une quelconque des revendications 1 à 9, qui comprend au moins une copie de
(a) la répétition en tandem du consensus de MUC1 GVTSAPDTRPAPGSTAPPAH (SEQ ID NO : 10),
(b) une permutation cyclique de celle-ci, ou
(c) une séquence pratiquement identique à (a) ou (b) ci-dessus.

11. Glycolipopeptide selon la revendication 10, qui comprend au moins deux copies de (a), (b) ou (c).

12. Glycolipopeptide selon la revendication 10, qui comprend au moins une copie de (a) ou (b).

13. Glycolipopeptide selon la revendication 10, qui comprend au moins deux copies de (a) ou (b).

14. Glycolipopeptide selon la revendication 10, qui comprend seulement deux copies de (a) ou (b).

15. Glycolipopeptide selon l'une quelconque des revendications précédentes, avec la séquence d'acides aminés TSAPDTRPAPGSTAPPAHGVTSAPDTRPAPGSTAPPAHGVSSL.

16. Glycolipopeptide selon l'une quelconque des revendications 1 à 15, dans lequel au moins un acide aminé glycosylé est O-glycosylé.

17. Glycolipopeptide selon l'une quelconque des revendications 1 à 15, dans lequel au moins un acide aminé glycosylé est N-glycosylé.

18. Glycolipopeptide selon l'une quelconque des revendications 1 à 15, dans lequel au moins un acide aminé glycosylé est S-glycosylé.

19. Glycolipopeptide selon l'une quelconque des revendications 1 à 18, qui comprend un épitope d'hydrate de carbone associé à une tumeur.

20. Glycolipopeptide selon la revendication 19, dans lequel l'épitope d'hydrate de carbone est GalNAc (Tn).

21. Glycolipopeptide selon la revendication 19, dans lequel l'épitope d'hydrate de carbone est sialyl Tn.

22. Glycolipopeptide selon la revendication 19, dans lequel l'épitope d'hydrate de carbone est Gal-GaINAc (TF).

23. Glycolipopeptide selon la revendication 2, dans lequel la thréonine de PDTRP est glycosylée.

24. Glycolipopeptide selon la revendication 23, dans lequel la thréonine de PDTRP est liée à Tn par liaison O.

25. Glycolipopeptide selon l'une quelconque des revendications 1 à 24, dans lequel au moins deux acides aminés sont glycosylés.

26. Glycolipopeptide selon l'une quelconque des revendications 1 à 25, dans lequel au moins deux acides aminés sont lipidés.

27. Glycolipopeptide selon l'une quelconque des revendications 1 à 26, dans lequel au moins deux acides aminés intérieurs sont lipidés.

28. Glycolipopeptide selon l'une quelconque des revendications 1 à 27, dans lequel tous les acides aminés lipidés sont des acides aminés intérieurs.

29. Glycolipopeptide selon l'une quelconque des revendications 1 à 28, **caractérisé par** une séquence carboxy-terminale SSL, où chacune des sérines est lipidée.

30. Glycolipopeptide selon l'une quelconque des revendications 1 à 29, dans lequel il n'y a pas plus de 200 acides aminés.

31. Glycolipopeptide selon l'une quelconque des revendications 1 à 30, dans lequel il n'y a pas plus de 75 acides aminés.

32. Glycolipopeptide selon l'une quelconque des revendications 1 à 31, dans lequel il n'y a pas plus de 50 acides aminés.

33. Glycolipopeptide selon l'une quelconque des revendications 1 à 32, comprenant deux répétitions en tandem effectives ne se chevauchant pas (pour un total de 40 acides aminés).

34. Glycolipopeptide selon l'une quelconque des revendications 1 à 33, dans lequel au moins un acide aminé lipidé comprend un groupe fortement lipophile comprenant au moins 13 atomes autres que l'hydrogène.

35. Glycolipopeptide selon l'une quelconque des revendications 1 à 34, dans lequel au moins un acide aminé lipidé comprend un groupe fortement lipophile comprenant au moins 21 atomes autres que l'hydrogène.

36. Glycolipopeptide selon l'une quelconque des revendications 1 à 35, dans lequel ledit groupe est constitué d'au plus 100 atomes autres que l'hydrogène.

37. Glycolipopeptide selon l'une quelconque des revendications 1 à 35, dans lequel ledit groupe est constitué d'au plus 40 atomes autres que l'hydrogène.

38. Glycolipopeptide selon l'une quelconque des revendications 1 à 37, dans lequel au moins un groupe fortement lipophile d'au moins un acide aminé lipidé a un logP, tel que prédit par l'algorithme de Meylan, d'au moins 2,7.

39. Glycolipopeptide selon l'une quelconque des revendications 1 à 38, dans lequel l'acide aminé amino-terminal comprend un groupe fortement lipophile.

40. Glycolipopeptide selon l'une quelconque des revendications 1 à 39, dans lequel l'acide aminé carboxy-terminal comprend un groupe fortement lipophile.

41. Glycolipopeptide selon l'une quelconque des revendications 1 à 40, dans lequel au moins un acide aminé lipidé est choisi dans le groupe constitué par les Ser, Thr, Asp, Glu, Cys, Tyr, Lys, Arg, Asn et Gln lipidés.

42. Glycolipopeptide selon la revendication 41, dans lequel l'acide aminé lipidé est Ser ou Thr lipidé.

43. Glycolipopeptide selon l'une quelconque des revendications 1 à 42, dans lequel au moins un acide aminé lipidé comprend une chaîne latérale de formule
-A-Y-Z i.
i) où A est optionnel mais, s'il est présent, est un groupe organique ayant au plus 12 atomes autres que l'hydrogène;
ii) Y est un espaceur ayant au plus 12 atomes autres que l'hydrogène, et comprenant de l'azote, de l'oxygène, du soufre ou du phosphore, et Z est un groupe fortement lipophile.

44. Glycolipopeptide selon la revendication 43, dans lequel A, s'il est présent, est un alkyle ayant de 1 à 4 atomes de carbone.

45. Glycolipopeptide selon la revendication 44, dans lequel A est présent et est -CH₂- ou -CH(CH₃)-.

46. Glycolipopeptide selon l'une quelconque des revendications 43 à 45, dans lequel Y comprend un groupe choisi dans l'ensemble constitué par -O-, -S-, -NH-, -NR-, -PO₄-, -C(=O)-, -C(=S)-, -C(=NH)- et -C(=NR)- où R est 1-4 alkyle.

47. Glycolipopeptide selon la revendication 43, dans lequel Y est -NHCO-, -OCO- ou -SCO-.

48. Glycolipopeptide selon la revendication 43, dans lequel Y est -CONH- ou -CH₂NH-.

49. Glycolipopeptide selon la revendication 43, dans lequel Y est -O-, -S- ou -NH-.

50. Glycolipopeptide selon l'une quelconque des revendications 43 à 49, dans lequel -Y-Z est lui-même un groupe fortement lipophile.

51. Glycolipopeptide selon la revendication 50, dans lequel A est présent et -A-Y-Z est lui-même un groupe fortement lipophile.

52. Glycolipopeptide selon l'une quelconque des revendications 43 à 51, dans lequel Z est au moins partiellement aromatique.

53. Glycolipopeptide selon l'une quelconque des revendications 43 à 52, dans lequel Z est aliphatique.

54. Glycolipopeptide selon l'une quelconque des revendications 43 à 53, dans lequel Z comprend au moins un fragment de forme -A'-Y'-Z' où A', Y' et Z' sont définies d'une manière analogue à A, Y et Z respectivement.

55. Glycolipopeptide selon la revendication 54, dans lequel Y' est -O- et Z' est un groupe alkyle.

56. Glycolipopeptide selon la revendication 55, dans lequel A est -(CH₂)ᵢ- où i vaut 0 ou 1, ou bien Z' est -(CH₂)ⱼCH₃ où j vaut de 6 à 26.

57. Glycolipopeptide selon l'une quelconque des revendications 54 à 56, dans lequel Z comprend -B(-Y'-Z'-)ₙ où B est un groupe organique ramifié ayant au plus 12 atomes autres que l'hydrogène, chaque Y' est un espaceur indépendamment choisi ayant au plus 12 atomes autres que l'hydrogène, et comprenant de l'azote, de l'oxygène, du soufre ou du phosphore, et chaque Z' est un groupe fortement lipophile indépendamment choisi, et n vaut au moins deux.

58. Glycolipopeptide selon la revendication 57, dans lequel n vaut 2 ou 3.

59. Glycolipopeptide selon la revendication 57 ou 58, dans lequel chaque Y' est -O- et chaque Z' est indépendamment -(CH₂)ⱼCH₃ où j vaut de 6 à 26.

60. Glycolipopeptide selon la revendication 59, dans lequel n vaut 2 et B est -CH(CH₂)₂-.

61. Glycolipopeptide selon la revendication 60, dans lequel n vaut 2 et B est -C(CH₂-)₃.

62. Glycolipopeptide selon l'une quelconque des revendications 34 à 61, dans lequel le groupe fortement lipophile d'au moins un acide aminé lipidé comprend au moins l'une des structures suivantes : 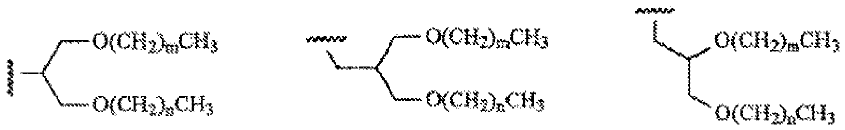 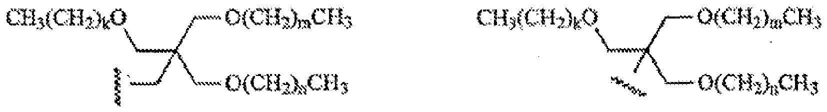 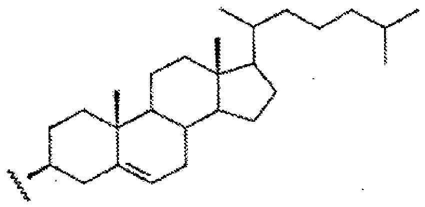 où m, n et k sont des entiers indépendants ayant des valeurs situées dans la plage allant de 3 à 30.

63. Glycolipopeptide selon l'une quelconque des revendications 1 à 62, dans lequel au moins un acide aminé lipidé comprend la structure 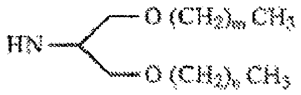 dans laquelle m et n sont des entiers indépendants ayant des valeurs situées dans la plage allant de 6 à 26.

64. Glycolipopeptide selon l'une quelconque des revendications 34 à 63, dans lequel le groupe fortement lipophile d'au moins un acide aminé lipidé comprend la structure 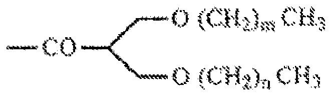 dans laquelle m et n sont des entiers de 6 à 26 indépendamment choisis.

65. Glycolipopeptide selon l'une quelconque des revendications 1 à 64, ayant la structure suivante : 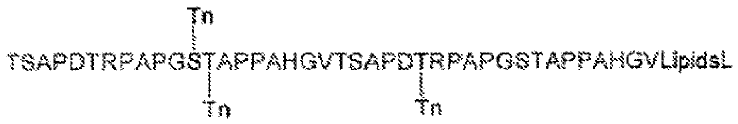 dans laquelle Tn est une N-acétylgalactosamine, et "Lipids" se réfère à deux ou plus de deux acides aminés lipidés consécutifs.

66. Glycolipopeptide selon l'une quelconque des revendications 1 à 64, ayant la structure suivante : 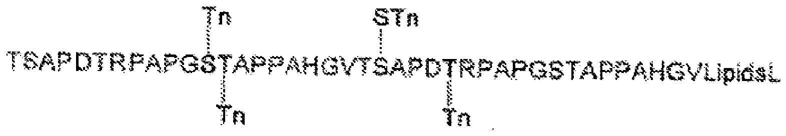 dans laquelle Tn est une N-acétylgalactosamine et STn est une (2-6) sialyle, une N-acétylgalactosamine, et "Lipids" se réfère à deux ou plus de deux acides aminés lipidés consécutifs.

67. Glycolipopeptide selon l'une quelconque des revendications 65 et 66, dans lequel les deux ou plus de deux acides aminés lipidés consécutifs sont des sérines lipidées.

68. Glycolipopeptide selon l'une quelconque des revendications 1 à 65 et 67, ayant la structure suivante :

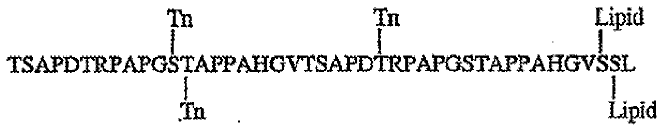

69. Glycolipopeptide selon l'une quelconque des revendications 1 à 64, 66 et 67, ayant la structure suivante :

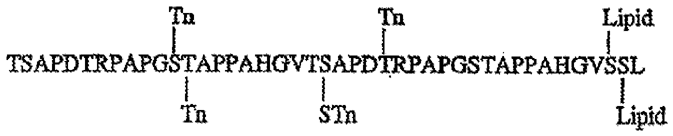

70. Glycolipopeptide selon l'une quelconque des revendications précédentes, dans lequel au moins un acide aminé glycosylé compris par ledit épitope peptidique qui lui-même comprend un épitope d'hydrate de carbone associé à un cancer.

71. Glycolipopeptide selon l'une quelconque des revendications précédentes, pour une utilisation en tant que médicament.

72. Utilisation du glycolipopeptide de l'une quelconque des revendications 1 à 70, pour la fabrication d'un médicament destiné à déclencher une réponse immunitaire après administration à un sujet.

73. Glycolipopeptide selon l'une quelconque des revendications 1 à 70, pour une utilisation dans un procédé pour déclencher une réponse immunitaire après administration à un sujet.

74. Utilisation selon la revendication 70, dans laquelle ledit sujet souffre d'une maladie à laquelle est associé ledit épitope associé à un cancer.

75. Glycolipopeptide selon la revendication 72, dans lequel ledit sujet souffre d'une maladie à laquelle est associé ledit épitope associé à un cancer.

76. Composition comprenant un glycolipopeptide de l'une quelconque des revendications 1 à 70, et un liposome.

77. Composition selon la revendication 76, qui comprend de la phosphatidylcholine.

78. Composition selon la revendication 76, qui comprend du cholestérol.

79. Composition selon la revendication 76, qui comprend du phosphatidylglycérol.

80. Composition selon l'une quelconque des revendications 76 à 79, pour une utilisation en tant que médicament.

81. Utilisation de la composition de l'une quelconque des revendications 76 à 80, pour la fabrication d'un médicament destiné à déclencher une réponse immunitaire après administration à un sujet.

82. Composition selon l'une quelconque des revendications 76 à 80, pour une utilisation dans un procédé pour déclencher une réponse immunitaire après administration à un sujet.
